(19) Europäisches Patentamt / European Patent Office / Office européen des brevets

(11) EP 2 119 704 A1

(12) EUROPEAN PATENT APPLICATION
published in accordance with Art. 153(4) EPC

(43) Date of publication:
**18.11.2009 Bulletin 2009/47**

(21) Application number: **08710858.5**

(22) Date of filing: **06.02.2008**

(51) Int Cl.:
***C07D 209/88*** (2006.01) ***A61K 31/403*** (2006.01)
***A61K 31/407*** (2006.01) ***A61K 31/4155*** (2006.01)
***A61K 31/437*** (2006.01) ***A61K 31/4439*** (2006.01)
***A61K 31/454*** (2006.01) ***A61K 31/496*** (2006.01)
***A61K 31/506*** (2006.01) ***A61K 31/5377*** (2006.01)
***A61P 25/00*** (2006.01) ***A61P 25/14*** (2006.01)
***A61P 25/18*** (2006.01) ***A61P 25/20*** (2006.01)
***A61P 25/22*** (2006.01) ***A61P 25/24*** (2006.01)
***A61P 25/28*** (2006.01) ***A61P 43/00*** (2006.01)
***C07D 401/04*** (2006.01) ***C07D 401/06*** (2006.01)

(86) International application number:
**PCT/JP2008/051962**

(87) International publication number:
**WO 2008/096791 (14.08.2008 Gazette 2008/33)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.02.2007 JP 2007028089**

(71) Applicant: **Astellas Pharma Inc.**
**Tokyo 103-8411 (JP)**

(72) Inventors:
- **KINOYAMA, Isao**
**Tokyo 103-8411 (JP)**
- **MIYAMOTO, Satoshi**
**Tokyo 103-8411 (JP)**
- **HOSHII, Hiroaki**
**Tokyo 103-8411 (JP)**
- **MIYAZAKI, Takehiro**
**Tokyo 103-8411 (JP)**
- **YAMAZAKI, Mayako**
**Tokyo 103-8411 (JP)**

(74) Representative: **HOFFMANN EITLE**
**Patent- und Rechtsanwälte**
**Arabellastrasse 4**
**81925 München (DE)**

# (54) ACYLGUANIDINE DERIVATIVE

(57) An object of the present invention is to provide a novel and excellent agent for treating or preventing dementia, schizophrenia and the like, based on the 5-$HT_{5A}$ receptor modulating action. It was confirmed that a compound **characterized by** a structure that a tricyclic hetero ring having a pyrrole ring at the center and guanidine are bonded via a carbonyl group has a potent 5-$HT_{5A}$ receptor modulating action and an excellent pharmacological action based thereon, and thus, it was found that the compound can be an excellent agent for treating or preventing dementia, schizophrenia, bipolar disorder, or attention deficit hyperactivity disorder, particularly for memory-related functional disorders such as cognitive impairments including dementia and schizophrenia, thereby completing the present invention.

EP 2 119 704 A1

## Description

Technical Field

The present invention relates to a pharmaceutical, in particular, a substituted guanidine derivative which has a 5-$HT_{5A}$ receptor modulating action and is useful as a pharmaceutical composition for treating or preventing dementia, schizophrenia and the like.

Background Art

Dementia is a syndrome based on memory impairment and judgment impairment, caused by a decrease in brain functions by acquired brain disorders, and vascular dementia and Alzheimer-type dementia are its representative primary diseases. Conventionally, agents for treating these have been investigated, however, these were not sufficient in clinical satisfaction. For example, it has been reported that a cholinesterase inhibitor such as Aricept and the like, that is widely used as an agent for treating Alzheimer-type dementia, does not have a sufficient effect (Curr. Neurol. Neurosci. Rep., 5 (6), 455-457, 2005; Eur. J. Pharmacol., 346, 1-13, 1998). Also, its side effects due to the stimulation of the peripheral cholinergic nervous system have been also pointed out (Curr. Psychiatry Rep., 2 (6), 473-478; J. Psychopharmacol., 14 (4), 406-408, 2000). In addition, an NMDA antagonist, such as memantine and the like, has been approved in some countries, but its side effects have been highlighted particularly for the patients with mental symptoms such as cognitive impairment, hallucinations, ataxia, mental disorders and the like (J. Clin. Psychiatry 66 (5), 658-659, 2005; Learning & memory, 8, 20-25, 2001).
On the other hand, schizophrenia is a mental disorder which shows diverse symptoms such as delusion, hallucinations, hyperactivity, depression and the like. Its symptoms are broadly classified into positive symptoms, negative symptoms, and cognitive impairment. Conventionally, for the treatment of schizophrenia, a D2 receptor blocker such as haloperidol and the like that is a first-generation typical antipsychotic drug, and olanzapine and the like that is a second-generation atypical antipsychotic drug have been used. However, side effects such as, extrapyramidal symptoms for haloperidol and the like, and obesity, hyperglycemia and diabetic ketoacidosis for olanzapine have been reported (Togoshicchosho-chiryoyaku to Kanja eno Setsumei (An agent for treating schizophrenia, and description thereof to a patient, 54, 287-304, 2003; Am J Psychiatry, 160, 1209-1222, 2003; Neuropsychopharmacology, 28 (8), 1400-1411, 2003; Diabetes Care, 27, 596, 2004; Rinsho-seishin-yakuri (Clinical Psychopharmacology), 8 (12), 2151-2164, 2005). In addition, conventional pharmaceutical agents can improve the positive symptoms, but are insufficient in the efficacy for the negative symptoms and the cognitive impairment (J. Abnorm. Psychol., 1997; Rinsho-seishin-yakuri (Clinical Psychopharmacology), 8 (12), 2151-2164, 2005).
From the background above, an agent for treating dementia and an agent for treating schizophrenia which are safe and highly effective are desired.
Recently, there has been suggested that a 5-$HT_{5A}$ receptor that is one of the serotonin receptor subtypes plays an important role in dementia and schizophrenia. For example, it has been reported that a new exploration is increased in 5-$HT_{5A}$ receptor-knockout mice and the overactivity by LSD is inhibited in 5-$HT_{5A}$ receptor-knockout mice (Neuron, 22, 581-591, 1999). From the results of the gene expression analyses, it has been reported that the 5-$HT_{5A}$ receptor is highly expressed in the brains of humans and rodents, and in brain, the expression is high in hippocampal CA1 and CA3 pyramidal cells which are involved in memory and in frontal lobe (cerebral cortex) which is deeply involved in schizophrenia (molecular Brain Reserch, 56, 1-8, 1998). Further, it has been reported that the gene polymorphism of the 5-$HT_{5A}$ receptor is related with schizophrenia (Neuroreport 11, 2017-2020, 2000; Mol. Psychiatr. 6, 217-219, 2001; J. Psychiatr. Res. 38, 371-376, 2004).
Hitherto, several compounds having high affinity for the 5-$HT_{5A}$ receptor have been reported. For example, it has been described that a guanidine derivative represented by the following general formula binds to the 5-$HT_{5A}$ receptor, and is used for the treatment of a variety of central diseases such as neurodegenerative diseases, neuropsychiatric diseases and the like (Patent Document 1).

[Chem. 1]

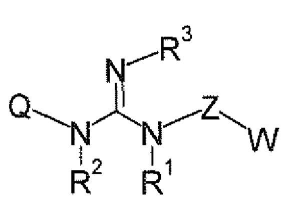

W: 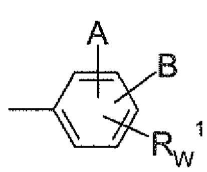

or 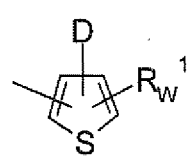

(wherein A represents $NO_2$, $NH_2$ and the like, B represents a hydrogen atom and the like, $R_w{}^1$ represents a hydrogen atom and the like, D represents a group represented in A, Q represents a di-substituted 5-membered heteroaryl, $R^1$, $R^2$, and $R^3$ represent a hydrogen atom and the like, Z represents $-(CR_z{}^1R_z{}^2)_a-(V_z)_b-(CR_z{}^3R_z{}^4)_c-$ (wherein a and c represent 0 to 4, b represents 0 or 1, $R_z{}^1$, $R_z{}^2$, $R_z{}^3$, and $R_z{}^4$ represents a hydrogen atom and the like, and $V_z$ represents CO and the like). For the details, refer to the publication.)

This applicant reported in a scientific meeting that the compound included in this patent application had exhibited effectiveness in a model for schizophrenia (Non-Patent Document 1).

In addition, as compounds having high affinity for the $5\text{-}HT_{5A}$ receptor, a biaryl compound (Patent Document 2) and a (3,4-dihydraquinazolin-2-yl)-indan-1-ylamine derivative (Patent Document 3) have been reported. These documents describe a number of uses for central nervous diseases. Further, a Patent Publication, that describes "A method for using 5-HT5 ligands to treat neurodegenerative diseases or neuropsychiatric diseases" in claims, has been published (Patent Document 4). This publication describes test results confirming the neroprotective action of the compound, using the compound described in German Patent No. 19724979.5 (a 3,4,5,6,7,8-hexahydropyrido[3',4':4,5]thieno[2,3-d]pyrimidine derivative).

Patent Document 5 describes that a compound represented by the following general formula is effective for treating a variety of neurodegenerative diseases, and mentions the terms Alzheimer's disease and dementia. The general formula of this international publication encompasses a compound having tricyclic heteroaryl, but specific disclosure of such a compound is not found in the specification.

[Chem. 2]

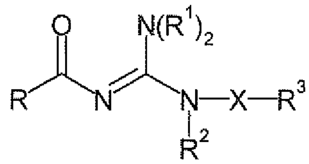

(wherein R represents cycloalkyl, aryl, mono- to tricyclic heteroaryl or the like, $R^1$ and $R^2$ independently represent H, alkyl, alkenyl or the like, X represents a bond, an alkene, an alkenylene or the like, and $R^3$ represents cycloalkyl, aryl, alkylaryl or the like. For the details, refer to the publication.)

Patent Document 6 describes that a compound represented by the following general formula has an NO synthase inhibitory activity and/or a reactive oxygen species scavenging action, and mentions the terms Alzheimer's disease and dementia along with most other indications. The general formula of this international publication includes those in which B is $NR^{13}R^{14}$, but specific disclosure of such a compound having guanidine is not found in the specification.

[Chem. 3]

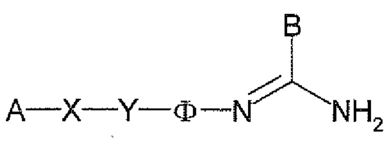

(D)

A: 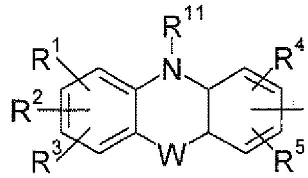 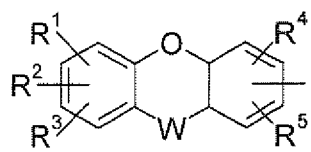

(wherein Φ represents a bond or a phenylene group, B represents $-CH_2-NO_2$, an alkyl group, an aryl group, $NR^{13}R^{14}$ or the like, in which $R^{13}$ and $R^{14}$ independently represent a hydrogen atom, an alkyl group, a cyano group or the like, X represents a bond, -O-, -S-, CO- or the like, Y represents a bond, $-(CH_2)_m-$ or the like, W is not present or represents a bond, an S atom, or $NR^{15}$, and $R^1$ to $R^5$ represent hydrogen, halogen or the like. For the details, refer to the publication.)

It has been reported that a fluorene derivative represented by the following general formula has an antagonistic activity on the $5\text{-}HT_{2B}$ and $5\text{-}HT_7$ receptors, and is effective for preventing migraines (Patent Documents 7 and 8).

[Chem. 4]

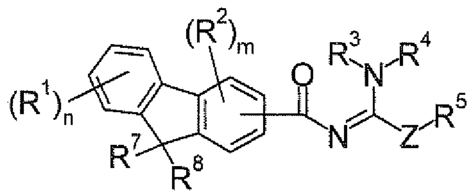

Moreover, some compounds of the present application are described in the international publication of the international application by the Applicant, published after the priority date of the present application (Patent Document 9). However, these publications have no disclosure about uses for dementia, schizophrenia, cognitive impairment and the like.

[Patent Document 1] Pamphlet of International Publication No. 05/082871
[Patent Document 2] Pamphlet of International Publication No. 04/096771
[Patent Document 3] Specification of U.S. Patent Application Publication No. 2006/0229323
[Patent Document 4] Pamphlet of International Publication No. 00/41696
[Patent Document 5] Pamphlet of International Publication No. 99/20599
[Patent Document 6] Pamphlet of International Publication No. 00/17191
[Patent Document 7] Pamphlet of International Publication No. 05/080322
[Patent Document 8] Pamphlet of International Publication No. 05/079845
[Patent Document 9] Pamphlet of International Publication No. 07/018168
[Non-Patent Document 1] Jongen-Relo A. L. et al., 36th Annual Meeting, Society of Neuroscience, October 14 to 18, 2006, Atlanta, Canada, Lecture Summary No. 529.26

DISCLOSURE OF THE INVENTION

PROBLEM THAT THE INVENTION IS TO SOLVE

An object of the present invention is to provide a novel and excellent pharmaceutical composition for treating or preventing dementia, schizophrenia and the like, based on a $5\text{-HT}_{5A}$ receptor modulating action.

MEANS FOR SOLVING THE PROBLEM

The present inventors have extensively studied on compounds having a $5\text{-HT}_{5A}$, receptor modulating action, and as a result, they have found a compound characterized by a structure that a tricyclic hetero ring having a pyrrole ring at the center and guanidine are bonded via a carbonyl group has a potent $5\text{-HT}_{5A}$ receptor modulating action and an excellent pharmacological action based thereon, and found that it can be an excellent agent for treating or preventing dementia, schizophrenia and the like, thereby completing the present invention.
A compound represented by the following general formula (I), which is an active ingredient of the pharmaceutical of the present invention is totally different in the structure from the conventionally reported compounds having high affinity for the $5\text{-HT}_{5A}$ receptor (aforementioned Patent Documents 1 to 4, and Non-Patent Document 1). Some of the compounds represented by the general formula (I) are included conceptually in claims at an international stage of Patent Document 5. However, Patent Document 5 has no specific disclosure of a compound having a tricyclic skeleton which is a characteristic of the compound of the present invention. Moreover, the compounds described in Examples are limited to ones in which this moiety is monocyclic. Some of the compounds represented by the general formula (I) are included conceptually in claims at an international stage of Patent Document 6. However, there is no specific disclosure about a compound having guanidine in Patent Document 6. Further, the compound in this Patent Document is different in the pharmacological action from the compound of the present invention, since it has an NO synthase inhibitory action and/or a reactive oxygen species scavenging action. The compound represented by the general formula (I) is different in its structure from the fluorene derivatives of Patent Documents 7 and 8 since it has a tricyclic hetero ring having a pyrrole ring at the center. In addition, the compounds of the Patent Documents are different in the indications from the compound of the present invention since they take prevention of migraine as indications.
Specifically, the present invention relates to a $5\text{-HT}_{5A}$ receptor modulator comprising a compound represented by the following general formula (I) or a salt thereof as an active ingredient.

[Chem. 5]

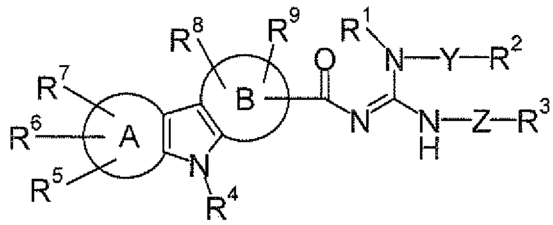

(I)

(the symbols in the formula represent the following meanings:

$R^1$: H, lower alkyl, halogeno-lower alkyl, $C_{2-6}$ alkylene-$OR^a$, or $C_{2-6}$ alkylene-$NR^aR^b$,
$R^2$ and $R^3$: the same as or different from each other, each representing H, $-OR^a$, $-NR^aR^b$, phenyl, cycloalkyl, or a monocyclic heterocyclic group, or $R^2$ together with $R^1$ and with a nitrogen atom may form a monocyclic nitrogen-containing heterocyclic group, wherein phenyl, cycloalkyl, the monocyclic heterocyclic group, and the monocyclic nitrogen-containing heterocyclic group may be substituted with lower alkyl or $-OR^a$,
$R^a$ and $R^b$: the same as or different from each other, each representing H or lower alkyl,
$R^4$: lower alkyl which may be substituted with one or two groups selected from the group represented by Group G, H, $-C(O)R^a$, $-S(O)_p$-lower alkyl, $-C(O)NR^aR^b$, or -L-X,
Group G: $-NR^aR^b$, $-OR^a$, or -O-lower alkylene-$OR^a$,
L: a bond, -C(O)-, $-S(O)_p-$, lower alkylene, or lower alkylene-O- lower alkylene, wherein lower alkylene may be substituted with $-OR^a$,
X: a heterocyclic group, aryl, cycloalkyl, or cycloalkenyl, wherein the ring group represented by X may be substituted with one or two groups selected from lower alkyl, halogen, $-OR^a$, $-C(O)R^a$, $-CO_2R^a$, $-S(O)_p$-lower alkyl, -CN, lower alkylene-CN, benzhydryl, phenyl, monocyclic heteroaryl, and oxo,
p: 0, 1, or 2,

[Chem. 6]

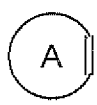 :

a benzene, thiophene, furan, cyclohexene, or tetrahydropyridine ring,
$R^5$, $R^6$, and $R^7$: the same as or different from each other, each representing H, lower alkyl, lower alkenyl, halogen, -O-halogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^a$, $-C(O)NR^aR^b$, lower alkylene-$OR^a$, or lower alkylene-$NR^aR^b$,

[Chem. 7]

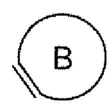 :

a benzene, cyclohexene or tetrahydropyridine ring,
$R^8$ and $R^9$: the same as or different from each other, each representing H, lower alkyl, lower alkenyl, halogen, -O-halogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^2$, $-C(O)NR^aR^b$, lower alkylene-$OR^a$, or lower alkylene-$NR^aR^b$, and

Y and Z: the same as or different from each other, each representing a bond, lower alkylene, or lower alkylene-O-.

Furthermore, the symbols as used in BEST MODE FOR CARRYING OUT THE INVENTION and thereafter have the same meanings.)

Further, the present invention relates to a pharmaceutical composition for preventing or treating dementia, schizophrenia, bipolar disorder, or attention deficit hyperactivity disorder, and preferably a pharmaceutical composition for preventing or treating dementia or schizophrenia, which comprises the compound represented by the aforementioned general formula (I) or a salt thereof as an active ingredient.

Also, in a further embodiment, it relates to a pharmaceutical composition for preventing or treating dementia, schizophrenia, bipolar disorder, or attention deficit hyperactivity disorder, and more preferably, a pharmaceutical composition for preventing or treating dementia or schizophrenia, which is a 5-$HT_{5A}$ receptor modulator comprising the compound represented by the aforementioned general formula (I) or a salt thereof as an active ingredient.

In addition, in an even further embodiment, it relates to use of the compound represented by the aforementioned formula (I) or a salt thereof for the manufacture of a pharmaceutical composition for preventing or treating dementia, schizophrenia, bipolar disorder, or attention deficit hyperactivity disorder, preferably, dementia or schizophrenia, and to a method for preventing or treating dementia, schizophrenia, bipolar disorder, or attention deficit hyperactivity disorder, preferably, dementia or schizophrenia, which comprises administering to a mammal an effective amount of the compound or a salt thereof.

Furthermore, the present invention relates to a novel compound represented by the following general formula (I') or a salt thereof, and a novel compound represented by the following general formula (I"), which have a 5-$HT_{5A}$ receptor modulating action, and are useful as an agent for treating or preventing 5-$HT_{5A}$ receptor-related diseases such as dementia, schizophrenia and the like. The compounds of the formula (I') and the formula (I") are included in the aforementioned general formula (I).

[Chem. 8]

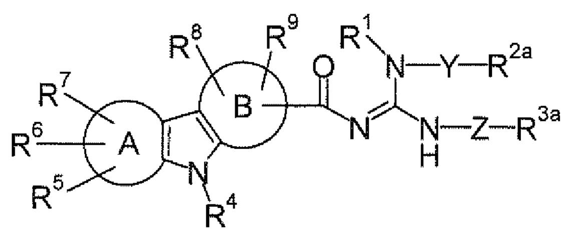

(I')

(the symbols in the formula represent the following meanings:

$R^1$: H, lower alkyl, halogeno-lower alkyl, $C_{2-6}$ alkylene-$OR^a$ or $C_{2-6}$ alkylene-$NR^aR^b$,

$R^{2a}$: H, -$OR^a$, -$NR^aR^b$, phenyl, cycloalkyl, or a monocyclic heterocyclic group, or $R^{2a}$ together with $R^1$ and with a nitrogen atom may form a monocyclic nitrogen-containing heterocyclic group,

$R^{3a}$: -$OR^a$, -$NR^aR^b$, phenyl, cycloalkyl, or a monocyclic heterocyclic group,

wherein phenyl, cycloalkyl, the monocyclic heterocyclic group, and the monocyclic nitrogen-containing heterocyclic group in aforementioned $R^{2a}$ and $R^{3a}$ may be substituted with lower alkyl or -$OR^a$,

$R^a$ and $R^b$: the same as or different from each other, each representing H or lower alkyl,

$R^4$: lower alkyl which may be substituted with one or two groups selected from the groups represented by Group G, H, -C(O)$R^a$, -$S(O)_p$-lower alkyl, -C(O)$NR^aR^b$, or -L-X,

Group G: -$NR^aR^b$, -$OR^a$, or -O-lower alkylene-$OR^a$,

L: a bond, -C(O)-, -$S(O)_p$-, lower alkylene, or lower alkylene-O-lower alkylene, wherein lower alkylene may be substituted with -$OR^a$,

X: a heterocyclic group, aryl, cycloalkyl, or cycloalkenyl, wherein the ring group represented by X may be substituted with one or two groups selected from lower alkyl, halogen, -$OR^a$, -C(O)$R^a$, -$CO_2R^a$, -$S(O)_p$-lower alkyl, -CN, lower alkylene-CN, benzhydryl, phenyl, monocyclic heteroaryl, and oxo,

p: 0, 1, or 2,

[Chem. 9]

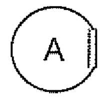 :

a benzene, thiophene, furan, cyclohexene or tetrahydropyridine ring,
$R^5$, $R^6$, and $R^7$: the same as or different from each other, each representing H, lower alkyl, lower alkenyl, halogen, -O-balogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^a$, $-C(O)NR^aR^b$, lower alkylene-$OR^a$ or lower alkylene-$NR^aR^b$,

[Chem. 10]

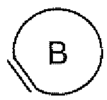 :

a benzene, cyclohexene or tetrahydropyridine ring,
$R^8$ and $R^9$: the same as or different from each other, each representing H, lower alkyl, lower alkenyl, halogen, -O-halogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^a$, $-C(O)NR^aR^b$, lower alkylene-$OR^a$, or lower alkylene-$NR^aR^b$, and
Y and Z: the same as or different from each other, each representing a bond, lower alkylene, or lower alkylene-O-.)

[Chem. 11]

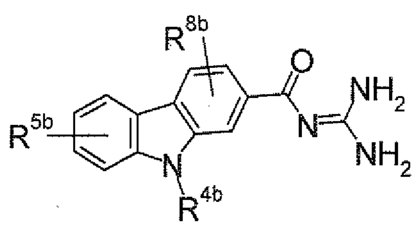 (I")

(the symbols in the formula represent the following meanings:

$R^{4b}$: isopropyl, tetrahydropyranyl, piperidyl, cyclohexyl, cyclohexenyl, phenyl, thienyl, pyridyl, thienylmethyl, or isoxazolylmethyl, wherein the piperidyl group may be substituted with cyanomethyl or phenyl, and the other groups may be substituted with one or two groups selected from F, -O-methyl, and methyl,
$R^{5b}$: H, lower alkyl, -OH, -S-lower alkyl, halogen, lower alkylene-OH, or lower alkylene-O-lower alkyl, and
$R^{8b}$: H, lower alkyl, halogen, or lower alkylene-OH,
provided that when $R^{4b}$ is isopropyl, $R^{5b}$ is -OH, and when $R^{4b}$ is unsubstituted tetrahydropyranyl, unsubstituted piperidyl, or unsubstituted cyclohexyl, either of $R^{5b}$ and $R^{8b}$ represents a group other than H).

The compound represented by (I") has a certain substituent at $R^{4b}$, $R^{5b}$, and $R^{8b}$ on a carbazole ring, and as a result, is excellent in any one of metabolic stability, safety, and oral absorbability.

Further, the present invention relates to a pharmaceutical composition which comprises the compound represented by the aforementioned formula (I') or (I") or a salt thereof as an active ingredient, that is, a pharmaceutical composition which comprises the compound represented by the formula (I') or (I") or a salt thereof and a pharmaceutically acceptable carrier. Preferably, it relates to the aforementioned pharmaceutical composition which is a $5-HT_{5A}$ receptor

modulator, more preferably a pharmaceutical composition for preventing or treating dementia, schizophrenia, bipolar disorder, or attention deficit hyperactivity disorder, and even more preferably a pharmaceutical composition for preventing or treating dementia or schizophrenia.

Also, in an even further embodiment, it relates to a pharmaceutical composition for preventing or treating dementia, schizophrenia, bipolar disorder, or attention deficit hyperactivity disorder, and preferably, pharmaceutical composition for preventing or treating dementia or schizophrenia, which comprises a compound represented by the aforementioned formula (I') or (I") or a salt thereof as an active ingredient.

In addition, in an even further embodiment, it relates to use of the compound represented by the aforementioned formula (I') or (I") or a salt thereof for the manufacture of a pharmaceutical composition for preventing or treating dementia, schizophrenia, bipolar disorder, or attention deficit hyperactivity disorder, and preferably, dementia or schizophrenia, and to a method for preventing or treating dementia, schizophrenia, bipolar disorder, or attention deficit hyperactivity disorder, preferably, dementia, or schizophrenia, which comprises administering to a mammal an effective amount of the compound or a salt thereof.

## EFFECT OF THE INVENTION

The compound that is an active ingredient of the pharmaceutical of the present invention has advantages that it has a 5-$HT_{5A}$ receptor modulating action, and an excellent pharmacological action based thereon. The pharmaceutical composition of the present invention is useful for treating or preventing 5-$HT_{5A}$ receptor-related diseases, and particularly, for treating or preventing dementia, schizophrenia, bipolar disorder, or attention deficit hyperactivity disorder. The compound that is an active ingredient of the pharmaceutical of the present invention particularly has the effects of improving memory-related functional disorders such as dementia and a cognitive impairment in schizophrenia.

## BEST MODE FOR CARRYING OUT THE INVENTION

Hereinbelow, the present invention will be described in detail.

In the present specification, the "5-$HT_{5A}$ receptor modulator" is a generic term referring to a compound which antagonizes to endogenous ligands thereby inhibiting activation of the 5-$HT_{5A}$ receptor (a 5-$HT_{5A}$ receptor antagonist), and a compound which exhibits an action of activating the 5-$HT_{5A}$ receptor (a 5-$HT_{5A}$ receptor agonist). For the "5-$HT_{5A}$ receptor modulating action", a 5-$HT_{5A}$ receptor antagonist is preferred.

The "lower alkyl" is preferably linear or branched alkyl having 1 to 6 carbon atoms (which is hereinafter simply referred to as $C_{1-6}$), and specifically, it includes methyl, ethyl, n-propyl, isopropyl, n-butyl, isobutyl, sec-butyl, tert-butyl, n-pentyl, n-hexyl group and the like. More preferably, it is $C_{1-4}$ alkyl, and even more preferably, it includes methyl, ethyl, n-propyl, and isopropyl.

The "lower alkylene" is preferably linear or branched, $C_{1-6}$ alkylene, and specifically, it includes methylene, ethylene, trimethylene, tetramethylene, pentamethylene, hexamethylene, propylene, methylmethylene, ethylethylene, 1,2-dimethylethylene, 1,1,2,2-tetramethylethylene group and the like. More preferably, it is $C_{1-4}$ alkylene, and even more preferably, it includes methylene, ethylene, trimethylene, and propylene group.

The "halogen" means F, Cl, Br, or I.

The "halogena-lower alkyl" refers to $C_{1-6}$ alkyl substituted with one or more halogen. It is preferably $C_{1-6}$ alkyl substituted with 1 to 5 halogens, and more preferably, it includes monofluoroethyl and trifluoromethyl group.

The "cycloalkyl" refers to a $C_{3-10}$ saturated hydrocarbon ring group and may have a bridge. Specifically, it includes cyclopropyl, cyclobutyl, cyclopentyl, cyclohexyl, cycloheptyl, cyclooctyl, adamantyl group and the like. It is preferably $C_{3-8}$ cycloalkyl, and more preferably $C_{3-6}$ cycloalkyl, and even more preferably it includes cyclopropyl, cyclobutyl, cyclopentyl, and cyclohexyl group.

The "cycloalkenyl" refers to $C_{5-10}$ cycloalkenyl, and preferably, it includes cyclopentenyl, cyclopentadienyl, cyclohexenyl, and cycloheptenyl group, and more preferably cyclohexenyl group.

The "aryl" refers to a $C_{6-14}$ monocyclic to tricyclic aromatic hydrocarbon ring group, and preferably, it includes phenyl, and naphthyl group, and more preferably phenyl group.

The "heterocyclic" group refers to a 3- to 15-membered, preferably 5- to 10-membered, monocyclic to tricyclic heterocyclic group containing I to 4 hetero atoms selected from oxygen, sulfur, and nitrogen, and it includes a saturated ring, an aromatic ring, and a partially hydrogenated ring group thereof. The ring atom, sulfur or nitrogen, may be oxidized to form an oxide or a dioxide. Specifically, it includes pyridyl, pyrrolyl, pyrazinyl, pyrimidinyl, pyridazinyl, imidazolyl, triazolyl, triazinyl, thienyl, furyl, thiazolyl, pyrazolyl, isothiazolyl, oxazolyl, isoxazolyl, thiadiazolyl, oxadiazolyl, azetidinyl, pyrrolidinyl, piperidyl, piperazinyl, azepanyl, diazepanyl, azocanyl, morpholinyl, thiomorpholinyl, tetrahydropyridinyl, oxiranyl, oxetanyl, tetrahydrofuryl, tetrahydropyranyl, 1,4-dioxoranyl, dioxanyl, tetrahydrothiopyranyl, quinolyl, isoquinolyl, tetrahydroquinolyl, tetrahydroisoquinolyl, quinazolinyl, quinoxalinyl, phthalazinyl, benzoimidazolyl, benzofuryl, benzothienyl, benzothiadiazolyl, benzothiazolyl, benzoisothiazolyl, benzoxazolyl, benzoisoxazolyl, methylenedioxyphenyl, ethylenedioxyphenyl, indolyl, isoindolyl, indolinyl, indazolyl, tetrahydrobenzoimidazolyl, dihydrobenzofuryl, chromanyl, chromonyl, 1,4-dithiaspiro[4.5]decanyl group and the like. More preferably, it is a 5- to 10-membered, monocyclic to bicyclic heterocyclic group, and even more preferably, it is a 5- to 6-membered, monocyclic heterocyclic group.
The "monocyclic heteroaryl" refers to a 5- to 6-membered monocyclic, aromatic ring group among the aforementioned heterocyclic group, and preferably, it includes pyridyl, pyrrolyl, pyrazinyl, pyrimidinyl, pyridazinyl, imidazolyl, triazolyl, thienyl, furyl, thiazolyl, pyrazolyl, isothiazolyl, oxazolyl, isoxazolyl, and tetrazolyl, and more preferably, pyridyl, pyrimidinyl, thienyl, furyl, and isoxazolyl.

The "monocyclic nitrogen-containing heterocyclic group" means a 5- to 8-membered monocyclic ring group which comprises one nitrogen atom, and may further comprise one of hetero atoms consisting of nitrogen, oxygen, and sulfur, among the aforementioned heterocyclic groups, and is a generic term referring to a "monocyclic nitrogen-containing saturated heterocyclic group" that is a saturated or partially unsaturated ring group, and a "monocyclic nitrogen-containing heteroaryl" that is an unsaturated ring group. The monocyclic nitrogen-containing saturated heterocyclic group preferably includes azetidinyl, pyrrolidinyl, piperidyl, piperazinyl, azepanyl, diazepanyl, azocanyl, morpholinyl, thiomorpholinyl, and tetrahydropyridinyl group. It more preferably includes pyrrolidinyl, piperidyl, piperazinyl, and diazepanyl group. The monocyclic nitrogen-containing heteroaryl preferably includes pyridyl, pyrimidinyl, and isoxazolyl.
The "monocyclic oxygen-containing saturated heterocycle" means a 3- to 7-membered, saturated monocyclic group which comprises one oxygen atom, and may further comprise one of hetero atoms consisting of nitrogen, oxygen, and sulfur, among the aforementioned heterocyclic group. It preferably includes oxiranyl, oxetanyl, tetrahydrofuryl, tetrahydropyranyl, and 1,4-dioxanyl group, and particularly preferably tetrahydropyranyl group.

The monocyclic heterocyclic group of $R^2$, $R^3$, $R^{2a}$, and $R^{3a}$ is preferably monocyclic heteroaryl and a monocyclic oxygen-containing saturated heterocycle, and more preferably, it includes furyl, thienyl, pyridyl, tetrahydrofuryl, tetrahydropyranyl, and 1,4-dioxanyl group.
The heterocyclic group of X is preferably a monocyclic heterocyclic group, and specifically, it includes thienyl, pyridyl, furyl, isoxazolyl, morpholinyl, pyrrolidinyl, piperidyl, oxiranyl, oxetanyl, tetrahydrofuryl, and tetrahydropyranyl group, and more preferably, thienyl, piperidyl, and tetrahydropyranyl group.
The groups represented by $R^5$, $R^6$, and $R^7$ preferably include H, lower alkyl, halogen, -CN, $-NO_2$, $-OR^a$, $-NR^aR^b$, -S$(O)_p$-lower alkyl, $-C(O)R^a$, lower alkylene-$OR^a$, and lower alkylene-$NR^aR^b$, and more preferably, H, lower alkyl, halogen, and lower alkylene-$OR^a$.
The groups represented by $R^8$ and $R^9$ preferably include H, lower alkyl, halogen, lower alkylene-$OR^a$, and lower alkylene-$NR^aR^b$.
Y and Z: the same as or different from each other, each representing a bond, lower alkylene, or lower alkylene-O-.)

Preferred embodiments in the compound of the general formula (I) that is an active ingredient of the pharmaceutical of the present invention are the following compounds of the (1A) to (1F), and the compounds represented by the aforementioned general formulae (I') and (I").

(1A) A compound, wherein A is a benzene ring.
(1B) The compound of (1A) above, wherein B is a benzene ring.
(1C) The compound of (1B) above, wherein $R^4$ is -L-X.
(1D) The compound of (1C) above, wherein L is a bond or $C_{1-4}$ alkylene, and X is a monocyclic heterocyclic group, phenyl, or cycloalkyl.
(1E) The compound of (1D) above, wherein X is a monocyclic heterocyclic group.
(1F) The compound of (1B) above, wherein both A and B are benzene rings, and $R^4$ is lower alkyl or $-C(O)R^a$.

Specific compound included in the general formula (I) is preferably a compound selected from the following group.
9-cyclohexyl-N-(diaminomethylene)-9H-carbazole-2-carboxamide, N-(diaminomethylene)-9-piperidin-4-yl-9H-carbazole-2-carboxamide, 9-cyclobutyl-N-(diaminomethylene)-9H-carbazole-2-carboxamide, N-(diaminomethylene)-9-(tetrahydro-2H-pyran-4-yl)-9H-carbazole-2-carboxamide, 9-acetyl-N-(diaminomethylene)-9H-carbazole-2-carboxamide, 9-benzyl-N-(diaminomethylene)-9H-carbazole-2-carboxamide, 5-chloro-N-(diaminomethylene)-9-isopropyl-9H-carbazole-2-carboxamide, and N-(diaminomethylene)-5-(hydroxymethyl)-9-isopropyl-9H-carbazole-2-carboxamide.

Preferred embodiments in the compound represented by the general formula (I') of the present invention are the following compounds.

(2A) A compound, wherein A is a benzene ring.
(2B) The compound of (2A) above, wherein B is a benzene ring.
(2C) The compound of (2B) above, wherein $R^4$ is -L-X.
(2D) The compound of (2C) above, wherein L is a bond or $C_{1-4}$ alkylene, and X is a monocyclic heterocyclic group, phenyl, cycloalkyl, or cycloalkenyl, wherein the monocyclic heterocyclic group, phenyl, cycloalkyl, or cycloalkenyl may be substituted with halogen, low alkyl, or $-OR^a$.

(2E) The compound of (2D) above, wherein X is a monocyclic heterocyclic group.
(2F) The compound of (2B) above, wherein both A and B are benzene rings, and $R^4$ is lower alkyl.
(2G) The compound of (2E) or (2F) above, wherein Y is a bond, both of $R^1$ and $R^2$ are H, Z is a bond, lower alkylene, or lower alkylene-O-, and $R^3$ is $-OR^a$, phenyl, or cycloalkyl, and wherein phenyl and cycloalkyl may be substituted with lower alkyl or $-OR^a$.

Specific compound included in the general formula (I') is preferably a compound selected from the following group. N-[amino(methylamino)methylene]-9-(tetrahydro-2H-pyran-4-yl)-9H-carbazole-2-carboxamide, N-{amino[(3-methoxypropyl)amino]methylene}-9-(tetrahydro-2H-pyran-4-yl)-9H-carbazole-2-carboxamide, N-{amino[(cyclopropylmethyl)amino]methylene}-9-(tetrahydro-2H-pyran-4-yl)-9H-carbazole-2-carboxamide, N-{amino[(4-methoxybenzyl)amino]methylene}-9-(tetrahydro-2H-pyran-4-yl)-9H-carbazole-2-carboxamide, N-{amino[(3-methoxybenzyl)amino]methylene}-9-(tetrahydro-2H-pyran-4-yl)-9H-carbazole-2-carboxamide, and N-{amino[(2,6-dimethoxybenzyl)amino]methylene}-9-(tetrahydro-2H-pyran-4-yl)-9H-carbazole-2-carboxamide.

Preferred embodiments in the compound of the present invention represented by the general formula (I") are a compound in which $R^{4b}$ is cyclohexyl or cyclohexenyl substituted with halogen, or thienylmethyl. Specific compound included in the general formula (I') is preferably a compound selected from the following group. N-(diaminomethylene)-5-fluoro-9-(tetrahydro-2H-pyran-4-yl)-9H-carbazole-2-carboxamide, N-(diaminomethylene)-4-methyl-9-(tetrahydro-2H-pyran-4-yl)-9H-carbazole-2-carboxamide, N-(diaminomethylene)-9-(4,4-difluorocyclohexyl)-9H-carbazole-2-carboxamide, N-(diaminomethylene)-9-(2-thienylmethyl)-9H-carbazole-2-carboxamide, N-(diaminomethylene)-5-fluoro-4-methyl-9-(tetrahydro-2H-pyran-4-yl)-9H-carbazole-2-carboxamide, N-(diaminomethylene)-4,5-difluoro-9-(tetrahydro-2H-pyran-4-yl)-9H-carbazole-2-carboxamide, and N-(diaminomethylene)-9-(4-fluorocyclohex-3-en-1-yl)-5-methyl-9H-carbazole-2-carboxamide.

A further embodiment in the compound of the general formula (I) that is an active ingredient of the pharmaceutical of the present invention is a compound represented by the general formula represented by the formula (I"), in which the symbols have the following meanings.

$R^{4b}$: isopropyl, tetrahydropyranyl, piperidyl, cyclohexyl, cyclohexenyl, phenyl, thienyl, pyridyl, thienylmethyl, or isoxazolylmethyl, wherein the piperidyl group may be substituted with cyanomethyl or phenyl, and the other groups may be substituted with one or two groups selected from the group consisting of F, -O-methyl, and methyl,
$R^{5b}$: H, lower alkyl, -OH, -S-lower alkyl, halogen, lower alkylene-OH, or lower alkylene-O-lower alkyl, and
$R^{8b}$: lower alkyl, halogen, or lower alkylene-OH.

Further, the compound represented by the general formula (I) that is an active ingredient of the pharmaceutical of the present invention (which is hereinafter simply referred to the compound (I)) may in some cases exist in the form of other tautomers or geometrical isomers depending on the kinds of substituent. In the present specification, the compound can be described in only one form of an isomer, but the present invention includes the isomers, the isolated forms of the isomers, or a mixture of these isomers. For example, in the acylguanidine moiety of the compound (I), two isomers that are different in the position of the double bond may exist as shown in the following scheme. Furthermore, in each of the isomers, an E-isomer and a Z-isomer may exist depending on the geometric configurations of the double bonds. The present invention includes all of these isomers.

[Chem. 12]

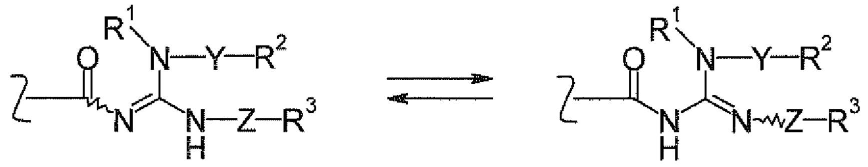

(the structure in the formula partially denotes the acylguanidine moiety of the compound (I). The bond denoted by a wavy line represents that either configuration of E and Z can be taken).
Furthermore, the present invention includes a pharmaceutically acceptable prodrug of the compound (I). The pharmaceutically acceptable prodrug refers to a compound having a group which can be converted into an amino group, OH, $CO_2H$ and the like, by solvolysis or under a physiological condition. Examples of the group to form a prodrug include the groups as described in Prog. Med., 5, 2157-2161 (1985), or "Iyakuhin no Kaihatsu (Pharmaceutical Research and Development, Drug Design)" (Hirokawa Publishing Company, 1990), vol. 7, Bunshi Sekkei (Molecular Design), pp.

163-198.

Moreover, the compound (I) may form a salt with an acid or a base, depending on the kinds of the substituents, and this salt is included in the present invention, as long as it is a pharmaceutically acceptable salt. Specifically, examples thereof include acid addition salts with inorganic acids such as hydrochloric acid, hydrobromic acid, hydroiodic acid, sulfuric acid, nitric acid, phosphoric acid and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid and the like, and salts with inorganic bases such as sodium, potassium, magnesium, calcium, aluminum and the like, and organic bases such as methylamine, ethylamine, ethanolamine, lysine, ornithine and the like, ammonium salts, and others.

Furthermore, the compound (I) and a salt thereof also include various hydrates or solvates, and polymorphic crystal substances. Also, the compound (I) and a salt thereof include the compounds labeled with various radioactive isotopes or non-radioactive isotopes.

(Production Process)

The compound (I) may be prepared by applying various known synthetic methods, using the characteristics based on their basic skeletons or the kinds of substituent. Here, depending on the kinds of functional groups, it is in some cases effective from the viewpoint of the preparation techniques to substitute the functional group with an appropriate protecting group (a group which is easily capable of being converted into the functional group) during the steps from starting materials to intermediates. Examples of such a functional group include an amino group, a hydroxyl group, a carboxyl group and the like, and examples of a protecting group thereof include those as described in "Protective Groups in Organic Synthesis (3rd edition, 1999)", edited by Greene and Wuts, which may be appropriately selected and used depending on the reaction conditions. In these methods, a desired compound can be obtained by introducing the protecting group to carry out the reaction, and then, if desired, removing the protecting group.

In addition, the prodrug of the compound (I) can be prepared by introducing a specific group during the steps from starting materials to intermediates, in the same manner as for the protecting groups mentioned above, or by carrying out the reaction with the compound (I) obtained. The reaction can be carried out by employing a method known to a person skilled in the art, such as common esterification, amidation, dehydration and the like.

Hereinbelow, the representative production processes of the compound of the present invention are described. Each of the production processes may also be carried out with reference to References appended in the present description. Further, the production processes of the present invention are not limited to the examples as shown below.

(First Production Process)

[Chem. 13]

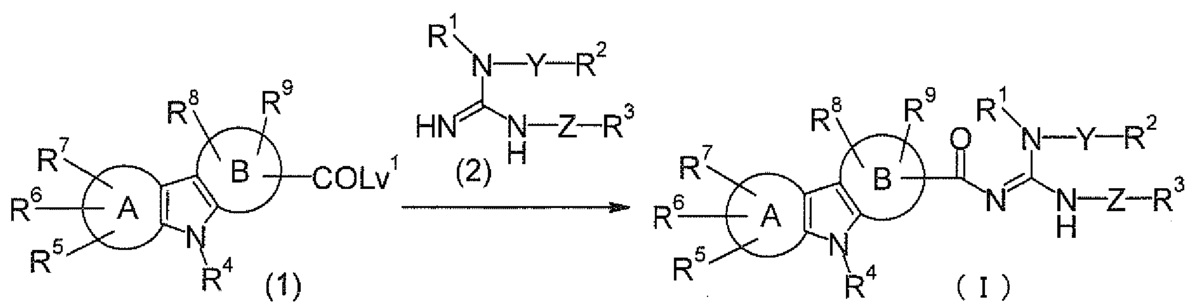

($Lv^1$ represents -OH or a leaving group.)

The compound (I) of the present invention can be prepared by subjecting a carboxylic acid or a reactive derivative thereof (1) and guanidine (2) or a salt thereof to amidation.

The reaction can be carried out using equivalent amounts of the carboxylic acid or a reactive derivative thereof (1) and guanidine (2), or in an excess amount of guanidine. It can be carried out under cooling or under heating, preferably at a temperature from -20˚C to 60˚C, in a solvent which is inert to the reaction, for example, aromatic hydrocarbons such

as benzene, toluene, xylene and the like, halogenated hydrocarbons, such as dichloromethane, 1,2-dichloroethane, chloroform and the like, ethers such as diethyl ether, tetrahydrofuran (THF), dioxane, dimethoxyethane (DME) and the like, N,N-dimethylformamide (DMF), dimethyl sulfoxide (DMSO), ethyl acetate (EtOAc), acetonitrile, water and the like, or a mixed liquid thereof.

If a free carboxylic acid wherein $Lv^1$ is OH is used as the starting compound (1), it is preferable to carry out the reaction in the presence of a condensing agent. Examples of the condensing agent in this case include N,N'-dicyclohexylcarbodiimide (DCC), 1-[3-(dimethylamino)propyl]-3-ethylcarbodiimide (WSC), 1,1'-carbonyldiimidazole (CDI), 2-(1H-benzotriazol-1-yl)-1,1,3,3-tetramethyluronium hexafluorophosphate (HBTU), diphenyl phosphoryl azide (DPPA), phosphorous oxychloride and the like. In some cases, it is preferable to further use an additive agent (for example, N-hydroxysuccinimide (HONSu), 1-hydroxybenzotriazole (HOBt) or the like). Relative to the carboxylic acid, an equivalent amount or excess amount of the condensing agent is usually used.

Examples of the reactive derivative of carboxylic acid wherein $Lv^1$ is a leaving group in the starting compound (1) include an acid halide (acid chloride, acid bromide or the like), an acid anhydride (a mixed acid anhydride with phenyl chloroformate, p-toluenesulfonic acid, isovaleric acid or the like or symmetric acid anhydride), an active ester (an ester which can be prepared using phenol that may be substituted with an electron withdrawing group such as a nitro group, a fluorine atom or the like, HOBt, HONSu and the like), a lower alkyl ester and the like, and any of them can be prepared from carboxylic acid using a reaction that is apparent to those skilled in the art. Depending on the kinds of reactive derivatives, it is sometimes advantageous for the smooth progress of the reaction to carry out the reaction in the presence of a base (organic bases such as triethylamine, diisopropylethylamine (DIPEA), N-methylmorpholine, pyridine, 4-(N,N-dimethylamino)pyridine and the like, inorganic bases such as sodium bicarbonate and the like, etc.). Pyridine can also serve as a solvent. Further, when a lower alkyl ester is used as the reactive derivative, it is preferable to carry out the reaction at room temperature or under heating under reflux.

(Second Production Process)

[Chem. 14]

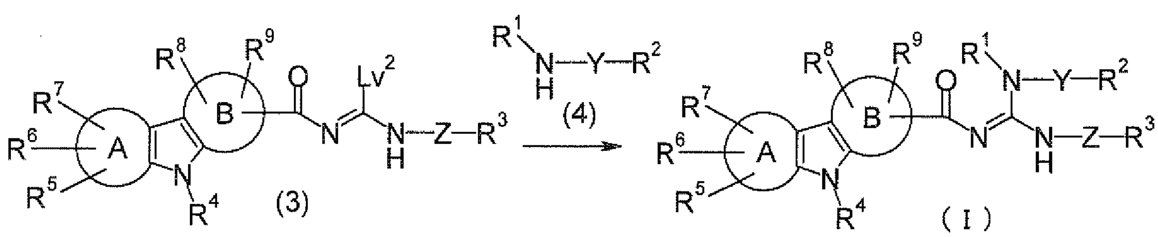

($Lv^2$ represents a leaving group such as pyrazol-1-yl which may be substituted with lower alkyl, or -S-lower alkyl, -O-phenyl, -Br,-Cl and the like).

The compound (I) of the present invention can be prepared by reacting an amidine compound (3) having a leaving group with an amine compound (4).

In this reaction, the compound (3) and the compound (4) are used in equivalent amounts, or either thereof in an excessive amount is used, and the mixture thereof is stirred under cooling to heating under reflux, preferably at a temperature from 0˚C to 80˚C, usually for 0.1 hour to 5 days, in a solvent which is inert to the reaction or without a solvent. Examples of the solvent as used herein are not particularly limited to but include aromatic hydrocarbons, ethers, halogenated hydrocarbons, DMF, DMSO, ethyl acetate, acetonitrile, and a mixture thereof. It is sometimes advantageous for the smooth progress of the reaction to carry out the reaction in the presence of an organic base such as triethylamine, N,N-diisopropylethylamine, N-methylmorpholine and the like, or an inorganic bases such as potassium carbonate, sodium carbonate, potassium hydroxide and the like.

(Third Production Process or Other Production Processes)

The compounds of the present invention having various functional groups such as an amino group, a carboxyl group, an amido group, a hydroxyl group, an alkylamino group and the like can be easily synthesized by those methods which are apparent to a skilled person in the art or a modified method thereof using the compound of the present invention having a corresponding nitro group, ester group, carboxyl group, amino group or the like as the starting materials. For

example, these can be prepared by the following reactions.

3-a: Reduction (1)

A compound having an amino group can be prepared by reducing a compound having a nitro group. For example, the reaction can be carried out using a hydrogenation reaction which uses palladium-carbon, Raney nickel or the like as a catalyst.

3-b: Reduction (2)

A compound having a hydroxyl group can be prepared by reducing a compound having a carbonyl group. For example, the reaction can be carried out using lithium aluminum hydride, sodium borohydride or the like as a reducing agent.

3-c: Hydrolysis

A compound having a carboxyl group or a hydroxyl group can be prepared by hydrolyzing a compound having an ester group. For example, this can be carried out in accordance with the deprotection reaction described in the aforementioned "Protective Groups in Organic Synthesis".

3-d: Amidation

A compound having an amide group can be prepared by the amidation of a compound having a carboxyl group or an amino group. This can be carried out in accordance with the aforementioned First Production Process.

3-e: Alkylation

A compound having an alkylamino group can be prepared by alkylating a compound having an amino group. As the alkylation reaction, the reaction can be carried out by a general method using various alkylating agents (for example, an alkyl halide, an alkyl sulfonic ester and the like). In addition, a compound having an alkylamino group can be prepared by carrying out reductive alkylation of a compound having an amino group with a carbonyl compound. The method described in "Jikken Kagaku Koza (Cources in Experimental Chemistry) (vol. 20) Yuki Gosei (Organic Synthesis) 2", edited by The Chemical Society of Japan, 4th edition, Maruzen, 1992, p. 300; or the like can be applied to the reaction.

3-f: Fluorination

A compound having a fluoro group can be prepared by treating a compound having a carbonyl group or a hydroxyl group with a fluorination reagent. Examples of the fluorination reagent include diethylaminosulfur trifluoride (DAST).

(Preparation of starting compounds)

The starting compounds (1) to (4) in the Production Processes as described above can be produced, for example, by the following method, a conventionally known method, or a modified method thereof.

(Starting Material Synthesis 1)

[Chem. 15]

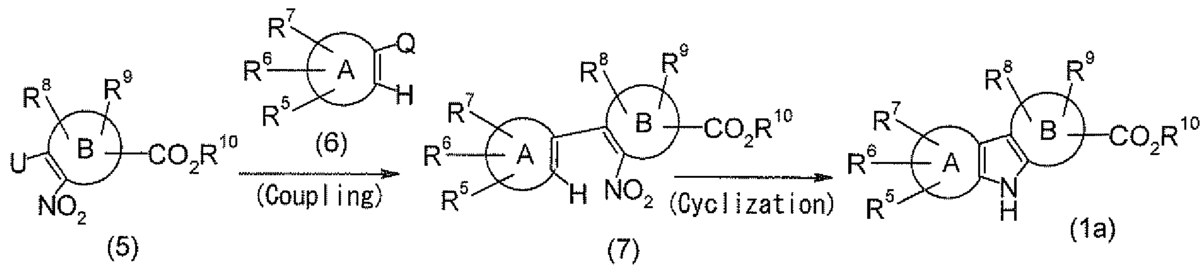


(wherein Q and U each represent a leaving group, and either thereof represents -Br, -Cl, -I or -O-$SO_2$-$CF_3$ or the like, and the other represents -$B(OH)_2$ or B(O-lower alkyl)$_2$ or the like. $R^{10}$ represents a protective group of a carboxyl group, such as lower alkyl, benzyl and the like),

Among the starting compounds (1), the compound in which $R^4$ is H can be prepared directly by the above reaction pathway, or by converting -$OR^{10}$ of thus prepared compound (1a) to a leaving group.

Here, the coupling reaction can be carried out by the methods described in "Synthetic Communications", (England), 1981, vol. 11, p. 513-519, "Synlett", (Germany), 2000, vol. 6, p. 829-831, or "Chemistry Letters", 1989, p. 1405-1408. The cyclization reaction can be carried out at room temperature or under heating in a solvent such as benzene, toluene and the like, or without a solvent, using triethyl phosphite, triphenylphosphine or the like.

(Starting Material Synthesis 2)

[Chem. 16]

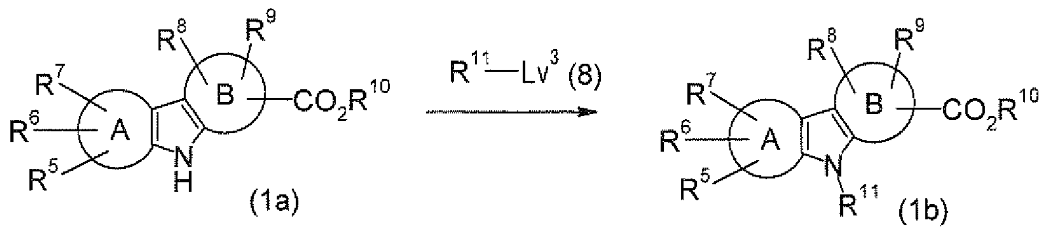

(Lv$^3$ represents a leaving group such as halogen, -O-methanesulfonyl, -O-p-toluenesulfonyl or the like, or -OH. $R^{11}$ represents a group other than H in $R^4$.)

Among the starting compounds (1), the compound in which $R^4$ is not H, namely $R^{11}$, can be prepared from the compound (1a) by the reaction such as alkylation, acylation, sulfonylation and the like by the compound (8), or by converting -$OR^{10}$ of thus prepared compound (1b) to a leaving group.

For the alkylation, in case where the compound (8) in which Lv$^3$ is a leaving group is used, the reaction can be carried out using a base such as sodium hydride, potassium hydride, potassium tert-butoxide and the like. In particular, in case where the compound (8) in which $R^{11}$ is aryl or heteroaryl, and Lv$^3$ is a leaving group is used, a typical coupling method can be used, and it may be carried out, for example, in accordance with the methods as described in "the Journal of the American Chemical Society", (US), 2001, Vol. 123, p. 7727. Further, in case where the compound (8) in which Lv$^3$ is -OH is used, the reaction can be carried out using a conventional method for the Mitsunobu reaction, and it may be carried out, for example, using the methods as described in "Tetrahedron Letters", (Netherlands), 2002, Vol. 43, p. 2187.

Regarding the acylation or the sulfonylation, the reaction can be carried out using an acid halide in which the leaving group of Lv$^3$ is halogen or the like as the compound (8), in the presence of a base such as potassium hydride, potassium tert-butoxide and the like.

Each of the products of the above-described Production Processes can be induced into corresponding carboxyl compounds by the deprotection of the $-CO_2R^{10}$ group. For example, the deprotection reaction described in the abovementioned "Protective Groups in Organic Synthesis" can be used.

(Starting Material Synthesis 3)

[Chem. 17]

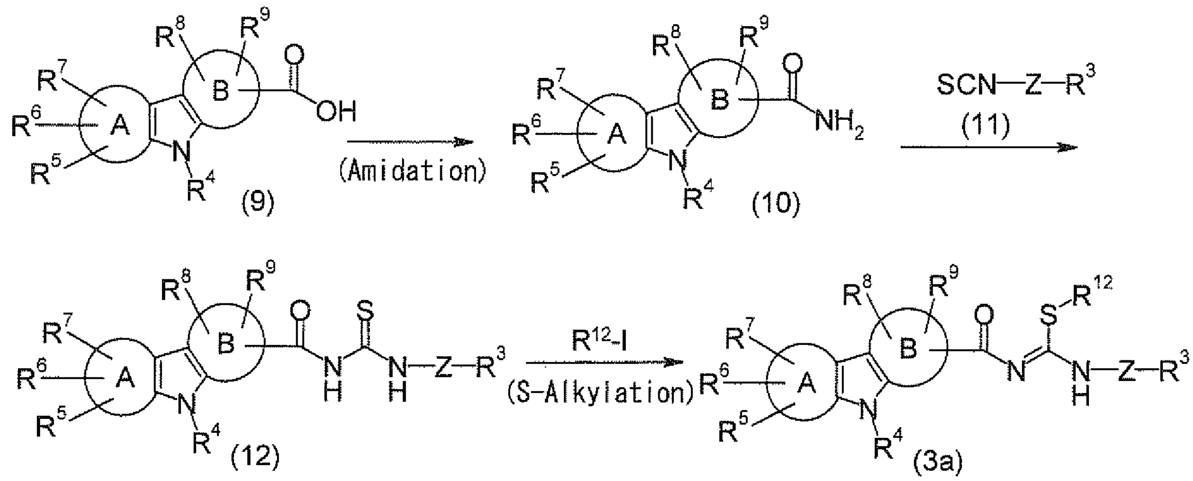


($R^{12}$ represents lower alkyl).
Among the starting compounds (3), the compound (3a) in which $Lv^2$ is -S-lower alkyl can be prepared by the above reaction pathway.
Here, the amidation can be carried out by condensation with ammonia or an equivalent thereof as in the First Production Process. A reaction for preparing an acylthiourea (12) from an amide (10) and a thioisocyanate (11) can be carried out by treatment with a base such as sodium hydride and the like at room temperature in a solvent that is inert to the reaction, such as DMF and the like.
The S-alkylation can be carried out using a conventional method, and it may be carried out, for example, in accordance with the methods as described in "Journal of Medicinal Chemistry", (US), 2005, Vol. 48, p. 1540.

The compound (I) thus prepared is isolated and purified as a free compound, a pharmaceutically acceptable salt, a hydrate, a solvate thereof, or a polymorphic crystal substance thereof. The pharmaceutically acceptable salt of the compound (I) can be prepared by a salt formation treatment within conventional technology by a skilled person in the art.
The isolation and purification can be carried out by employing common chemical operations such as extraction, fractional crystallization, various types of fractional chromatography and the like.
Various isomers can be separated by selecting a suitable starting compound, or by making use of the difference in the physicochemical properties between isomers. For example, optical isomers can be lead into each stereochemically pure isomer by means of general optical resolution methods (for example, fractional crystallization after forming diastereomeric salts with optically active bases or acids, chromatography using a chiral column and the like, etc.). In addition, an isomer can also be prepared from an appropriate optically active starting material.

Examples

Hereinbelow, the methods for preparing the compound included in the formula (I) that is an active ingredient of the present invention are described with reference to Examples. Further, the methods for preparing the compound used as a starting material are described with reference to Production Examples. Furthermore, the methods for preparing the compound (I) are not limited to the specific production processes of the Examples below, and thus, the compounds can be prepared by a combination of these preparation methods, a known production method, or a modified method

thereof.
The following abbreviations are used for the analytical data of mass spectroscopy in the description of Production Examples and Tables as below.
ESI+: ESI-MS$[M+H]^+$; ESI-: ESI-MS$[M-H]^-$; FAB+: FAB-MS$[M+H]^+$ or FAB-MS$[M]^+$; FAB-: FAB-MS$[M-H]^-$; APCI+: APCI-MS$[M+H]^+$; APCI-: APCI-MS$[M-H]^-$; EI+: EI$[M]^+$.

Production Example 1

Methyl 2-nitrobiphenyl-4-carboxylate was obtained by allowing methyl 3-nitro-4-{[(trifluoromethyl)sulfonyl]oxy} benzoate with phenyl boric acid, potassium phosphate, and tetrakistriphenylphosphine palladium to undergo the reaction in DMF under heating. FAB+: 258.

Production Example 2

Methyl 9H-carbazole-2-carboxylate was obtained by allowing methyl 2-nitrobiphenyl-4-carboxylate and triethyl phosphite to undergo the reaction under heating. FAB+: 226.

Production Example 3

Methyl 9-isopropyl-9H-carbazole-2-carboxylate was obtained by allowing methyl 9H-carbazole-2-carboxylate, 2-propanol, and (tributylphosphoranylidene)acetonitrile to undergo the reaction in toluene under heating. ESI+: 268.

Production Example 4

9-Isopropyl-9H-carbazole-2-carboxylic acid was obtained by allowing methyl 9-isopropyl-9H-carbazole-2-carboxylate and, a 1 M aqueous sodium hydroxide solution to undergo the reaction in ethanol under heating. ESI-: 252.

Production Example 5

Methyl 5-bromomethyl-9-isopropyl-9H-carbazole-2-carboxylate was obtained by allowing methyl 9-isopropyl-5-methyl-9H-carbazole-2-carboxylate, N-bromosuccinimide, and 2,2'-azobisisobutyronitrile to undergo the reaction in carbon tetrachloride under heating. FAB+: 360, 362.

Production Example 6

Methyl 5-dimethylaminomethyl-9-isopropyl-9H-carbazole-2-carboxylate was obtained by allowing methyl 5-bromomethyl-9-isopropyl-9H-carbazole-2-carboxylate, dimethylamine (2 M, a methanol solution), and potassium carbonate to undergo the reaction in THF at room temperature. FAB+: 325.

Production Example 7

Methyl 5-acetoxymethyl-9-isopropyl-9H-carbazole-2-carboxylate was obtained by allowing methyl 5-bromomethyl-9-isopropyl-9H-carbazole-2-carboxylate and potassium acetate to undergo the reaction in DMF at room temperature. EI+: 339.

Production Example 8

Methyl 5-hydroxymethyl-9-isopropyl-9H-carbazole-2-carboxylate was obtained by allowing methyl 5-acetoxymethyl-9-isopropyl-9H-carbazole-2-carboxylate and potassium carbonate to undergo the reaction in methanol-THF at room temperature. FAB+; 297.

Production Example 9

Methyl 9-isopropyl-5-methoxymethyl-9H-carbazole-2-carboxylate was obtained by allowing methyl 5-hydroxymethyl-9-isopropyl-9H-carbazole-2-carboxylate, methyl iodide, and silver oxide to undergo the reaction in acetonitrile under heating. FAB+: 311.

Production Example 10

Benzyl 9-isobutyryl-9H-carbazole-2-carboxylate was obtained by allowing benzyl 9H-carbazole-2-carboxylate and 2-methylpropionyl chloride to undergo the reaction in DMF in the presence of sodium hydride at room temperature. ESI+: 372.

Production Example 11

9-Isobutyryl-9H-carbazole-2-carboxylic acid was obtained by allowing benzyl 9-isobutyryl-9H-carbazole-2-carboxylate and palladium-carbon to undergo the reaction in ethanol-DMF at room temperature under a hydrogen gas atmosphere. ESI+: 282.

Production Example 12

Methyl 9-isopropyl-6-nitro-9H-carbazole-2-carboxylate was obtained by allowing methyl 9-isopropyl-9H-carbazole-2-carboxylate and concentrated nitric acid to undergo the reaction in acetic acid at room temperature. FAB+: 313.

Production Example 13

Methyl 5-formyl-9-isopropyl-9H-carbazole-2-carboxylate was obtained by allowing methyl 5-hydroxymethyl-9-isopropyl-9H-carbazole-2-carboxylate and manganese dioxide to undergo the reaction in chloroform at room temperature. FAB+: 296. Production Example 14
9-Methyl-9H-carbazole-2-carboxylic acid was obtained by allowing methyl 9H-carbazole-2-carboxylate, methyl iodide, and potassium hydroxide to undergo the reaction in DMF at room temperature. FAB+: 226.

Production Example 15

Ethyl 9-ethyl-9H-carbazole-2-carboxylate was obtained by allowing methyl 9H-carbazole-2-carboxylate, ethyl iodide, and potassium hydroxide to undergo the reaction in DMF under heating. ESI+: 268.

Production Example 16a and Production Example 16b

A mixture of 2,3,4,9-tetrahydro-1H-carbazole-7-carbaxylic acid and 2,3,4,9-tetrahydro-1H-carbazole-5-carboxylic acid was obtained by allowing cyclohexanone and 3-hydrazinobenzoic acid to undergo the reaction in acetic acid under heating. This mixture was separated and purified by silica gel column chromatography to obtain 2,3,4,9-tetrahydro-1H-carbazole-5-carboxylic acid [Production Example 16a: FAB+: 216], 2,3,4,9-tetrahydro-1H-carbazole-7-carboxylic acid [Production Example 16b: FAB+: 216].

Production Example 17a and Production Example 17b

Methyl 2,3,4,9-tetrahydro-1H-carbazole-7-carboxylate [Production Example 17a: ESI+: 230] and methyl 2,3,4,9-tetrahydro-1H-carbazole-5-carboxylate [Production Example 17b: ESI+: 230] were prepared by adding thionyl chloride to a methanol solution of a mixture of 2,3,4,9-tetrahydro-1H-carbazole-7-carboxylic acid and 2,3,4,9-tetrahydro-1H-carbazole-5-carboxylic acid at -10˚C, followed by reaction under heating, and then separation and purification by column chromatography.

Production Example 18

3-{2-[1-(Ethoxycarbonyl)piperidin-4-ylidene]hydrazino} benzoic acid was obtained by allowing ethyl 4-oxopiperidine-1-carboxylate and 3-hydrazinobenzoic acid to undergo the reaction in acetic acid under heating. ESI+: 306.

Production Example 19

A mixture of diethyl 1,3,4,5-tetrahydro-2H-pyrido[4,3-b]indole-2,7-dicarboxylate and diethyl 1,3,4,5-tetrahydro-2H-pyrido[4,3-b]indole-2,9-dicarboxylate was obtained by allowing 3-{2-[1-(ethoxycarbanyl)piperidin-4-ylidene]hydrazino}benzoic acid and concentrated hydrochloric acid to undergo the reaction in ethanol under heating. ESI+: 317.

Production Example 20

A mixture of 2-(ethoxycarbonyl)-2,3,4,5-tetrahydro-1H-pyrido[4,3-b]indole-7-carboxylic acid and 2-(ethoxycarbonyl)-2,3,4,5-tetrahydro-1H-pyrido[4,3-b]indole-9-carboxylic acid was obtained by allowing a mixture of diethyl 1,3,4,5-tetrahydro-2H-pyrido[4,3-b]indole-2,7-dicarboxylate and diethyl 1,3,4,5-tetrahydro-2H-pyrido[4,3-b]indole-2,9-dicarboxylate, and potassium hydroxide to undergo the reaction in methanol-water under heating. ESI-: 287.

Production Example 21

3-Fluoro-4-hydroxy-5-nitrobenzoic acid was obtained by allowing 3-fluoro-4-hydroxybenzoic acid and fuming nitric acid to undergo the reaction in concentrated sulfuric acid at -5˚C to room temperature.

Production Example 22

Ethyl 3-fluoro-4-hydroxy-5-nitrobenzoate was obtained by allowing 3-fluoro-4-hydroxy-5-nitrobenzoic acid and concentrated sulfuric acid to undergo the reaction in ethanol under heating.

Production Example 23

Ethyl 3-fluoro-5-nitro-4-{[(trifluoromethyl)sulfonyl]oxy}benzoate was obtained by allowing ethyl 3-fluoro-4-hydroxy-5-nitrobenzoate, pyridine, and trifluoromethanesulfuric anhydride to undergo the reaction in dichloromethane at 0˚C to room temperature.

Production Example 24

9-(Tetrahydro-2H-pyran-4-yl)-9H-carbazole-2-carboxamide was obtained by allowing 9-(tetrahydro-2H-pyran-4-yl)-9H-carbazole-2-carboxylic acid, thionyl chloride, and DMF to undergo the reaction, and then to undergo the reaction with an aqueous ammonia solution at room temperature.

Production Example 25

N-[(Methylamino)carbonothioyl]-9-(tetrahydro-2H-pyran-4-yl)-9H-carbazole-2-carboxamide was obtained by performing the reaction with methylthioisocyanate in a mixed solution of 9-(tetrahydro-2H-pyran-4-yl)-9H-carbazole-2-carboxamide and NaH in DMF at room temperature.

Production Example 26

N-Methyl-N'-{[9-(tetrahydro-2H-pyran-4-yl)-9H-carbazole-2-yl]carbonyl}imidethiocarbamate was obtained by allowing N-[(methylamino)carbonothioyl]-9-(tetrahydro-2H-pyran-4-yl)-9H-carbazole-2-carboxamide and methyl iodide to undergo the reaction in THF under heating.

Production Example 27

Methyl 9-phenyl-9H-carbazole-2-carboxylate was obtained by allowing methyl 9H-carbazole-2-carboxylate, potassium phosphate, copper iodide, (1R,2R)-1,2-cyclohexanediamine, and iodobenzene to undergo the reaction in dioxane under heating.

Production Example 28

Methyl 9-(1-methylpiperidin-4-yl)-9H-carbazole-2-carboxylate was obtained by allowing methyl 9-piperidin-4-yl-9H-carbazole-2-carboxylate hydrochloride, formaldehyde, triacetoxy sodium borohydride, and acetic acid to undergo the reaction in dichloromethane at room temperature.

Production Example 29

Methyl 9-(1-acetylpiperidin-4-yl)-9H-carbazole-2-carboxylate was obtained by allowing methyl 9-piperidin-4-yl-9H-carbazole-2-carboxylate hydrochloride, acetyl chloride, and DIPEA to undergo the reaction in dichloromethane at room temperature.

Production Example 30

Methyl 9-[1-(methanesulfonyl)piperidin-4-yl]-9H-carbazole-2-carboxylate was obtained by allowing methyl 9-piperidin-4-yl-9H-carbazole-2-carboxylate hydrochloride, methanesulfonyl chloride, and DIPEA to undergo the reaction in dichloromethane at room temperature.

Production Example 31

Methyl 9-[1-(methoxycarbonyl)piperidin-4-yl]-9H-carbazole-2-carboxylate was obtained by performing the reaction with ethyl chloroformate in a mixed solution of methyl 9-piperidin-4-yl-9H-carbazole-2-carboxylate hydrochloride and DIPEA in dichloromethane at room temperature.

Production Example 32

Methyl 9-(4-oxocyclohexyl)-9H-carbazole-2-carboxylate was obtained by allowing a mixed solution of methyl 9-(1,4-dioxaspiro[4,5]dec-8-yl)-9H-carbazole-2-carboxylate, 1 M hydrochloric acid, THF, and ethanol to undergo the reaction at room temperature.

Production Example 33

Methyl 9-(trans-4-hydroxycyclohexyl)-9H-carbazole-2-carboxylate and methyl 9-(cis-4-hydroxycyclohexyl)-9H-carbazole-2-carboxylate were obtained by allowing methyl 9-(4-oxocyclohexyl)-9H-carbazole-2-carboxylate and sodium borohydride to undergo the reaction in methanol and THF at 0˚C.

Production Example 34

Methyl 9-(4,4-difluorocyclohexyl)-9H-carbazole-2-carboxylate was obtained by allowing methyl 9-(4-oxocyclohexyl)-9H-carbazole-2-carboxylate and diethylaminosulfur trifluoride to undergo the reaction in dichloromethane at room temperature.

Production Example 35

Methyl 9-(cis-4-methoxycyclohexyl)-9H-carbazole-2-carboxylate was obtained by allowing methyl 9-(cis-4-hydroxycyclohexyl)-9H-carbazole-2-carboxylate, methyl iodide, and NaH to undergo the reaction in THF at 0˚C.

Production Example 36

9-[2-Hydroxy-1-(hydroxymethyl)ethyl]-9H-carbazole-2-carboxylic acid was obtained by allowing methyl 9-[2-methoxy-1-(methoxymethyl)ethyl]-9H-carbazole-2-carboxylate and boron tribromide to undergo the reaction in dichloromethane at -78˚C to room temperature.

Production Example 37

Methyl 9-(1-benzylpiperidin-4-yl)-9H-carbazole-2-carboxylate was obtained by allowing methyl 9-piperidin-4-yl-9H-carbazole-2-carboxylate hydrochloride, benzyl bromide, and potassium carbonate to undergo the reaction in DMF under heating.

Production Example 38

Methyl 9-(1-phenylpiperidin-4-yl)-9H-carbazole-2-carboxylate was obtained by allowing methyl 9-piperidin-4-yl-9H-carbazole-2-carboxylate hydrochloride, tris(dibenzylideneacetone)dipalladium (0), and (R)-(+)-2,2'-bis(diphenylphosphino)-1,1'-binaphthyl, bromobenzene to undergo the reaction in toluene under heating.

Production Example 39

Methyl 5-hydroxy-9-isopropyl-9H-carbazole-2-carboxylate was obtained by allowing methyl 5-(benzyloxy)-9-isopropyl-9H-carbazole-2-carboxylate and 10% palladium-carbon to undergo the reaction in methanol at room temperature in a hydrogen atmosphere.

Production Example 40

Methyl 9-(1,1-dioxidetetrahydro-2H-thiopyran-4-yl)-9H-carbazole-2-carboxylate was obtained by allowing methyl 9-(tetrahydro-2H-thiopyran-4-yl)-9H-carbazole-2-carboxylate and MCPBA to undergo the reaction in dichloromethane at room temperature.

Production Example 41

Methyl 2'-(dimethoxymethyl)-2-nitrobiphenyl-4-carboxylate was obtained by allowing methyl 2'-formyl-2-nitrobiphenyl-4-carboxylate and idodine to undergo the reaction in methanol under heating.

Production Example 42

Methyl 5-(acetoxymethyl)-9H-carbazole-2-carboxylate was obtained by allowing methyl 5-(hydroxymethyl)-9H-carbazole-2-carboxylate and acetic acid by performing the condensation using WSC hydrochloride and a catalytic amount of N,N-dimethylpyridine-4-amine in methylene chloride.
The compounds of Production Examples shown in the following Tables 1 to 24 were prepared in the same manner as the methods of Production Examples 1 to 42 above, using each corresponding starting materials. Further, the mass spectroscopic values of the compounds of Production Examples 21 to 42 are shown in Table 25, the mass spectroscopic values of the compounds of Production Examples 43 to 154 are shown in Tables 1 to 6, and the mass spectroscopic values of the compounds of Production Examples 155 to 405 are shown in Tables 25 to 27.

Example 1

To a solution of 140 mg of 9-isopropyl-9H-carbazole-2-carboxylic acid in 4 ml of DMF was added 134 mg of CDI, followed by stirring at 50˚C for 1 hour. After leaving it to be cooled to room temperature, 238 mg of guanidine carbonate was added thereto, followed by stirring at room temperature overnight. The solvent was removed by evaporation, water was added thereto, and the precipitated solid was purified by silica gel column chromatography (Chromatorex (registered trademark), methanol/chloroform) to obtain 157 mg of N-(diaminomethylene)-9-isopropyl-9H-carbazole-2-carboxamide as a pale yellow solid.

Example 2

To a solution of 573 mg of guanidine hydrochloride in 6.5 ml of DMF was added 192 mg of sodium hydride (60%), followed by stirring at room temperature for 1 hour. To this solution was added a solution of 270 mg of methyl 9H-carbazole-2-carboxylate in 6.5 ml of DMF, followed by stirring at 70˚C for 2.5 hours. After leaving it to be cooled to room temperature and removing the solvent by evaporation, water was added thereto and the precipitated solid was purified by Chromatorex (methanol/chloroform) to obtain 236 mg of N-(diaminomethylene)-9H-carbazole-2-carboxamide as a pale yellow solid.

Example 3

To a solution of 300 mg of N-(diaminomethylene)-9-[1-(diphenylmethyl)azetidin-3-yl]-9H-carbazole-2-carboxamide in 9 ml of ethanol were added 1.26 ml of 1 M hydrochloric acid and 30 mg of 20% palladium hydroxide, followed by stirring at room temperature under a hydrogen gas atmosphere for 4 days, A 1 M aqueous sodium hydroxide solution was added thereto, followed by filtration through Celite. The solvent was then removed by evaporation, and the residue was purified by Chromatorex (methanol/chloroform) to obtain 89 mg of 9-azetidin-3-yl-N-(diaminomethylene)-9H-carbazole-2-carboxamide,

Example 4

To a solution of 393 mg of N-(diaminomethylene)-9-[2-(benzyloxy)ethyl]-9H-carbazole-2-carboxamide in 9 ml of ethanol - 3 ml of THF were added 1.0 ml of 1 M hydrochloric acid and 40 mg of 10% palladium-carbon, followed by stirring at room temperature under a hydrogen gas atmosphere for 3 days. A 1 M aqueous sodium hydroxide solution was added thereto, followed by filtration through Celite. The organic solvent was then removed by evaporation, and the aqueous layer was extracted with chloroform, washed with brine, and dried over anhydrous magnesium sulfate. The solvent was removed by evaporation to obtain 140 mg of N-(diaminomethylene)-9-(2-hydroxyethyl)-9H-carbazole-2-carboxamide.

Example 5

To a solution of 106 mg of N-(diaminomethylene)-9-isopropyl-6-nitro-9H-carbazole-2-carboxamide in 5 ml of ethanol - 3 ml of THF was added 20 mg of 10% palladium-carbon, followed by stirring at room temperature under a hydrogen gas atmosphere for 4 hours. After filtration through Celite, the solvent was then removed by evaporation to obtain 128 mg of 6-amino-N-(diaminomethylene)-9-isopropyl-9H-carbazole-2-carboxamide.

Example 6

To a solution of 201 mg of tert-butyl 4-(2-{[(diaminomethylene)amino]carbonyl}-9H-carbazole-9-yl)piperidine-1-earboxylate that had been synthesized in the same manner as in Example 1 in 4.4 ml of ethanol was added 0.6 ml of 4 M hydrogen chloride/ethyl acetate, followed by stirring at room temperature overnight. The solid precipitated was collected by filtration, and washed with ethanol to obtain 125 mg of N-(diaminomethylene)-9-piperidin-4-yl-9H-carbazole-2-carboxamide dihydrochloride as a pale yellow solid.

Example 7

A solution of guanidine hydrochloride (882 mg) and sodium methoxide (499 mg) in methanol (4 mL) was stirred at room temperature for 1 hour, and the reaction liquid was concentrated under reduced pressure. To the resulting residue was added a mixed solution of 2,3,4,5-tetrahydro-1H-pyrido[4,3-b]indole (265 mg) that had been separately prepared and CDI (274 mg) in NMP (N-methylpyrrolidin-2-one) (8 mL), followed by stirring at 100˚C for 30 minutes under heating. The reaction liquid was returned to room temperature, diluted with water, and extracted with EtOAc. The organic layer was concentrated under reduced pressure, and the resulting residue was purified by silica gel column chromatography ("Chromatorex (registered trademark), NH2", chloroform/methanol = 100/0-90/10), and then made into its oxalate to obtain N-(diaminomethylene)-1,3,4,5-tetrahydro-2H-pyrido[4,3,-b]indole-2-carboxamide oxalate (187 mg).

Example 8

A mixed solution of 9-(tetrahydro-2H-pyran-4-yl)-9H-carbazole-2-carboxylic acid (300 mg), WSC hydrochloride (292 mg), and HOBt (96 mg) in DMF (10 mL) was stirred at room temperature for 5 minutes, and then 3,5-dimethyl-1H-pyrazol-1-carboxyimidamide nitrate (245 mg) and DIPEA (0.27 mL) were added thereto, followed by stirring at room temperature for an additional 19 hours. The reaction liquid was diluted with a saturated aqueous $NH_4Cl$ solution, and then extracted with EtOAc. The organic layer was concentrated under reduced pressure, and the resulting residue was purified by silica gel column chromatography (silica gel 60N, spherical, neutral, n-hexane/EtOAc = 5/2) to obtain N-[(1Z)-amino(3,5-dimethyl-1H-pyrazol-1-yl)methylene]-9-(tetrahydro-2H-pyran-4-yl)-9H-carbazole-2-carboxamide (450 mg).

Example 9

A mixed solution of N-[(3,5-dimethyl-1H-pyrazol-1-yl)(imino)methyl]-9-(tetrahydro-2H-pyran-4-yl)-9H-carbazole-2-carboxamide (250 mg) and piperazine (518 mg) in DMF (5 mL) was stirred at 80˚C for 6 hours under heating. The reaction liquid was returned to room temperature, diluted with water, and then extracted with EtOAc. The organic layer was concentrated under reduced pressure, and the resulting residue was purified by silica gel column chromatography ("Chromatorex (registered trademark), NH2", EtOAc), and then formed into its salt to obtain N-[(1Z)-amino(piperazin-1-yl)methylene]-9-(tetrahydro-2H-pyran-4-yl)-9H-carbazole-2-carboxamide dihydrochloride (90 mg).

Example 10

A mixed solution of methyl N-methyl-N'-{[9-(tetrahydro-2H-pyran-4-yl)-9H-carbazole-2-yl]carbonyl}imidethiocarbamate (172 mg), methylamine (335 mg), and DIPEA (0.78 mL) in DMF (30 mL) was stirred at 85˚C for 16 hours under heating. The reaction liquid was returned to room temperature, diluted with a saturated aqueous $NH_4Cl$ solution, and then extracted with EtOAc. The organic layer was concentrated under reduced pressure, and the resulting residue was purified by silica gel column chromatography (silica gel 60N, spherical, neutral, EtOAc), and then formed into its salt to obtain N-[bis(methylamino)methylene]-9-(tetrahydro-2H-pyran-4-yl)-9H-carbazole-2-carboxamide hydrochloride (95 mg).

Example 11

A solution of ethyl 4-fluoro-9-(tetrahydro-2H-pyran-4-yl)-9H-carbazole-2-carboxylate (260 mg) and a 1 M aqueous sodium hydroxide solution (3 mL) in methanol (10 mL) and THF (10 mL) was stirred at 60˚C for 3 hours under heating. The reaction liquid was concentrated under reduced pressure, and the resulting residue was then diluted with water. This was neutralized with 1 M hydrochloric acid (3 mL), and the precipitate was then collected by filtration, and dried under reduced pressure. The precipitate and CDI (165 mg) in DMF (30 mL) were stirred at room temperature for 15 minutes, and guanidine carbonate (735 mg) was then added thereto, followed by stirring at room temperature for an additional 20 hours. The reaction liquid was diluted with water, and then extracted with EtOAc, and the organic layer was concentrated under reduced pressure. The resulting residue was purified by silica gel column chromatography (silica gel 60N, spherical, neutral, chloroform/methanol/29% aqueous ammonia solution), and then formed into its salt to form N-(diaminomethylene)-4-fluoro-9-(tetrahydro-2H-pyran-4-yl)-9H-carbazole-2-carboxamide hydrochloride (137 mg).

The compounds of Examples shown in the following Tables 28 to 43 were prepared in the same manner as the methods of Examples 1 to 11 above, using each corresponding starting materials (provided that in Example 65, a starting material having the hydroxyl group protected with an acetyl group was used). The physical properties of the compounds of Examples 1 to 6 and 12 to 71 are shown in Tables 28 to 33, and the physical properties of the compounds of Examples of 7 to 11 and 72 to 227 are shown in Tables 44 to 51.

The following abbreviations are used in Tables below.

REx: Production Example number, Ex: Example number, No: compound number, Str: structural formula, Dat: physicochemical data (NMR: δ (ppm) of the characteristic peak in DMSO-$d_6$ by $^1$HNMR), ND: Not determined, Sal: salt (a blank or no description means that it is a free form and the numeral in front of the acid component means a molar ratio. For example, a description of 2HCl means that the compound is a dihydrochloride salt.), Oxal: oxalic acid, Me: methyl, Et: ethyl, nPr: normal propyl, cPr: cyclopropyl, iPr: isopropyl, nBu: normal butyl, tBu: tert-butyl, cBu: cyclobutyl, nPen: normal pentyl, cPen: cyclopentyl, cHex: cyclohexyl, Ph: phenyl, Bn: benzyl, Ac: acetyl, Ms: methanesulfonyl, Boc: tert-butoxycarbonyl, null: unsubstituted. The numeral in front of the substituted group means the position to be substituted, and for example, 5-F means 5-fluoro. RSyn and Syn: preparation method (the numeral shows that the compound was prepared using a corresponding starting material in the same manner as in the compound having its number as the Production Example number or Example number. A case in which two or more numerals are shown indicates that the compound was prepared by sequentially carrying out the same manner as in the Production Example or Example having the number.).

In the column "Syn" regarding the preparation method in Tables below, identical Example number is given to the each compound with various salt form which is prepared by a different salt forming process, but a same kind of the reaction.

[Table 1]

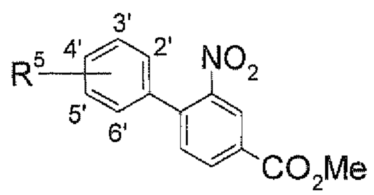

| REx | RSyn | $R^5$ | Dat | REx | RSyn | $R^5$ | Dat |
|---|---|---|---|---|---|---|---|
| 43 | 1 | 2'-F | FAB+ : 275 | 51 | 1 | 4'-OMe | FAB+ : 288 |
| 44 | 1 | 3'-F | FAB+ : 276 | 52 | 1 | 2'-Cl | FAB+ : 292 |
| 45 | 1 | 4'-F | FAB+ : 276 | 53 | 1 | 3'-Cl | FAB+ : 291 |
| 46 | 1 | 2'-Me | FAB+ : 272 | 54 | 1 | 4'-Cl | FAB+ : 292 |
| 47 | 1 | 3'-Me | FAB+ : 272 | 55 | 1 | 2'-CN | FAB+ : 283 |
| 48 | 1 | 4'-Me | FAB+ : 272 | 56 | 1 | 3'-CN | FAB+ : 283 |
| 49 | 1 | 2'-OMe | FAB+ : 288 | 57 | 1 | 4'-CN | FAB+ : 283 |
| 50 | 1 | 3'-OMe | FAB+ : 288 | | | | |

[Table 2]

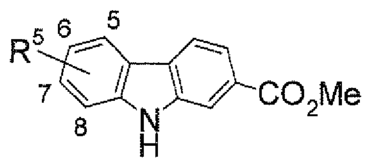

| REx | RSyn | $R^5$ | Dat | REx | RSyn | $R^5$ | Dat |
|---|---|---|---|---|---|---|---|
| 58 | 2 | 5-F | FAB+ : 244 | 68 | 2 | 7-OMe | FAB+ : 256 |
| 59 | 2 | 6-F | FAB+ : 244 | 69 | 2 | 8-OMe | FAB+ : 256 |
| 60 | 2 | 7-F | FAB+ : 244 | 70 | 2 | 5-Cl | FAB+ : 259 |
| 61 | 2 | 8-F | FAB+ : 244 | 71 | 2 | 6-Cl | FAB+ : 260 |
| 62 | 2 | 5-Me | FAB+ : 240 | 72 | 2 | 7-Cl | FAB+ : 260 |
| 63 | 2 | 6-Me | FAB+ : 240 | 73 | 2 | 8-Cl | FAB+ : 260 |
| 64 | 2 | 7-Me | FAB+ : 240 | 74 | 2 | 5-CN | FAB- : 249 |
| 65 | 2 | 8-Me | FAB+ : 240 | 75 | 2 | 6-CN | ESI- : 249 |
| 66 | 2 | 5-OMe | FAB+ : 256 | 76 | 2 | 7-CN | ESI- : 249 |
| 67 | 2 | 6-OMe | FAB+ : 255 | 77 | 2 | 8-CN | ESI- : 249 |

[Table 3]

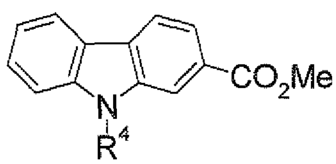

| REx | RSyn | $R^4$ | Dat | REx | RSyn | $R^4$ | Dat |
|---|---|---|---|---|---|---|---|
| 78 | 3 | nPr | ESI+ : 268 | 89 | 3 | cHex | ESI+ : 308 |
| 79 | 3 | nBu | ESI+ : 282 | 90 | 3 | $-CH(C_2H_5)_2$ | ESI+ : 296 |
| 80 | 3 | nPen | ESI+ : 296 | 91 | 3 | 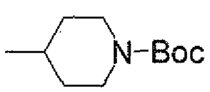 | FAB+ : 408 |
| 81 | 3 | $-(CH_2)_2OMe$ | ESI+ : 284 | 92 | 3 | 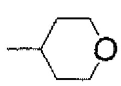 | EI+ : 309 |
| 82 | 3 | $-(CH_2)_2OBn$ | ESI+ : 360 | 93 | 3 | $-CH_2$-cPr | ESI+ : 280 |
| 83 | 3 | $-(CH_2)_2NMe_2$ | ESI+ : 297 | 94 | 3 | 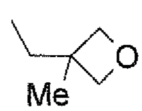 | APCI+ : 310 |

(continued)

| REx | RSyn | $R^4$ | Dat | REx | RSyn | $R^4$ | Dat |
|---|---|---|---|---|---|---|---|
| 84 | 3 | $-(CH_2)_3OMe$ | FAB+ : 297 | 95 | 3 | 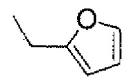 | ESI+ : 306 |
| 85 | 3 | $-(CH_2)_2Ph$ | FAB+ : 330 | 96 | 3 | 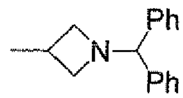 | ESI+ : 447 |
| 86 | 3 | Bn | ESI+ : 316 | 97 | 3 | 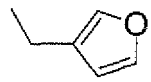 | ESI+ : 306 |
| 87 | 3 | cBu | MSI+ : 280 | 98 | 3 | 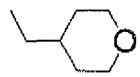 | FAB+ : 323 |
| 88 | 3 | cPen | ESI+ : 294 | 99 | 10 | 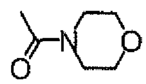 | FAB+ : 339 |

[Table 4]

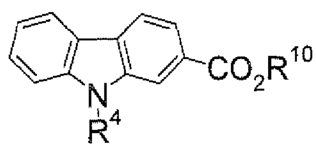

| REx | RSyn | $R^4$ | $R^{10}$ | Dat | REx | RSyn | $R^4$ | $R^{10}$ | Dat |
|---|---|---|---|---|---|---|---|---|---|
| 100 | 3 | Et | Et | ESI+ : 268 | 103 | 10 | $-S(O)_2-Me$ | Bn | EI+ : 379 |
| 101 | 3 | 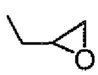 | Bn | EI+ : 357 | 104 | 10 | $-S(O)_2-iPr$ | Bn | ESI+ : 408 |
| 102 | 10 | Ac | Bn | ESI+ : 344 | 105 | 10 | $-C(O)NMe_2$ | Bn | FAB+ : 373 |

[Table 5]

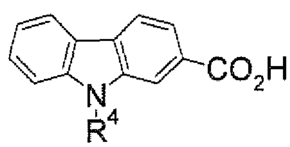

| REx | RSyn | $R^4$ | Dat | REx | RSyn | $R^4$ | Dat |
|---|---|---|---|---|---|---|---|
| 106 | 4 | nPr | ESI- : 252 | 118 | 4 | cPen | ESI- : 278 |
| 107 | 4 | nBu | ESI- : 266 | 119 | 4 | cHex | ESI- : 292 |
| 108 | 4 | nPen | ESI- : 280 | 120 | 4 | $-CH(C_2H_5)_2$ | ESI- : 280 |
| 109 | 4 | $-(CH_2)_2OBn$ | ESI- : 344 | 121 | 4 | $-CH_2-cPr$ | ESI- : 264 |
| 110 | 4 | $-(CH_2)_3OMe$ | ESI- : 282 | 122 | 11 | Ac | ESI+ : 254 |

(continued)

| REx | RSyn | $R^4$ | Dat | REx | RSyn | $R^4$ | Dat |
|---|---|---|---|---|---|---|---|
| 111 | 4 | Bn | ESI- : 300 | 123 | 11 | $-S(O)_2-Me$ | FAB+ : 289 |
| 112 | 4 | $-(CH_2)_2Ph$ | ESI- : 314 | 124 | 11 | $-S(O)_2-iPr$ | ESI- : 316 |
| 113 | 4 | cBu | FAB+ 266 | 125 | 11 | $-C(O)NMe_2$ | FAB+ : 283 |
| 114 | 4 | 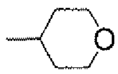 | ESI- : 294 | 126 | 4 | 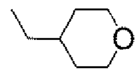 | ESI- : 308 |
| 115 | 4 | 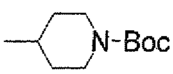 | ESI- : 393 | 127 | 4 | 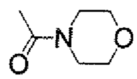 | FAB+ : 325 |
| 116 | 4 | 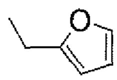 | ESI- : 290 | 128 | 11 | 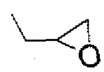 | ESI- : 266 |
| 117 | 4 | 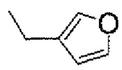 | ESI- : 290 | | | | |

[Table 6]

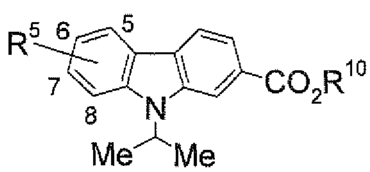

| REx | RSyn | $R^5$ | $R^{10}$ | Dat | REx | RSyn | $R^5$ | $R^{10}$ | Dat |
|---|---|---|---|---|---|---|---|---|---|
| 129 | 3 | 5-F | Me | FAB+ : 286 | 142 | 3 | 5-Cl | Me | ESI+ : 302 |
| 130 | 3 | 6-F | Me | FAB+ : 286 | 143 | 3 | 7-Cl | Me | ESI+ : 302 |
| 131 | 3 | 7-F | Me | FAB+ : 286 | 144 | 3 | 8-Cl | Me | FAB+ : 302 |
| 132 | 3 | 8-F | Me | FAB+ : 286 | 145 | 3 | 5-CN | Me | FAB+ : 293 |
| 133 | 3 | 5-Me | Me | FAB+ : 282 | 146 | 3 | 6-CN | Me | FAB+ : 293 |
| 134 | 3 | 6-Me | Me | FAB+ : 282 | 147 | 3 | 7-CN | Me | FAB+ : 293 |
| 135 | 3 | 7-Me | Me | FAB+ : 282 | 148 | 3 | 8-CN | Me | FAB+ : 293 |
| 136 | 3 | 8-Me | Me | FAB+ : 282 | 149 | 4 | 5-CN | H | FAB+ : 279 |
| 137 | 3 | 5-OMe | Me | FAB+ : 298 | 150 | 4 | 6-CN | H | FAB- : 277 |
| 138 | 3 | 6-OMe | Me | FAB+ : 297 | 151 | 4 | 7-CN | H | FAB+ : 279 |
| 139 | 3 | 7-OMe | Me | FAB+ : 298 | 152 | 4 | 8-CN | H | FAB+ : 279 |
| 140 | 3 | 8-OMe | Me | FAB+ : 298 | 153 | 4 | $6-NO_2$ | H | FAB- : 297 |
| 141 | 3 | 6-Cl | Me | FAB+ : 302 | 154 | 4 | 5-C(O)H | H | FAB+ : 282 |

[Table 7]

| REx | Str | REx | Str |
|---|---|---|---|
| 21 | 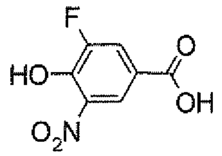 | 29 | 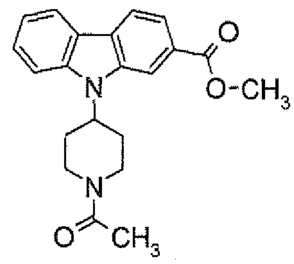 |
| 22 | 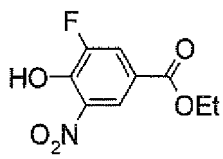 | | |
| 23 | 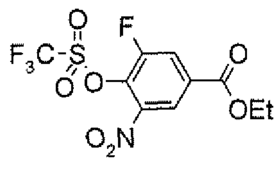 | 30 | 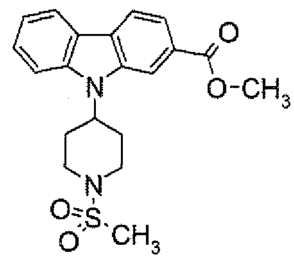 |
| 24 | 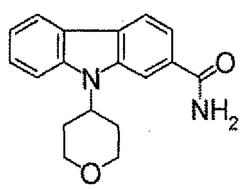 | 31 | 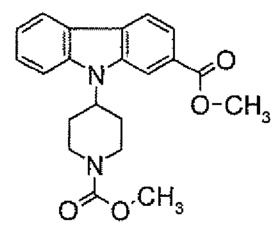 |
| 25 | 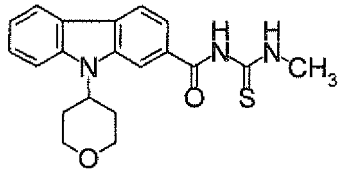 | 32 | 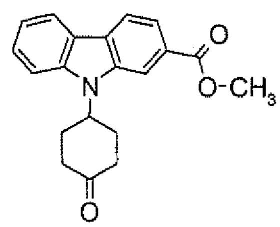 |
| 26 | 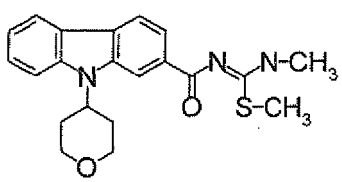 | 33 | 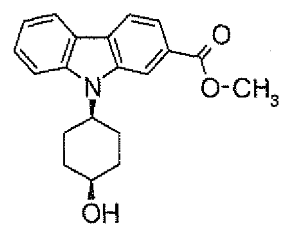 |
| 27 | 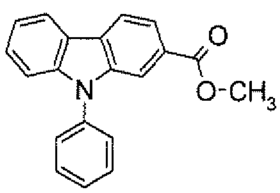 | 34 | 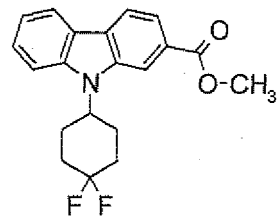 |

(continued)

| REx | Str | | REx | Str |
|---|---|---|---|---|
| 28 | 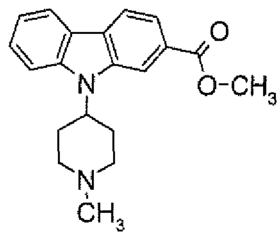 | | 35 | 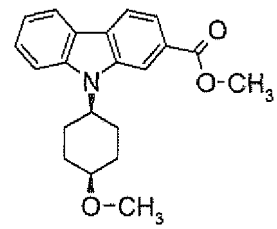 |

[Table 8]

| REx | Str | | REx | Str |
|---|---|---|---|---|
| 36 | 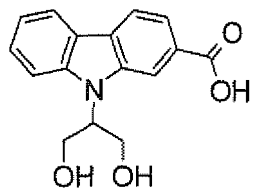 | | 156 | 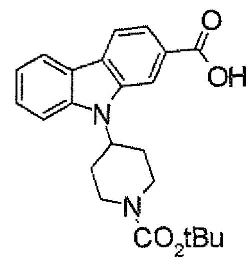 |
| 37 | 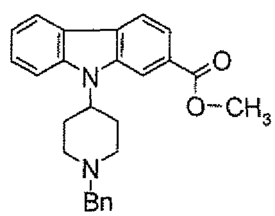 | | 157 | 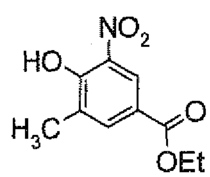 |
| 38 | 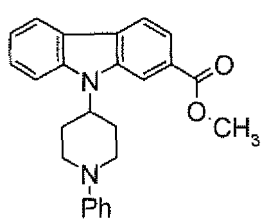 | | 158 | 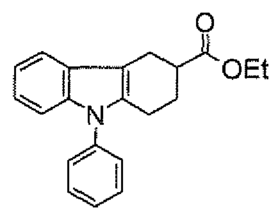 |
| 39 | 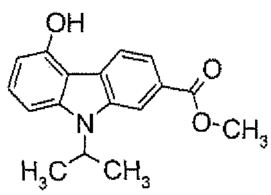 | | 159 | 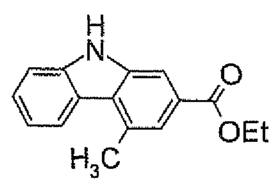 |
| 40 | 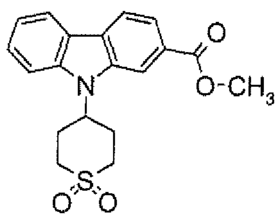 | | 160 | 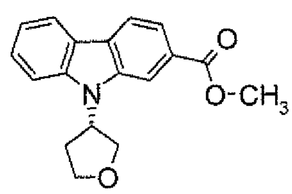 |

(continued)

| REx | Str | | REx | Str |
|---|---|---|---|---|
| 41 | 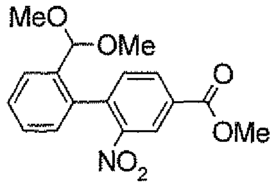 | | 161 | 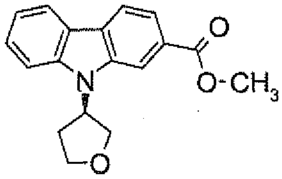 |
| 42 | 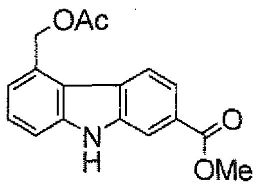 | | 162 | 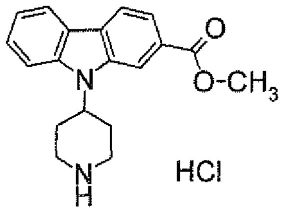 |
| 155 | 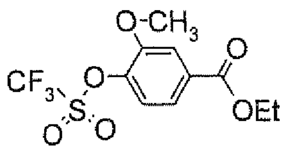 | | 163 | 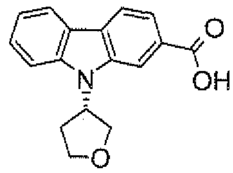 |

[Table 9]

| REx | Str | | REx | Str |
|---|---|---|---|---|
| 164 | 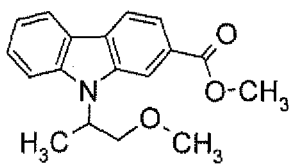 | | 172 | 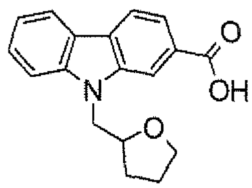 |
| 165 | 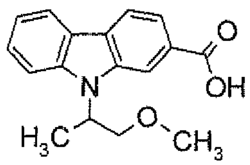 | | 173 | 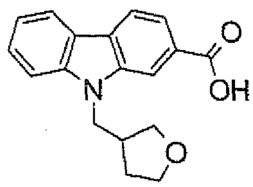 |
| 166 | 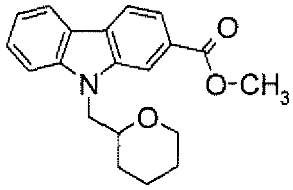 | | 174 | 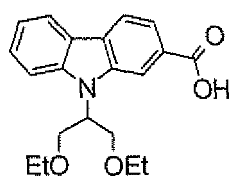 |
| 167 | 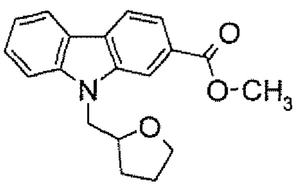 | | 175 | 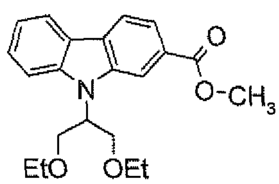 |

(continued)

| REx | Str | | REx | Str |
|---|---|---|---|---|
| 168 | 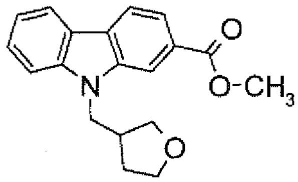 | | 176 | 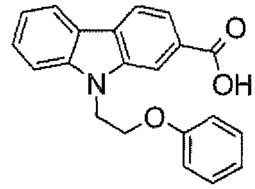 |
| 169 | 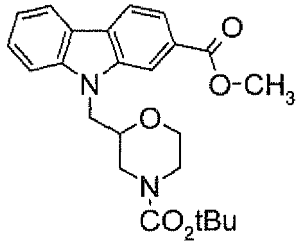 | | 177 | 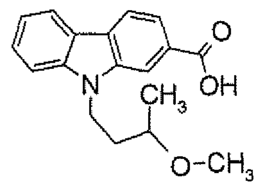 |
| 170 | 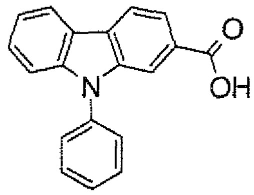 | | 178 | 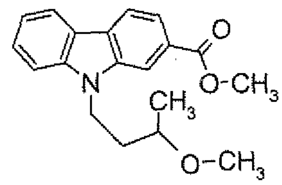 |
| 171 | 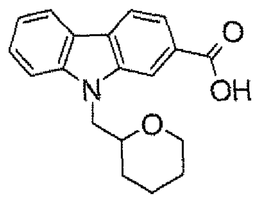 | | 179 | 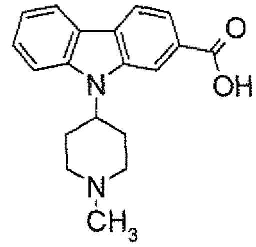 |

[Table 10]

| REx | Str | | REx | Str |
|---|---|---|---|---|
| 180 | 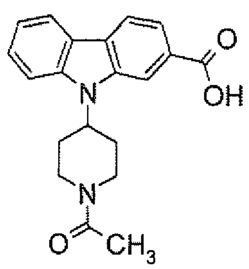 | | 188 | 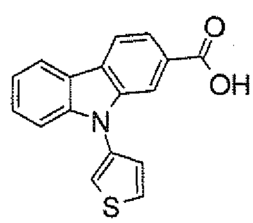 |
| 181 | 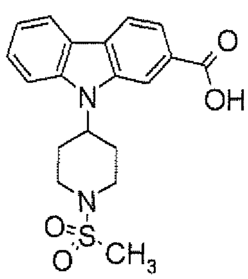 | | 189 | 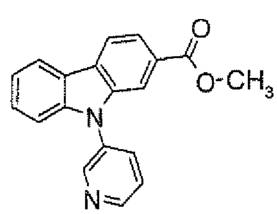 |

(continued)

| REx | Str | | REx | Str |
|---|---|---|---|---|
| 182 | 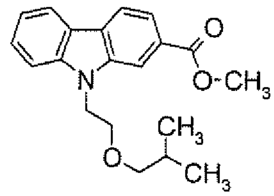 | | 190 | 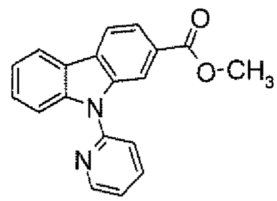 |
| 183 | 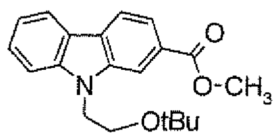 | | 191 | 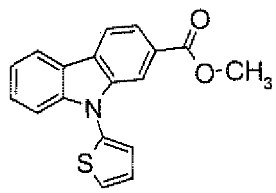 |
| 184 | 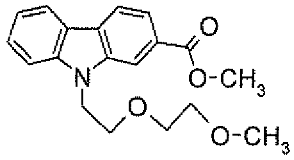 | | 192 | 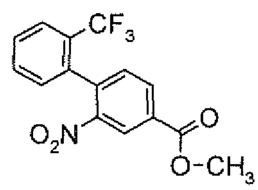 |
| 185 | 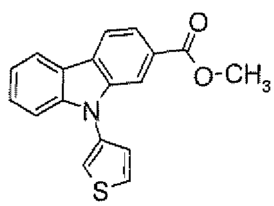 | | 193 | 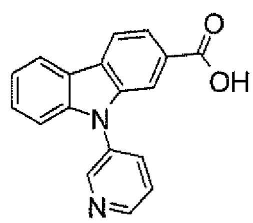 |
| 186 | 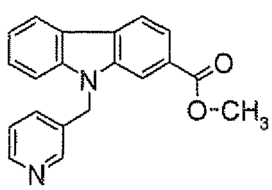 | | 194 | 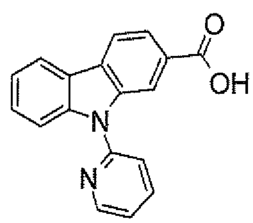 |
| 187 | 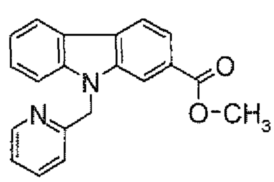 | | 195 | 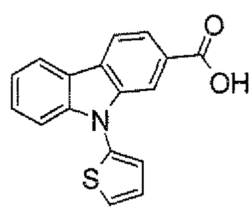 |

[Table 11]

| REx | Str | | REx | Str |
|---|---|---|---|---|
| 196 | 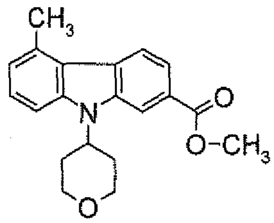 | | 204 | 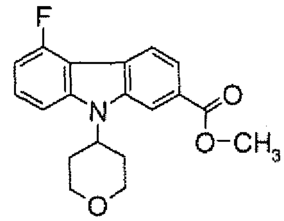 |
| 197 | 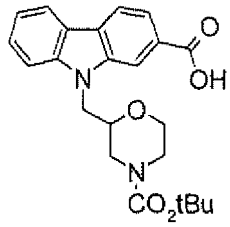 | | 205 | 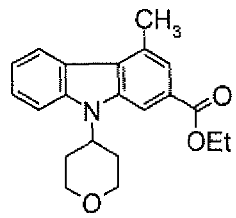 |
| 198 | 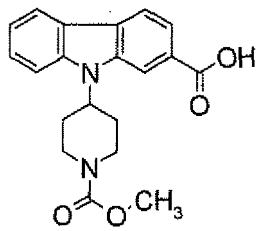 | | 206 | 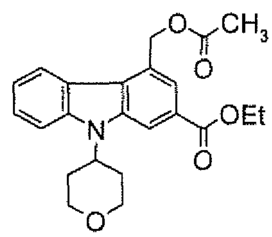 |
| 199 | 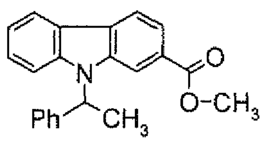 | | 207 | 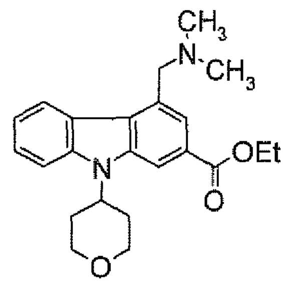 |
| 200 | 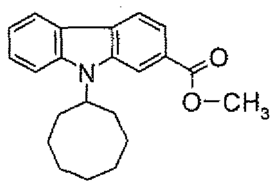 | | 208 | 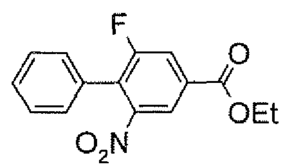 |
| 201 | 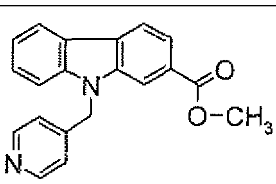 | | 209 | 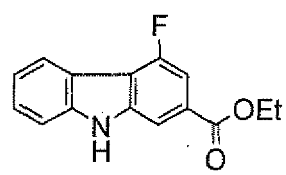 |
| 202 | 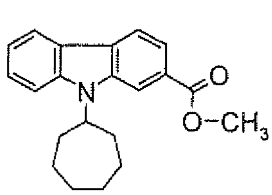 | | 210 | 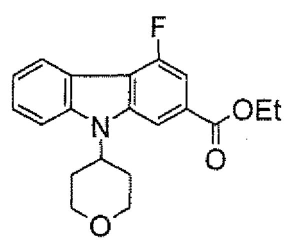 |

(continued)

| REx | Str | | REx | Str |
|---|---|---|---|---|
| 203 | 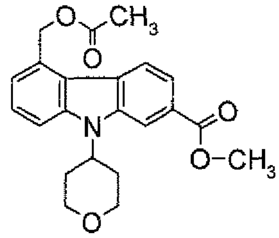 | | | |

[Table 12]

| REx | Str | | REx | Str |
|---|---|---|---|---|
| 211 | 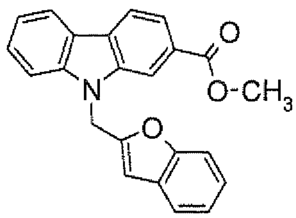 | | 218 | 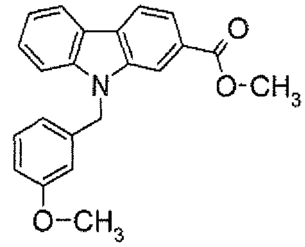 |
| 212 | 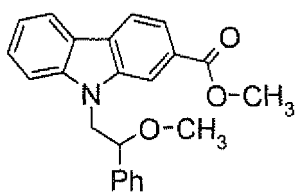 | | 219 | 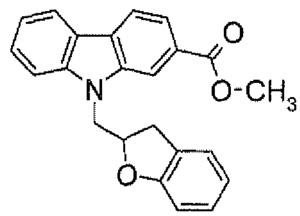 |
| 213 | 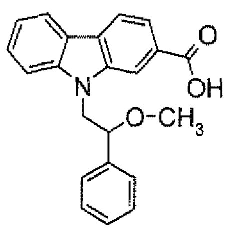 | | 220 | 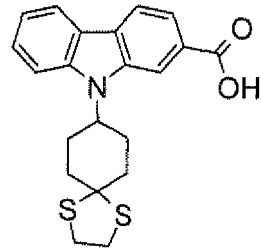 |
| 214 | 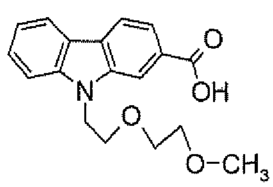 | | 221 | 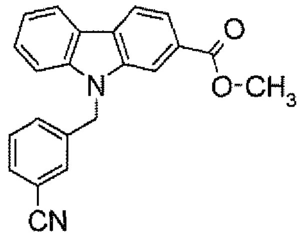 |

(continued)

| REx | Str | | REx | Str |
|---|---|---|---|---|
| 215 | 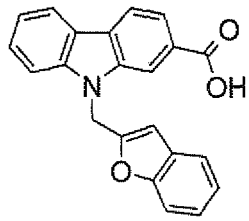 | | 222 | 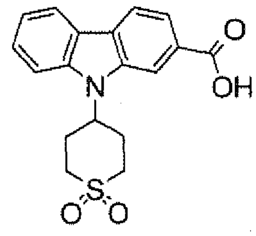 |
| 216 | 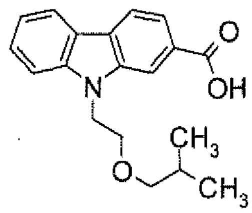 | | 223 | 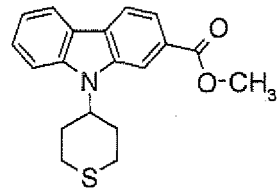 |
| 217 | 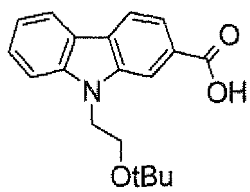 | | 224 | 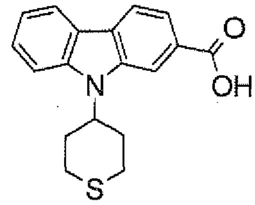 |

[Table 13]

| REx | Str | | REx | Str |
|---|---|---|---|---|
| 225 | 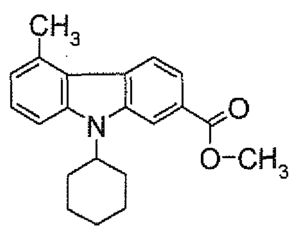 | | 232 | 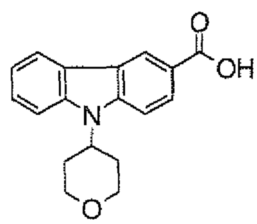 |
| 226 | 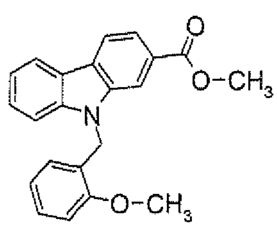 | | 233 | 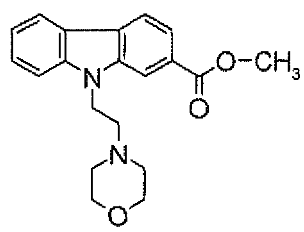 |
| 227 | 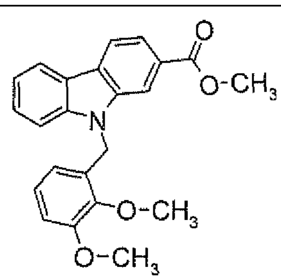 | | 234 | 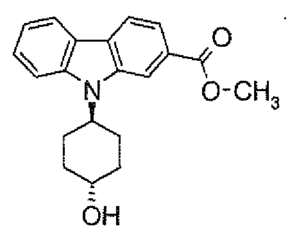 |

(continued)

| REx | Str | | REx | Str |
|---|---|---|---|---|
| 228 | 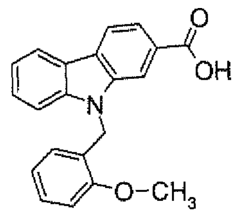 | | 235 | 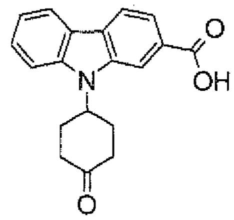 |
| 229 | 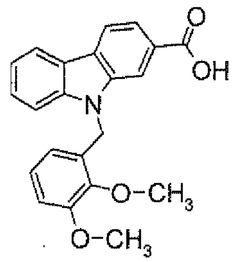 | | 236 | 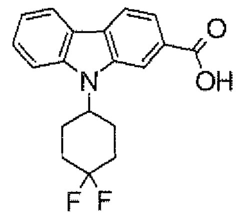 |
| | | | 237 | 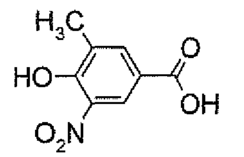 |
| 230 | 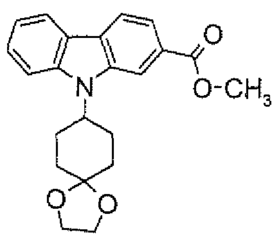 | | 238 | 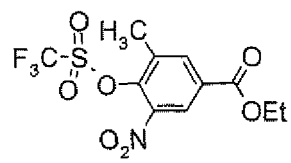 |
| 231 | 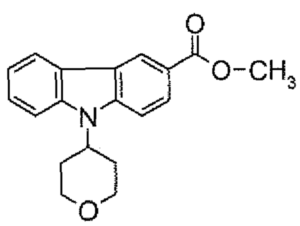 | | 239 | 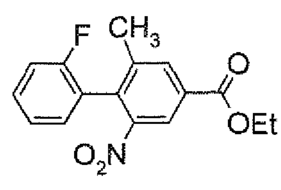 |

[Table 14]

| REx | Str | | REx | Str |
|---|---|---|---|---|
| 240 | 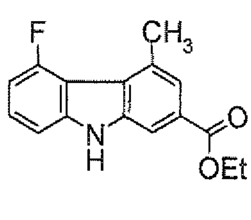 | | 249 | 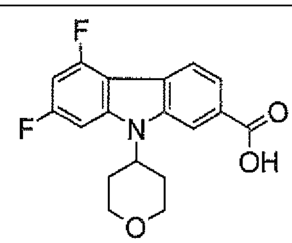 |

(continued)

| REx | Str | | REx | Str |
|---|---|---|---|---|
| 241 | 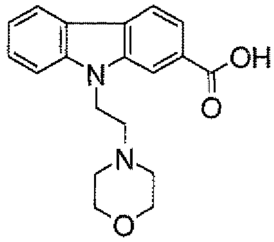 | | 250 | 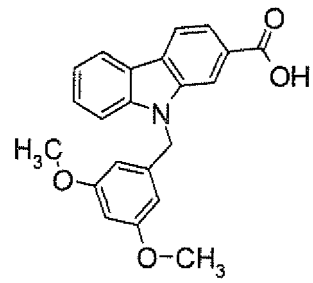 |
| 242 | 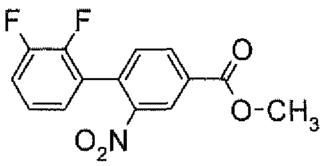 | | | |
| 243 | 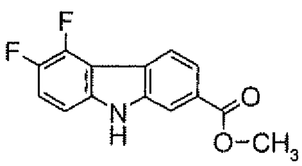 | | 251 | 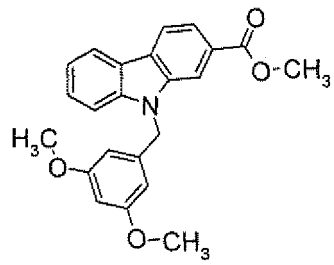 |
| 244 | 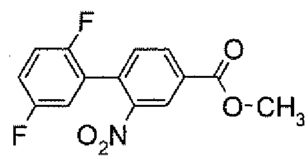 | | 252 | 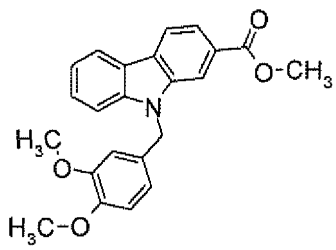 |
| 245 | 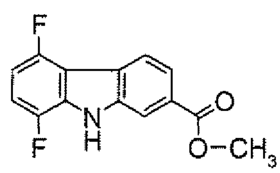 | | | |
| 246 | 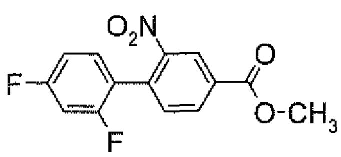 | | 253 | 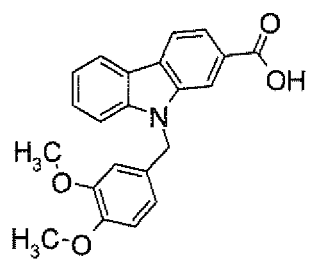 |

(continued)

| REx | Str | | REx | Str |
|---|---|---|---|---|
| 247 | 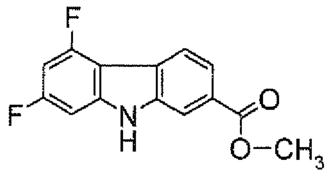 | | 254 | 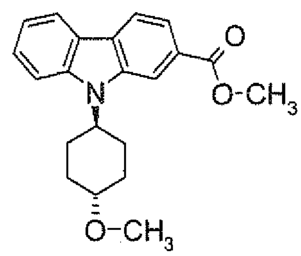 |
| 248 | 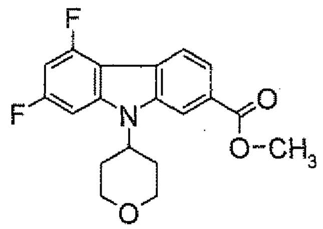 | | | |

[Table 15]

| REx | Str | | REx | Str |
|---|---|---|---|---|
| 255 | 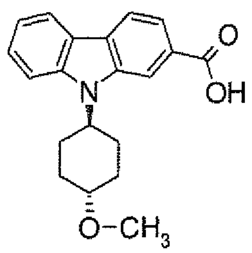 | | 264 | 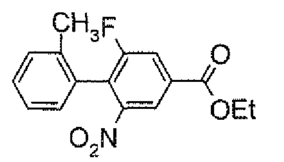 |
| 256 | 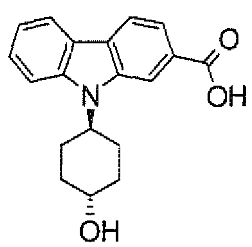 | | 265 | 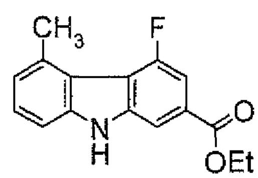 |
| 257 | 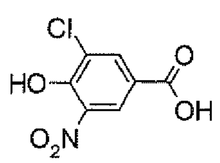 | | 266 | 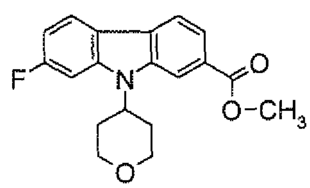 |
| 258 | 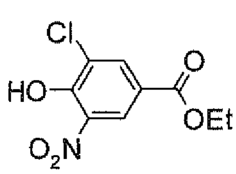 | | 267 | 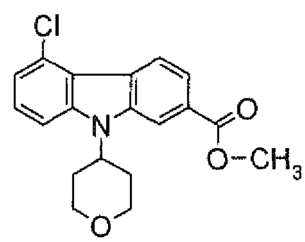 |

(continued)

| REx | Str | | REx | Str |
|---|---|---|---|---|
| 259 | 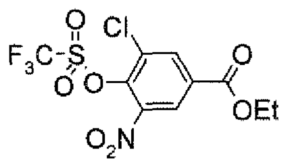 | | 268 | 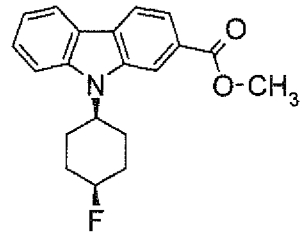 |
| 260 | 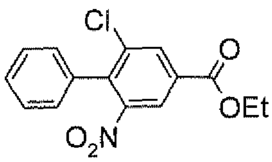 | | 269 | 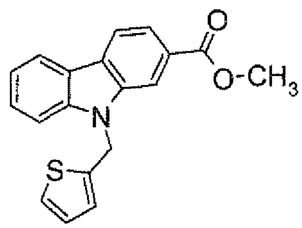 |
| 261 | 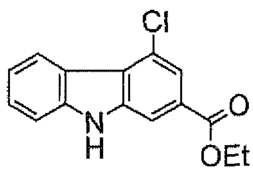 | | 270 | 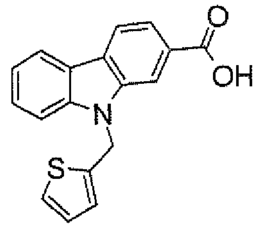 |
| 262 | 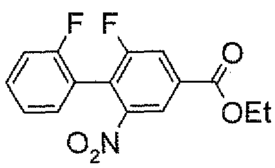 | | | |
| 263 | 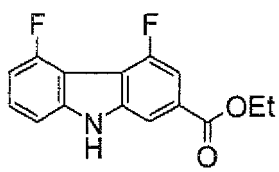 | | | |

[Table 16]

| REx | Str | | REx | Str |
|---|---|---|---|---|
| 271 | 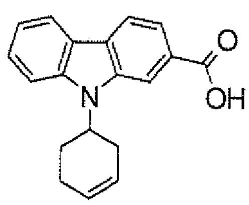 | | 278 | 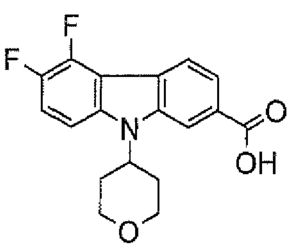 |

(continued)

| REx | Str | | REx | Str |
|---|---|---|---|---|
| 272 | 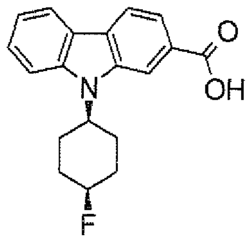 | | 279 | 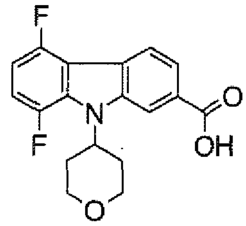 |
| 273 | 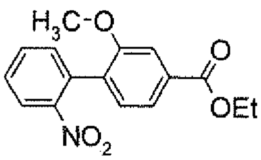 | | 280 | 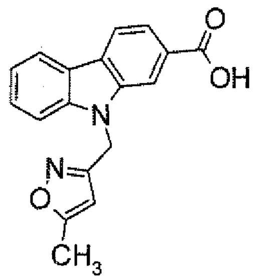 |
| 274 | 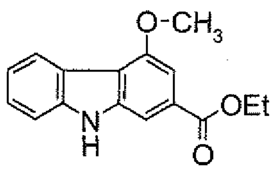 | | 281 | 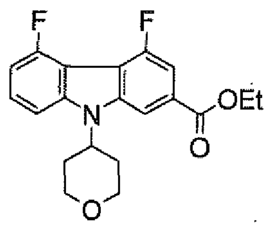 |
| 275 | 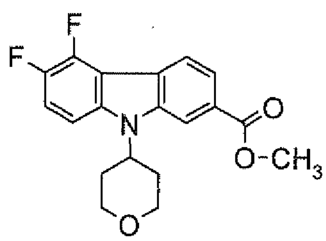 | | 282 | 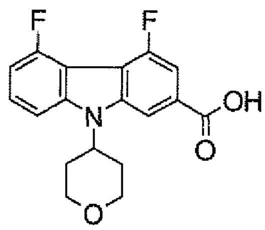 |
| 276 | 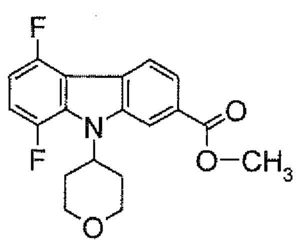 | | 283 | 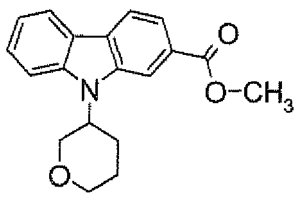 |
| 277 | 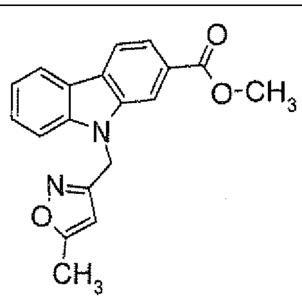 | | 284 | 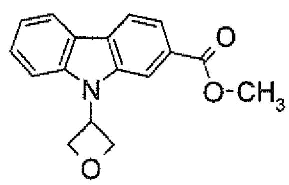 |

[Table 17]

| REx | Str | | REx | Str |
|---|---|---|---|---|
| 285 | 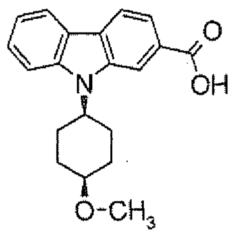 | | 292 | 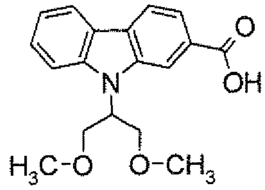 |
| 286 | 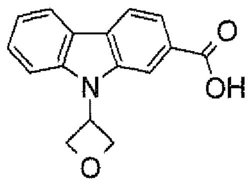 | | 293 | 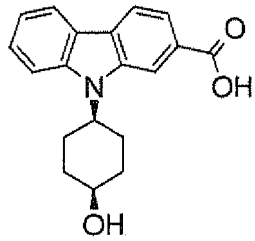 |
| 287 | 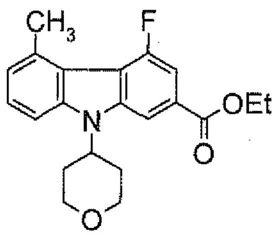 | | 294 | 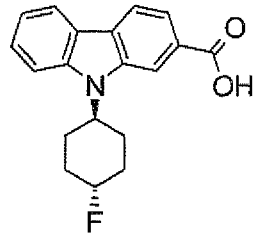 |
| 288 | 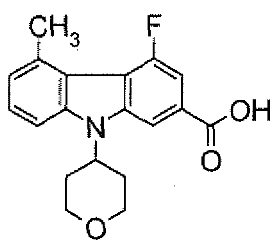 | | 295 | 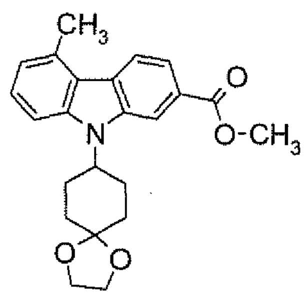 |
| 289 | 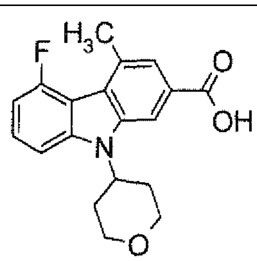 | | 296 | 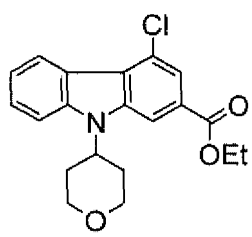 |
| 290 | 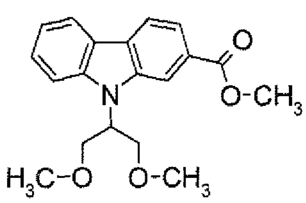 | | 297 | 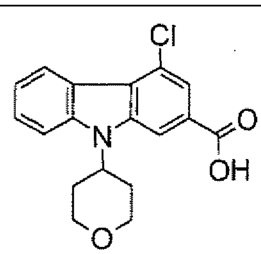 |

(continued)

| REx | Str | | REx | Str |
|---|---|---|---|---|
| 291 | 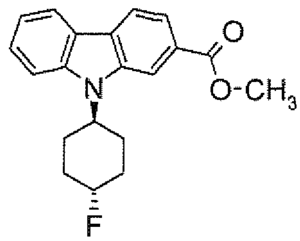 | | | |

[Table 18]

| REx | Str | | REx | Str |
|---|---|---|---|---|
| 298 | 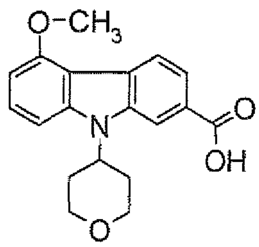 | | 306 | 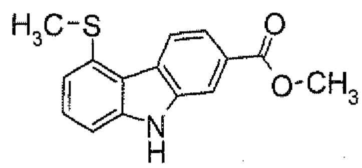 |
| 299 | 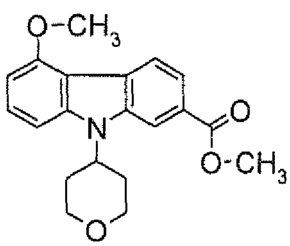 | | 307 | 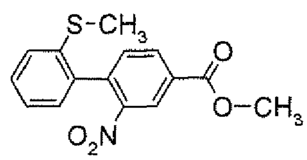 |
| 300 | 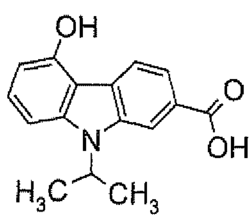 | | 308 | 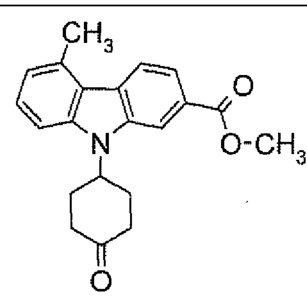 |
| 301 | 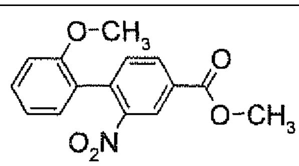 | | 309 | 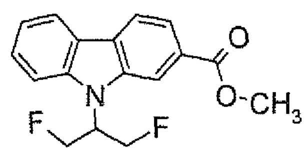 |
| 302 | 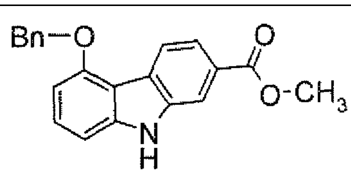 | | 310 | 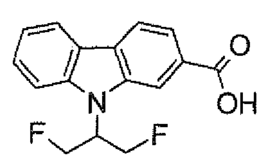 |

(continued)

| REx | Str | | REx | Str |
|---|---|---|---|---|
| 303 | 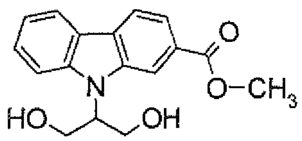 | | 311 | 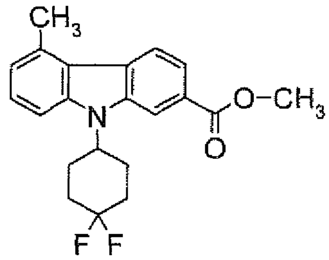 |
| 304 | 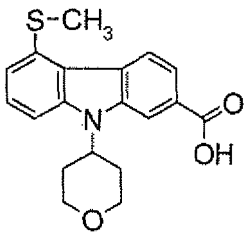 | | 312 | 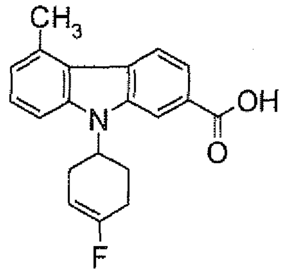 |
| 305 | 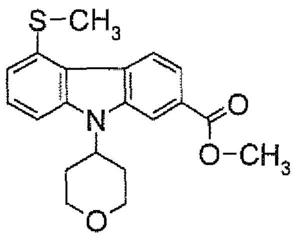 | | 313 | 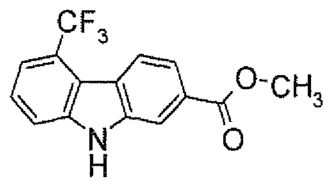 |

[Table 19]

| REx | Str | | REx | Str |
|---|---|---|---|---|
| 314 | 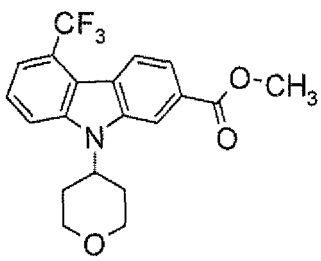 | | 321 | 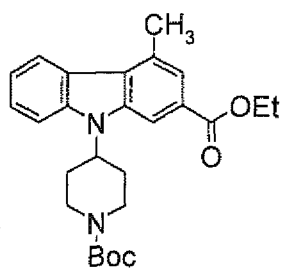 |
| 315 | 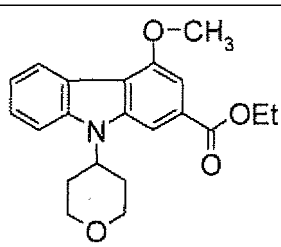 | | 322 | 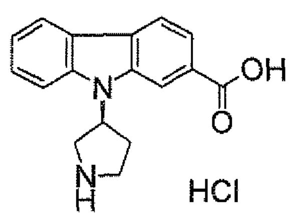 |

(continued)

| REx | Str | | REx | Str |
|---|---|---|---|---|
| 316 | 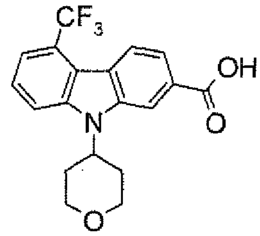 | | 323 | 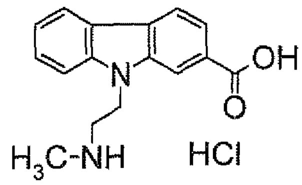 |
| 317 | 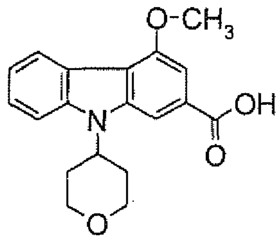 | | 324 | 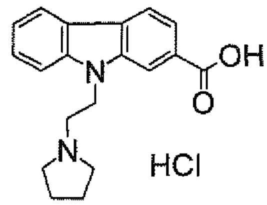 |
| 318 | 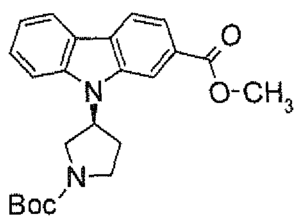 | | 325 | 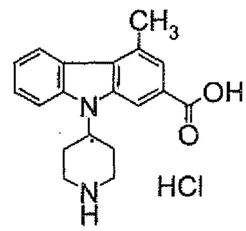 |
| 319 | 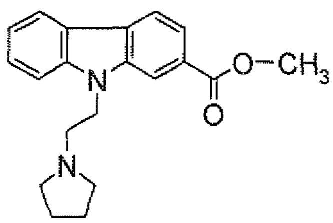 | | 326 | 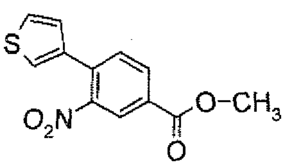 |
| 320 | 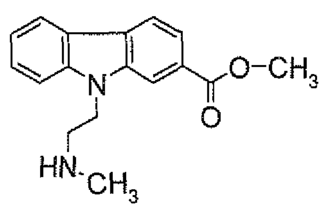 | | 327 | 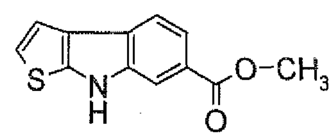 |
| | | | 328 | 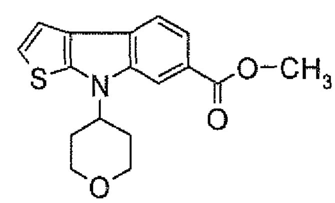 |

[Table 20]

| REx | Str | | REx | Str |
|---|---|---|---|---|
| 329 | 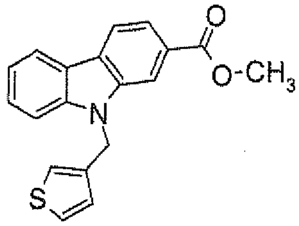 | | 337 | 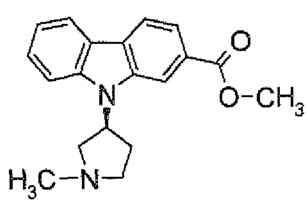 |
| 330 | 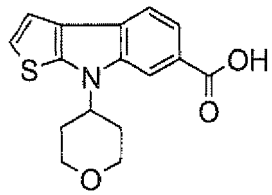 | | 338 | 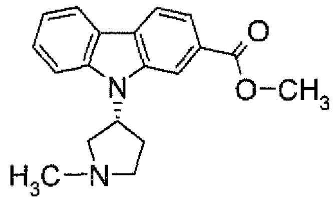 |
| 331 | 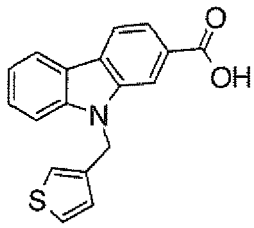 | | 339 | 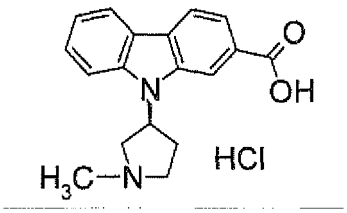 HCl |
| 332 | 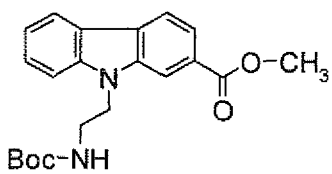 | | 340 | 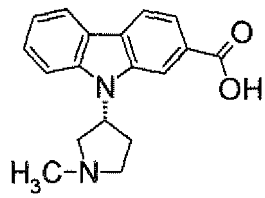 |
| 333 | 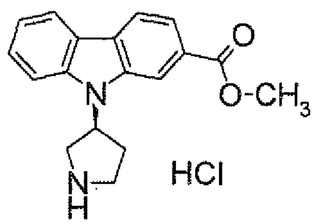 HCl | | 341 | 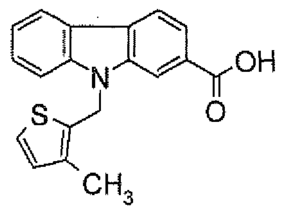 |
| 334 | 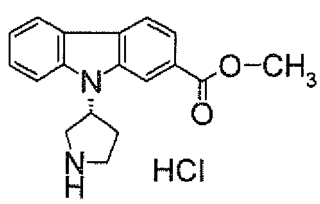 HCl | | 342 | 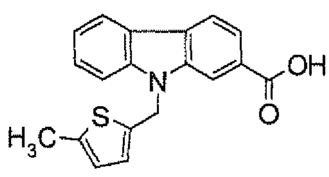 |
| 335 | 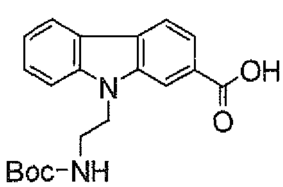 | | 343 | 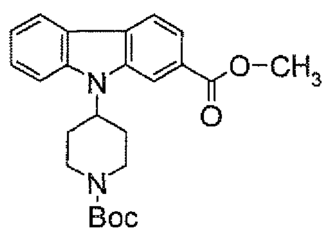 |

(continued)

| REx | Str | | REx | Str |
|---|---|---|---|---|
| 336 | 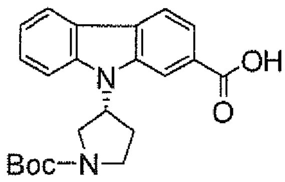 | | 344 | 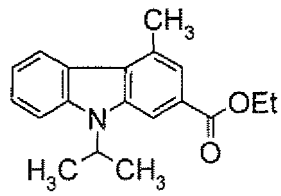 |

[Table 21]

| REx | Str | | REx | Str |
|---|---|---|---|---|
| 345 | 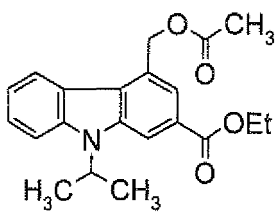 | | 352 | 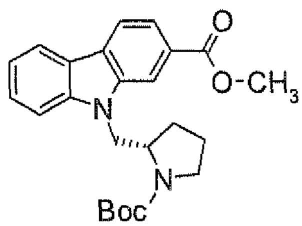 |
| 346 | 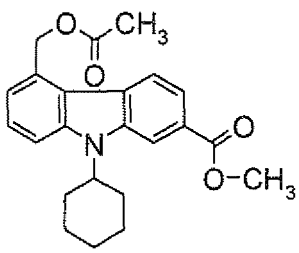 | | 353 | 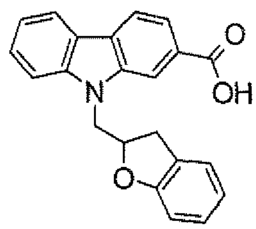 |
| 347 | 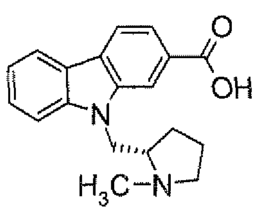 | | 354 | 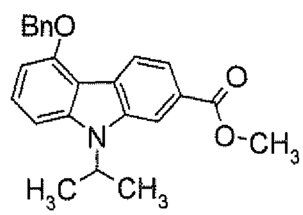 |
| 348 | 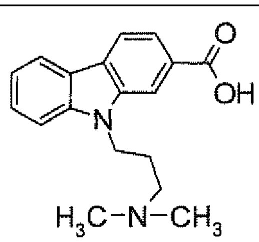 | | 355 | 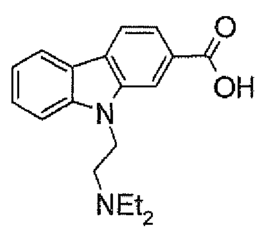 |
| 349 | 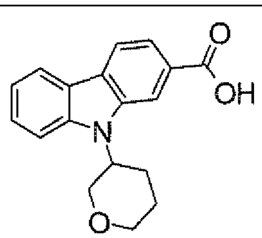 | | 356 | 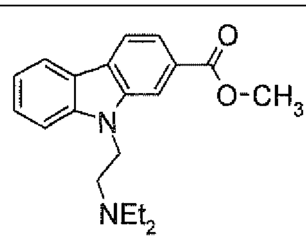 |

(continued)

| REx | Str | | REx | Str |
|---|---|---|---|---|
| 350 | 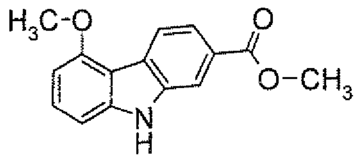 | | 357 | 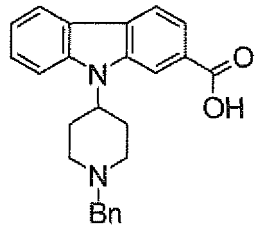 |
| 351 | 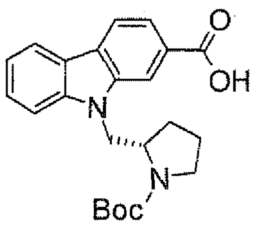 | | 358 | 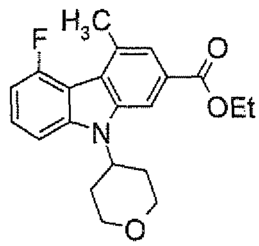 |

[Table 22]

| REx | Str | | REx | Str |
|---|---|---|---|---|
| 359 | 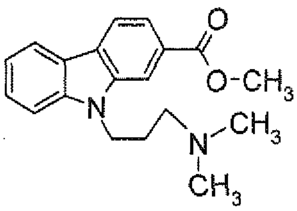 | | 366 | 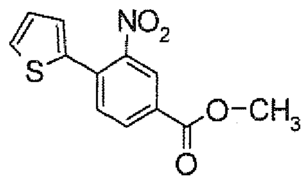 |
| 360 | 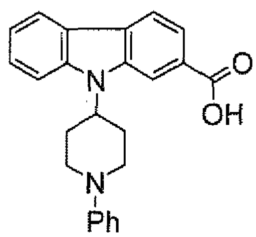 | | 367 | 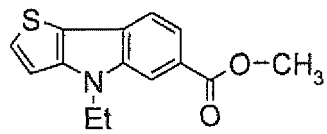 |
| 361 | 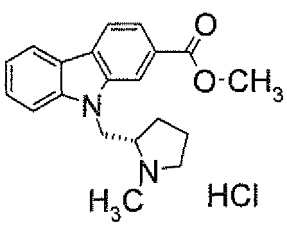 | | 368 | 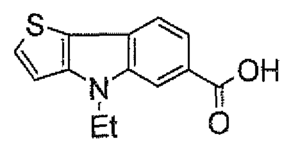 |
| 362 | 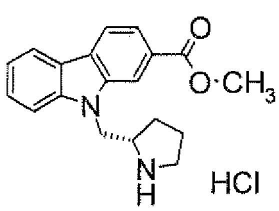 | | 369 | 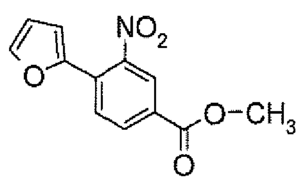 |

(continued)

| REx | Str | | REx | Str |
|---|---|---|---|---|
| 363 | 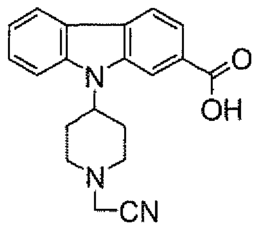 | | 370 | 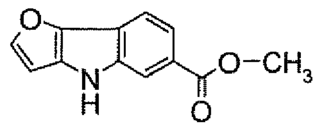 |
| 364 | 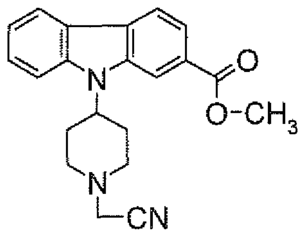 | | 371 | 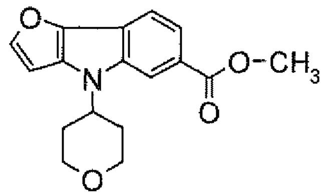 |
| 365 | 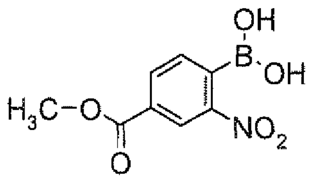 | | 372 | 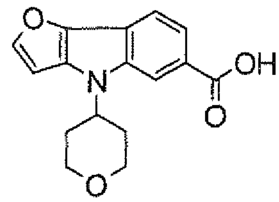 |
| | | | 373 | 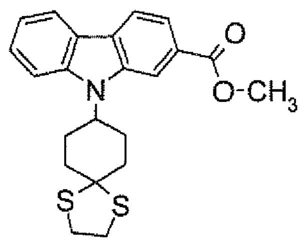 |
| | | | 374 | 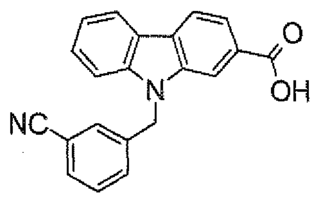 |

[Table 23]

| REx | Str | | REx | Str |
|---|---|---|---|---|
| 375 | 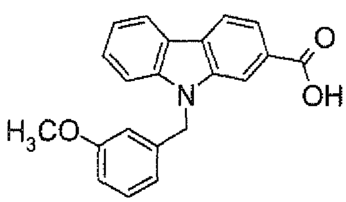 | | 382 | 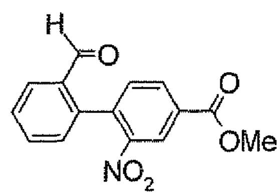 |

(continued)

| REx | Str | | REx | Str |
|---|---|---|---|---|
| 376 | 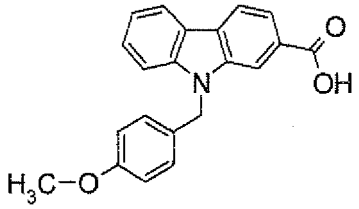 | | 383 | 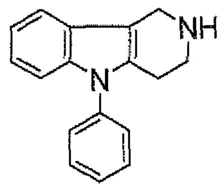 |
| 377 | 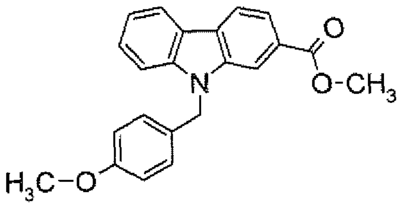 | | 384 | 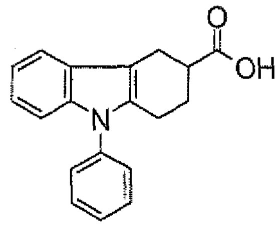 |
| 378 | 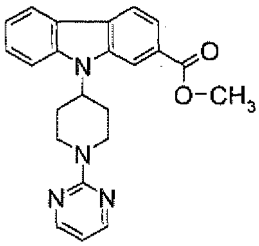 | | 385 | 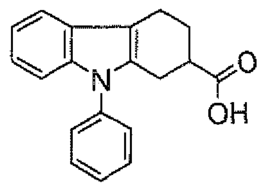 |
| 379 | 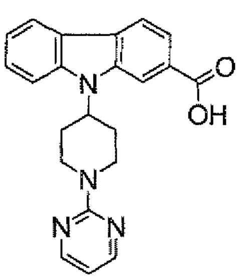 | | 386 | 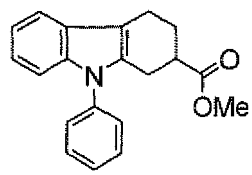 |
| 380 | 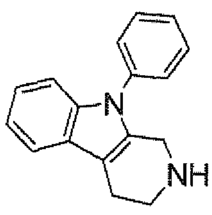 | | 387 | 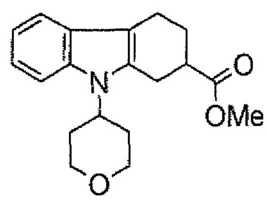 |
| 381 | 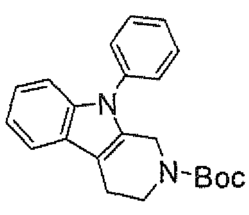 | | 388 | 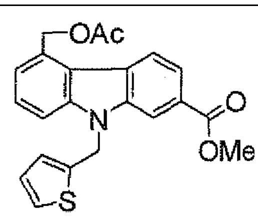 |
| | | | 389 | 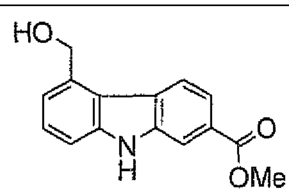 |

[Table 24]

| REx | Str | | REx | Str |
|---|---|---|---|---|
| 390 | 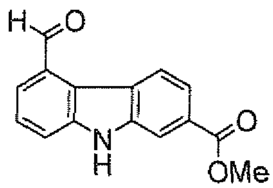 | | 398 | 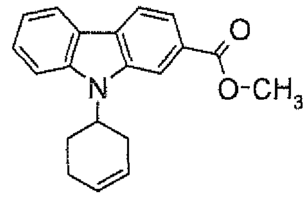 |
| 391 | 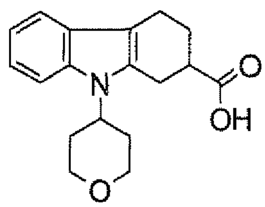 | | 399 | 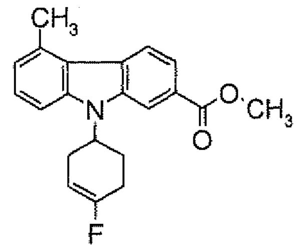 |
| 392 | 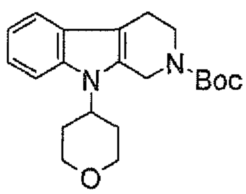 | | 400 | 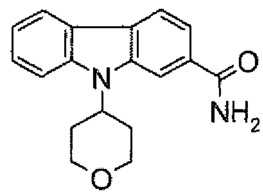 |
| 393 | 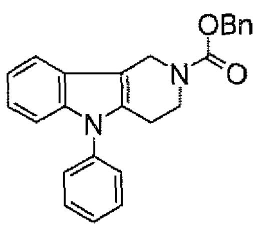 | | 401 | 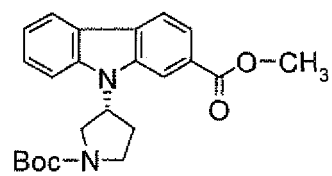 |
| 394 | 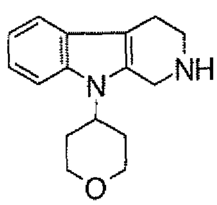 | | 402 | 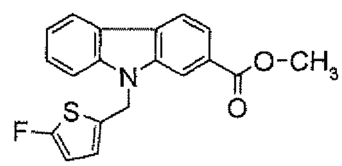 |
| 395 | 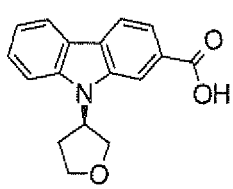 | | 403 | 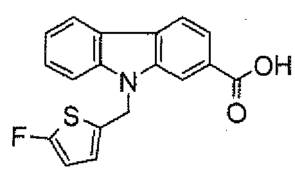 |
| 396 | 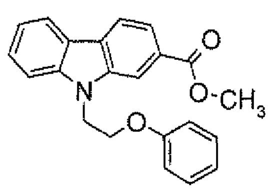 | | 404 | 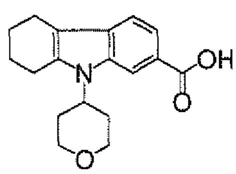 |

(continued)

| REx | Str | | REx | Str |
|---|---|---|---|---|
| 397 | 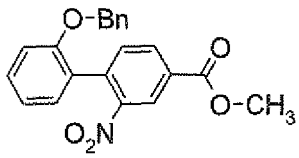 | | 405 | 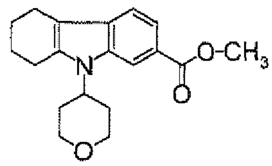 |

[Table 25]

| REx | RSyn | Dat | | REx | RSyn | Dat | | REx | RSyn | Dat |
|---|---|---|---|---|---|---|---|---|---|---|
| 21 | 21 | ESI-: 200 | | 169 | 3 | FAB+: 424 | | 205 | 3 | ESI+: 360 |
| 22 | 21,22 | ESI-: 228 | | 170 | 4 | FAB-: 286 | | 206 | 5, 7 | ESI+: 418 |
| 22 | 22 | ESI-: 228 | | 171 | 4 | FAB-: 308 | | 207 | 5, 6 | ESI+: 381 |
| 23 | 23 | ND | | 172 | 4 | FAB+: 296 | | 208 | 23, 1 | FAB-: 289 |
| 25 | 25 | ESI+: 368 | | 173 | 4 | FAB-: 294 | | 209 | 2 | ESI-: 256 |
| 26 | 26 | ESI+: 382 | | 174 | 4 | FAB+:342 | | 210 | 3 | ESI+: 342 |
| 27 | 27 | FAB+: 301 | | 175 | 3 | FAB+:356 | | 211 | 3 | Fay-: 355 |
| 28 | 28 | FAB+: 323 | | 176 | 4 | FAB-:332 | | 212 | 3 | EI+:359 |
| 29 | 29 | FAB+: 351 | | 177 | 4 | FAB+:298 | | 213 | 4 | FAB-:344 |
| 30 | 30 | FAB+: 386 | | 178 | 3 | FAB+:312 | | 214 | 4 | FAB-:312 |
| 31 | 31 | ESI+: 389 | | 179 | 4 | FAB+: 309 | | 215 | 4 | FAB-:340 |
| 32 | 32 | EI+: 321 | | 180 | 4 | ESI+:337 | | 216 | 4 | FAB+:312 |
| 33 | 33 | ESI+: 324 | | 181 | 4 | FAB-: 371 | | 217 | 4 | FAB+:311 |
| 34 | 34 | EI+: 343 | | 182 | 3 | FAB+:326 | | 218 | 3 | EI+:345 |
| 35 | 35 | EI+: 337 | | 183 | 3 | ND | | 219 | 3 | EI+:357 |
| 36 | 36 | ESI-: 284 | | 184 | 3 | FAB+:328 | | 220 | 4 | FAB+:383 |
| 37 | 37 | FAB+:399 | | 185 | 27 | FAB+: 308 | | 221 | 3 | EI+:340 |
| 38 | 38 | FAB+:385 | | 186 | 3 | ESI+: 317 | | 222 | 4 | ESI-:342 |
| 39 | 21 | FAB+:284 | | 187 | 3 | FAB+: 317 | | 223 | 3 | EI+:325 |
| 40 | 40 | FAB+:357 | | 188 | 4 | FAB-: 292 | | 224 | 4 | FAB-:310 |
| 41 | 41 | FAB-:331 | | 189 | 27 | FAB+: 303 | | 225 | 3 | ESI+: 322 |
| 42 | 42 | EI+:297 | | 190 | 27 | FAB+: 303 | | 226 | 3 | FAB+: 345 |
| 155 | 23 | ND | | 191 | 27 | FAB+: 308 | | 227 | 3 | EI+: 375 |
| 156 | 4 | FAB+: 394 | | 192 | 1 | FAB+: 326 | | 228 | 4 | FAB-: 330 |
| 157 | 22 | ND | | 193 | 4 | ESI+: 289 | | 229 | 4 | FAB-: 360 |
| 158 | 27 | FAB+:320 | | 194 | 4 | ESI+: 289 | | 230 | 3 | FAB+: 365 |
| 159 | 2 | ESI: 252 | | 195 | 4 | FAB+: 293 | | 231 | 3 | ESI+: 310 |
| 160 | 3 | FAB+: 250 | | 196 | 3 | ESI+: 324 | | 232 | 4 | ESI+: 296 |
| 161 | 3 | FAB+: 295 | | 197 | 4 | ESI-: 409 | | 233 | 3 | FAB+:339 |
| 162 | 3, 32 | ESI+: 309 | | 198 | 4 | ESI+: 375 | | 234 | 33 | ESI+: 324 |

(continued)

| REx | RSyn | Dat | | REx | RSyn | Dat | | REx | RSyn | Dat |
|---|---|---|---|---|---|---|---|---|---|---|
| 163 | 4 | FAB-: 280 | | 199 | 3 | ESI+: 330 | | 235 | 4 | ESI-: 306 |
| 164 | 3 | FAB+: 298 | | 200 | 3 | FAB+: 335 | | 236 | 4 | ESI-: 328 |
| 165 | 4 | FAB+: 284 | | 201 | 3 | ESI+: 317 | | 237 | 21 | ND |
| 166 | 3 | FAB+: 324 | | 202 | 3 | ESI+: 322 | | 238 | 23 | ND |
| 167 | 3 | FAB+: 310 | | 203 | 5,7 | FAB+: 381 | | 239 | 1 | ESI+: 304 |
| 168 | 3 | FAB+: 310 | | 204 | 3 | ESI+: 328 | | 240 | 2 | ESI-: 270 |

[Table 26]

| REx | RSyn | Dat | | REx | RSyn | Dat | | REx | RSyn | Dat |
|---|---|---|---|---|---|---|---|---|---|---|
| 241 | 4 | ESI+:325 | | 277 | 3 | FAB+: 320 | | 313 | 2 | APCI-: 292 |
| 242 | 1 | FAB+: 294 | | 278 | 4 | ESI-: 330 | | 314 | 3 | FAB+: 377 |
| 243 | 2 | ESI-: 260 | | 279 | 4 | ESI-: 330 | | 315 | 3 | FAB+: 354 |
| 244 | 1 | EI+: 293 | | 280 | 4 | FAB-: 305 | | 316 | 4 | ESI-: 362 |
| 245 | 2 | ESI-: 260 | | 281 | 3 | FAB+:359 | | 317 | 4 | ESI-: 324 |
| 246 | 23, 1 | ESI+: 294 | | 282 | 4 | FAB-:330 | | 318 | 3 | ESI+: 395 |
| 247 | 2 | ESI-: 260 | | 283 | 3 | EI+:309 | | 319 | 3 | ESI+: 323 |
| 248 | 3 | ESI+: 346 | | 284 | 3 | EI+: 281 | | 320 | 3,32 | ESI+: 283 |
| 249 | 4 | ESI+: 332 | | 285 | 4 | FAB-: 322 | | 321 | 3 | FAB+: 436 |
| 250 | 4 | FAB+: 362 | | 286 | 4 | FAB+: 268 | | 322 | 4,32 | FAB-: 279 |
| 251 | 3 | FAB+: 375 | | 287 | 3 | FAB+:355 | | 323 | 4 | ESI-: 267 |
| 252 | 3 | FAB+: 375 | | 288 | 4 | FAB-:326 | | 324 | 4 | FAB+: 309 |
| 253 | 4 | FAB-: 360 | | 289 | 4 | FAB-:326 | | 325 | 4,32 | FAB-: 307 |
| 254 | 35 | EI+: 337 | | 290 | 3 | FAB+: 328 | | 326 | 1 | EI+: 263 |
| 255 | 4 | FAB+: 324 | | 291 | 34 | EI+: 325 | | 327 | 2 | EI+: 23 |
| 256 | 4 | FAB+: 310 | | 292 | 4 | FAB-: 312 | | 328 | 3 | EI+: 315 |
| 257 | 21 | ESI-: 216 | | 293 | 4 | ESI+: 310 | | 329 | 3 | EI+: 321 |
| 258 | 22 | ESI-: 244 | | 294 | 4 | FAB+: 311 | | 330 | 4 | FAB-: 300 |
| 259 | 23 | FAB+: 378 | | 295 | 3 | FAB+: 380 | | 331 | 4 | FAB-: 306 |
| 260 | 1 | ESI+: 306 | | 296 | 3 | FAB+: 357 | | 332 | 3 | FAB+: 368 |
| 261 | 2 | ESI-: 272 | | 297 | 4 | ESI-: 328 | | 333 | 32 | FAB+: 295 |
| 262 | 1 | ESI+: 308 | | 298 | 4 | FAB+:326 | | 334 | 32 | ESI+: 295 |
| 263 | 2 | ESI-: 274 | | 299 | 3 | FAB+:339 | | 335 | 4 | FAB+: 354 |
| 264 | 1 | ESI+: 304 | | 300 | 4 | FAB-:268 | | 336 | 4 | ESI-: 379 |
| 265 | 2 | ESI-: 270 | | 301 | 1 | EI+:287 | | 337 | 28 | FAB+: 309 |
| 266 | 3 | ESI+: 350 | | 302 | 2 | FAB+:332 | | 338 | 28 | FAB+: 309 |
| 267 | 3 | ESI+: 344 | | 303 | 22 | FAB+: 299 | | 339 | 4 | ESI+: 295 |
| 268 | 34 | EI+: 326 | | 304 | 4 | FAB-:340 | | 340 | 4 | ESI+: 295 |

(continued)

| REx | RSyn | Dat | | REx | RSyn | Dat | | REx | RSyn | Dat |
|---|---|---|---|---|---|---|---|---|---|---|
| 269 | 3 | CI+: 322 | | 305 | 3 | FAB+:355 | | 341 | 3, 4 | FAB-: 320 |
| 270 | 4 | FAB-: 306 | | 306 | 2 | FAB-:270 | | 342 | 3, 4 | FAB-: 320 |
| 271 | 4 | FAB+: 292 | | 307 | 1 | EI+:303 | | 343 | 3 | FAB+: 409 |
| 272 | 4 | FAB+: 312 | | 308 | 32 | FAB+: 335 | | 344 | 3 | ESI+: 296 |
| 273 | 1 | ESI+: 302 | | 309 | 34 | ESI+: 304 | | 345 | 5, 7 | ESI+: 376 |
| 274 | 2 | ESI+: 270 | | 310 | 4 | FAB+: 290 | | 346 | 7 | ESI+: 402 |
| 275 | 3 | FAB+: 345 | | 311 | 34 | ESI+:358 | | 347 | 4 | ESI+:309 |
| 276 | 3 | FAB+: 345 | | 312 | 4 | FAB+: 324 | | 348 | 4 | FAB+:297 |

[Table 27]

| REx | RSyn | Dat | | REx | RSyn | Dat | | REx | RSyn | Dat |
|---|---|---|---|---|---|---|---|---|---|---|
| 349 | 4 | FAB-:294 | | 368 | 4 | ESI-: 244 | | 387 | 3 | FAB+:314 |
| 350 | 2 | FAB-:254 | | 369 | 1 | ND | | 388 | 3 | FAB+:393 |
| 351 | 4 | FAB-:393 | | 370 | 2 | FAB-: 214 | | 389 | 33 | FAB+:255 |
| 352 | 3 | FAB+409 | | 371 | 3 | EI+: 299 | | 390 | 2,32 | FAB+:254 |
| 353 | 4 | FAB-:342 | | 372 | 4 | FAB-: 284 | | 391 | 4 | FAB+:300 |
| 354 | 3 | FAB+:374 | | 373 | 3 | ESI+:398 | | 392 | 3 | FAB+:357 |
| 355 | 4 | FAB+:311 | | 374 | 4 | FAB-:325 | | 393 | 27 | FAB+:383 |
| 356 | 3 | ESI+:325 | | 375 | 4 | FAB-:330 | | 394 | 32 | EI+:256 |
| 357 | 4 | FAB+:385 | | 376 | 4 | FAB-:330 | | 395 | 4 | ESI-: 280 |
| 358 | 4 | EI+:355 | | 377 | 3 | CI+: 346 | | 396 | 3 | EI+:345 |
| 359 | 3 | FAB+:311 | | 378 | 3 | ESI+:387 | | 397 | 1 | FAB+:363 |
| 360 | 4 | FAB+:371 | | 379 | 4 | ESI+:373 | | 398 | 34 | EI+: 306 |
| 361 | 28 | FAB+:323 | | 380 | 32 | EI+:248 | | 399 | 34 | ESI+: 338 |
| 362 | 32 | FAB+:309 | | 381 | 27 | FAB+:348 | | 400 | 24 | ESI+: 295 |
| 363 | 4 | FAB-:332 | | 382 | 1 | FAB+:286 | | 401 | 3 | FAB+: 394 |
| 364 | 37 | FAB+:347 | | 383 | 39 | EI+:248 | | 402 | 3 | EI+: 339 |
| 365 | 21 | ND | | 384 | 4 | FAB+:292 | | 403 | 4 | FAB-: 324 |
| 366 | 1 | FAB+: 264 | | 385 | 4 | FAB+:292 | | 404 | 4 | ESI+: 300 |
| 367 | 2 | ND | | 386 | 27 | EI+:305 | | 405 | 3 | FAB+: 314 |

[Table 28]

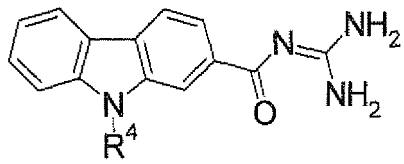

| Ex | Syn | $R^4$ | Sal | Dat |
|---|---|---|---|---|
| 1 | 1 | iPr | HCl | NMR : 1.69 (6H, d, *J* = 6.9 Hz), 5.31 (1H, sept, *J* = 6.9 Hz), 8.65 (1H, s). ; FAB+ : 295 |
| 2 | 2 | H | HCl | NMR : 7.24 (1H, dt, *J* = 7.3, 1.0 Hz), 7.51 (1H, dt, *J* = 7.3, 1.0 Hz), 8.33 (1H, s). ; FAB+ : 253 |
| 3 | 3 | 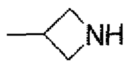 | 2HCl | NMR : 4.52 (2H, dd, *J* = 8.3, 8.3 Hz), 4.99 (2H, dd, *J* = 8.3, 8.3 Hz), 8.76 (1H, s). ; FAB+ : 308 |
| 4 | 4 | $-(CH_2)_2OH$ | HCl | NMR : 3.84 (2H, t, *J* = 5.4 Hz), 4.58 (2H, t, *J* = 5.4 Hz), 8.67 (1H, s). ; FAB+ : 297 |
| 12 | 1 | Me | HCl | NMR : 4.01 (3H, s), 7.29 (1H, dt, *J* = 7.3, 1.0 Hz), 8.71(1H, d, *J* = 1.5 Hz). ; FAB+ : 267 |
| 13 | 2 | Et | HCl | NMR : 1.37 (3H, t, *J* = 7.3 Hz), 4.59 (2H, q, *J* = 7.3 Hz), 8.72 (1H, d, *J* = 1.5 Hz). ; FAB+ : 281 |
| 14 | 1 | nPr | HCl | NMR : 0.92 (3H, t, *J* = 7.3 Hz), 1.86 (2H, tq, *J* = 7.3, 7.3 Hz), 8.78 (1H, s). ; FAB+ : 295 |
| 15 | 1 | nBu | HCl | NMR : 0.89 (3H, t, *J* = 7.3 Hz), 1.35 (2H, tq, *J* = 7.4, 7.3 Hz), 8.74 (1H, d, *J* = 1.5 Hz). ; FAB+: 309 |
| 16 | 1 | nPen | HCl | NMR : 0.81 (3H, t, *J* = 6.8 Hz), 1.82 (2H, tt, *J* = 7.4, 6.8 Hz), 8.70 (1H, d, *J* =1.5 Hz). ; FAB+ : 323 |
| 17 | 1 | $-CH(Et)_2$ | HCl | NMR : 0.66 (6H, t, *J* = 6.4 Hz), 4.60-5.00 (1H, m), 8.89 (1H, s). ; ESI+ : 323 |
| 18 | 2 | $-(CH_2)_2OMe$ | HCl | NMR : 3.18 (3H, s), 4.69 (2H, t, *J* = 5.2 Hz), 8.58 (1H, s). ; ESI+: 311 |
| 19 | 1 | $-(CH_2)_2OBn$ | HCl | NMR : 3.90 (2H, t, *J* = 4.9 Hz), 4.45 (2H, s), 8.84(1H, s). ; FAB+ : 387 |
| 20 | 1 | $-(CH_2)_3OMe$ | HCl | NMR : 2.07 (2H, tt, *J* = 6.9, 6.3 Hz), 3.20 (3H, s), 8.65 (1H, d, *J* = 0.9 Hz). ; FAB+ : 325 |
| 21 | 2 | $-(CH_2)_2N(Me)_2$ | 2HCl | NMR : 2.97 (6H, s), 4.96 (2H, brt, *J* = 7.8 Hz), 8.83 (1H, s). ; FAB+ : 324 |
| 22 | 1 | cBu | HCl | NMR : 1.92-2.00 (1H, m), 5.49 (1H, quint, *J* = 8.8 Hz), 8.57 (1H, d, *J* = 1.6 Hz). ; ESI+ : 307 |
| 23 | 1 | cPen | HCl | NMR : 1.75-1.88 (2H, m), 5.47 (1H, quint, *J* = 9.0 Hz), 8.58 (1H, s). ; FAB+ : 321 |
| 24 | 1 | cHex | HCl | NMR : 1.64-1.77 (4H, m), 1.84-1.93 (4H, m), 8.80 (1H, s). ; FAB+ : 335 |

[Table 29]

| Ex | Syn | $R^4$ | Sal | Dat |
|---|---|---|---|---|
| 25 | 1 | 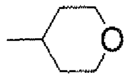 | HCl | NMR : 1.82 (2H, brd, J = 11.5 Hz), 4.09 (2H, brdd, J = 11.5, 2.0 Hz), 8.65-8.88 (3H, m). ; FAB+ : 337 |
| 6 | 6 | 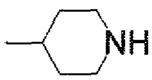 | 2HCl | NMR : 2.01 (2H, brd, *J* = 11.2 Hz), 5.33-5.43 (1H, m), 8.99 (1H, s). ; FAB+ : 336 |
| 26 | 1 | Ac | HCl | NMR : 3.00 (3H, s), 8.40 (1H, d, *J* = 8.3 Hz), 8.93 (1H, d, *J* = 0.9 Hz). ; FAB+ : 295 |
| 27 | 1 | -C(O)-iPr | HCl | NMR : 1.35 (6H, d, *J* = 6.3 Hz), 3.88 (1H, sept, *J* = 6.3 Hz), 8.94 (1H, s). ; FAB+ : 323 |
| 28 | 1 | $-S(O)_2$-iPr | HCl | NMR : 1.20 (6H, d, *J* = 6.8 Hz), 4.10 (1H, sept, *J* = 6.8 Hz), 8.69 (1H, d, *J* = 1.4 Hz). ; FAB+ : 359 |
| 29 | 1 | $-C(O)-NMe_2$ | HCl | NMR : 3.09 (6H, s), 7.38-7.43 (1H, m), 8.37 (1H, s). ; FAB+ : 324 |
| 30 | 1 | 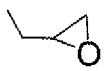 | HCl | NMR : 3.78 (1H, dd, *J* = 5.9, 11.2 Hz), 3.85 (1H, dd, *J* = 11.2, 3.9 Hz), 8.74 (1R, s). ; EEI+ :308 |
| 31 | 2 | 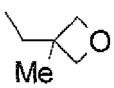 | | NMR : 1.34 (3H, s), 4.54 (2H, s), 8.27 (1H, s). ; FAB+ : 337 |
| 32 | 1 | $-CH_2$-cPr | HCl | NMR : 0.43-0.47 (2H, m), 4.48 (2H, d, *J* = 7.3 Hz), 8.79 (1H, d, *J* = 1.5 Hz). ; FAB+ : 307 |
| 33 | 1 | 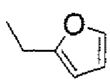 | HCl | NMR : 5.80 (2H, s), 6.37 (1H, dd, *J* = 3.4, 2.0 Hz), 8.91 (1H, s). ; FAB+ : 333 |
| 34 | 1 | 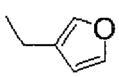 | HCl | NMR : 5.62 (2H, s), 6.37 (1H, d, *J* = 1.5 Hz), 8.90 (1H, d, *J* = 1.4 Hz). ; FAB+ : 333 |
| 35 | 1 | $-CH_2CH_2Ph$ | HCl | NMR : 3.13 (2H, t, *J* = 7.3 Hz), 4.74 (2H, t, *J* = 7.3 Hz), 8.67 (1H, d, *J* = 1.0 Hz). ; FAB+ : 357 |
| 36 | 1 | 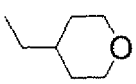 | HCl | NMR : 3.18 (2H, dd, J = 11.7, 11.7 Hz), 3.79 (2H, dd, *J* = 11.7, 2.4 Hz), 8.84 (1H, d, *J* = 0.9 Hz). ; FAB+: 351 |
| 37 | 1 | Bn | HCl | NMR : 5.83 (2H, s), 8.40 (1H, d, *J* = 8.3 Hz), 8.77 (1H, s). ; FAB+ : 343 |
| 38 | 1 | $-S(O)_2$-Me | HCl | NMR : 3.29 (3H, s), 7.48 (1H, t, *J* = 7.8 Hz), 8.79 (1H, s). ; FAB+ : 331 |
| 39 | 2 | 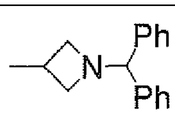 | | NMR : 4.76 (1H, s), 5.45-5.48 (1H, m), 8.73 (1H, s). ; ESI+ : 474 |
| 40 | 1 | 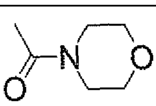 | HCl | NMR : 3.53-3.63 (4H, m), 7.42 (1H, t, *J* = 7.8 Hz), 8.45 (1H, d, *J* = 1.0 Hz). ; FAB+ : 366 |

[Table 30]

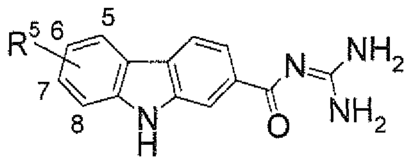

| Ex | Syn | $R^5$ | Sal | Dat |
|---|---|---|---|---|
| 41 | 2 | 5-F | HCl | NMR : 7.04 (1H, dd, *J* = 10.3, 7.8 Hz), 7.51 (1H, dt, *J* = 7.8, 5.6 Hz), 8.35 (1H, s). ; FAB+ : 271 |
| 42 | 2 | 7-F | HCl | NMR : 7.09 (1H, ddd, *J* = 9.5, 8.8, 2.5 Hz), 7.35 (1H, dd, *J* = 9.8, 2.5 Hz), 8.32 (1H, s). ; FAB+ : 271 |

[Table 31]

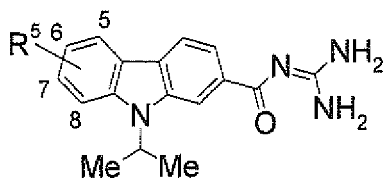

| Ex | Syn | $R^5$ | Sal | Dat |
|---|---|---|---|---|
| 5 | 5 | 6-$NH_2$ | 2HCl | NMR : 1.69 (6H, d, *J* = 6.8 Hz), 7.55 (1H, d, *J* = 8.8 Hz), 8.74 (1H, s). ; FAB+: 310 |
| 43 | 2 | 5-F | HCl | NMR : 1.70 (6H, d, *J* = 6.8 Hz), 7.08 (1H, dd, *J* = 10.3, 7.9 Hz), 8.74 (1H, s). ; FAB+ : 313 |
| 44 | 2 | 6-F | HCl | NMR : 1.68 (6H, d, *J* = 7.4 Hz), 7.40 (1H, dt, *J* = 9.3, 2.8 Hz), 8.64 (1H, s). ; FAB+ : 313 |
| 45 | 2 | 7-F | HCl | NMR : 1.68 (6H, d, *J*= 6.9 Hz), 7.12 (1H, dt, *J* = 9.1, 2.0 Hz), 8.64 (1H, s). ; FAB+: 313 |
| 46 | 2 | 8-F | HCl | NMR : 1.68 (6H, d, *J* = 6.9 Hz), 7.12 (1H, dt, *J* = 9.1, 2.0 Hz), 8.64 (1H, s). ; FAB+: 313 |
| 47 | 2 | 5-Me | HCl | NMR : 1.69 (6H, d, *J* = 7.3 Hz), 2.85 (3H, s), 8.64 (1H,s). ; FAB+ : 309 |
| 48 | 2 | 6-Me | HCl | NMR : 1.67 (6H, d, *J* = 7.3 Hz), 2.49(3H, s), 8.62 (1H, s). ; FAB+ : 309 |
| 49 | 2 | 7-Me | HCl | NMR : 1.69 (6H, d, *J* = 6.8 Hz), 2.54 (3H, s), 8.55 (1H, s). ; FAB+ : 309 |
| 50 | 2 | 8-Me | HCl | NMR : 1.75 (6H, d, *J* = 6.8 Hz), 2.82 (3H, s), 8.47 (1H, s). ; FAB+ : 309 |
| 51 | 2 | 5-OMe | HCl | NMR : 1.68 (6H, d, *J* = 6.8 Hz), 4.06 (3H, s), 8.61 (1H, s). ; FAB+ : 325 |
| 52 | 2 | 6-OMe | HCl | NMR : 1.66 (6H, d, *J* = 6.9 Hz), 3.87 (3H, s), 8.53 (1H, s). ; ESI+ : 325 |
| 53 | 2 | 7-OMe | HCl | NMR : 1.69 (6H, d, *J* = 6.8 Hz), 3.92 (3H, s), 8.56 (1H, s). ; FAB+: 325 |
| 54 | 2 | 8-OMe | HCl | NMR : 1.68 (6H, d, *J*= 6.8 Hz), 4.01 (3H, s), 8.59 (1H, s). ; ESI+ : 325 |
| 55 | 1 | 5-CN | HCl | NMR : 1.72 (6H, d, *J* = 6.8 Hz), 7.72 (1H, t, *J* = 7.3 Hz), 8.79 (1H, s). ; FAB+ : 320 |
| 56 | 1 | 6-CN | HCl | NMR : 1.71 (6H, d, *J* = 6.8 Hz), 7.89 (1H, dd, *J* = 8.8, 1.5 Hz), 8.90 (1H, d, *J* =1.5 Hz). ; FAB+ : 320 |

(continued)

| Ex | Syn | $R^5$ | Sal | Dat |
|---|---|---|---|---|
| 57 | 1 | 7-CN | HCl | NMR : 1.72 (6H, d, *J* = 7.4 Hz), 7.65 (1H, dd, *J* = 8.3, 1.0 Hz), 8.71 (1H, s). ; FAB+: 320 |
| 58 | 1 | 8-CN | HCl | NMR : 1.83 (6H, d, *J* = 7.3 Hz), 7.43 (1H, t, *J* = 7.8 Hz), 8.64 (1H, s). ; FAB+ : 320 |

[Table 32]

| Ex | Syn | $R^5$ | Sal | Dat |
|---|---|---|---|---|
| 59 | 2 | 5-Cl | HCl | NMR : 1.70 (6H, d, *J* = 6.9 Hz), 7.34 (1H, t, *J* = 7.8 Hz), 8.72 (1H, s). ; FAB+:329 |
| 60 | 2 | 6-Cl | HCl | NMR : 1.68 (6H, d, *J* = 6.9 Hz), 7.54 (1H, dd, *J* = 8.7, 1.9 Hz), 8.65 (1H, s). ; FAB+:329 |
| 61 | 2 | 7-Cl | HCl | NMR : 1.69 (6H, d, *J* = 6.8 Hz), 7.30 (1H, dd, *J* = 8.3, 2.0 Hz), 8.65 (1H, s). ; FAB+ : 329 |
| 62 | 2 | 8-Cl | HCl | NMR : 1.76 (6H, d, *J* = 7.3 Hz), 7.27 (1H, t, *J* = 7.8 Hz), 8.54 (1H, s). ; FAB+:329 |
| 63 | 1 | 6-$NO_2$ | HCl | NMR : 1.73 (6H, d, *J* = 6.8 Hz), 8.02 (1H, d, *J* = 9.2 Hz), 8.77 (1H, s). ; FAB+: 340 |
| 64 | 2 | 5-$CH_2NMe_2$ | 2HCl | NMR : 1.71 (6H, d, *J* = 6.8 Hz), 2.89 (6H, s), 8.81 (1H,s). ; FAB+: 352 |
| 65 | 2 | 5-$CH_2OH$ | HCl | NMR : 1.69 (6H, d, *J*= 7.3 Hz), 5.10 (2H, s), 8.66 (1H, s). ; FAB+: 325 |
| 66 | 2 | 5-$CH_2OMe$ | HCl | NMR : 1.70 (6H, d, *J* = 6.8 Hz), 3.42 (3H, s), 8.65 (1H, s). ; FAB+ : 339 |
| 67 | 1 | 5-C(O)H | HCl | NMR : 1.73 (6H, d, *J* = 6.8 Hz), 8.71 (1H, s), 10.44 (1H, s). ; FAB+ : 323 |

[Table 33]

| Ex | Syn | Str | Sal | Dat |
|---|---|---|---|---|
| 68 | 1 | 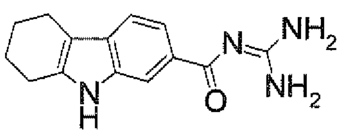 | HCl | NMR : 1.76-1.89 (4H, m), 2.63-2.68 (2H, m), 8.15 (1H, d, *J* = 1.5 Hz). ; FAB+ : 257 |
| 69 | 1 | 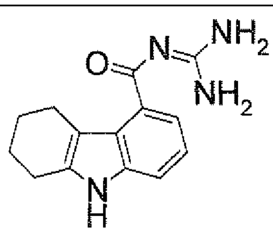 | HCl | NMR : 1.71-1.79 (2H, m), 1.79-1.86 (2H, m), 7.11 (1H, t, *J* = 7.9 Hz). ; FAB+ : 257 |
| 70 | 1 | 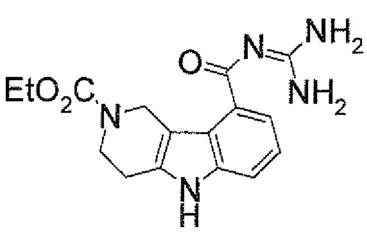 | HCl | NMR : 1.20 (3H, t, *J* = 6.9 Hz), 2.86 (2H, brt, *J* = 5.3 Hz), 7.18 (1H, t, *J* = 7.8 Hz). ; FAB+:330 |

(continued)

| Ex | Syn | Str | Sal | Dat |
|---|---|---|---|---|
| 71 | 1 | 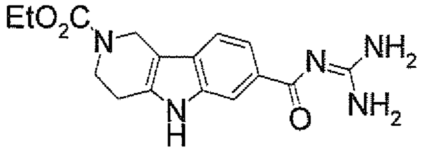 | HCl | NMR : 1.22 (3H, t, *J* = 6.9 Hz), 2.87 (2H, brt, *J* = 5.6Hz), 8.18 (1H, s). ; FAB+ : 330 |

[Table 34]

| Ex | Sal | Str | Ex | Sal | Str |
|---|---|---|---|---|---|
| 7 | HCl | 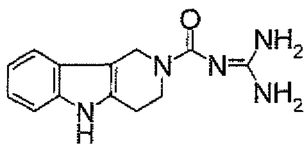 | 75 | HCl | 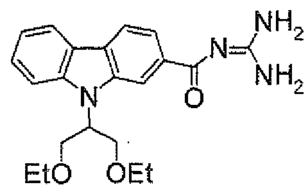 |
| 8 | | 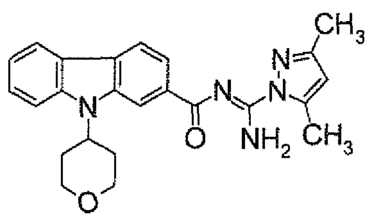 | 76 | HCl | 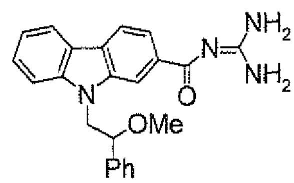 |
| 9 | 2HCl | 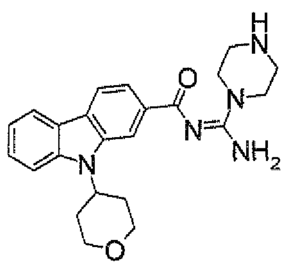 | 77 | HCl | 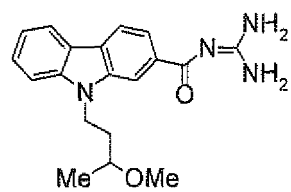 |
| | | | 78 | HCl | 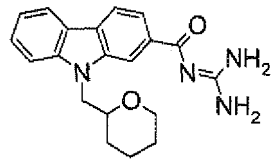 |
| 10 | HCl | 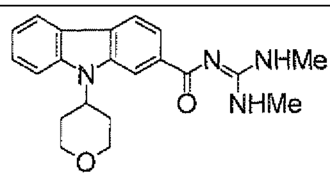 | 79 | HCl | 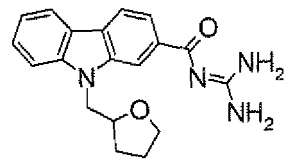 |
| 11 | HCl | 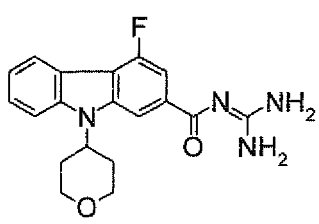 | 80 | HCl | 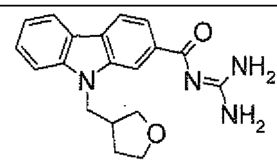 |
| 72 | HCl | 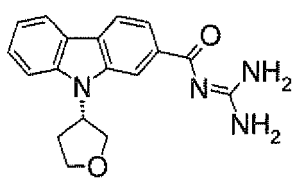 | 81 | HCl | 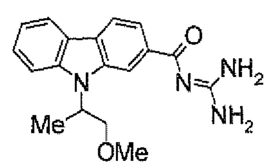 |

(continued)

| Ex | Sal | Str | | Ex | Sal | Str |
|---|---|---|---|---|---|---|
| 73 | HCl | 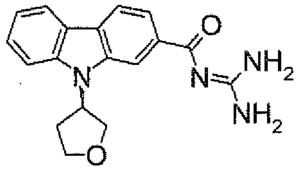 | | 82 | HCl | 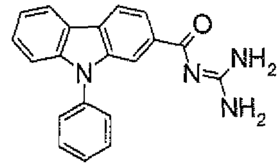 |
| 74 | HCl | 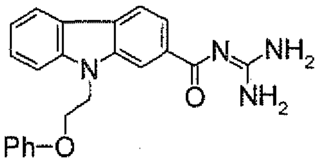 | | 83 | 2HCl | 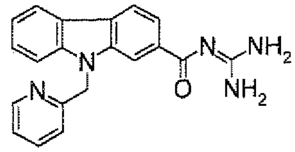 |

[Table 35]

| Ex | Sal | Str | | Ex | Sal | Str |
|---|---|---|---|---|---|---|
| 84 | 2HCl | 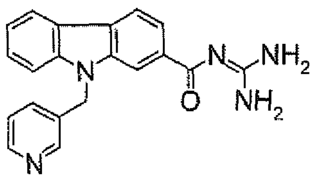 | | 92 | HCl | 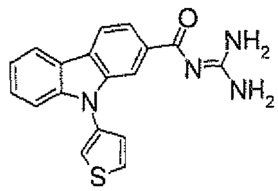 |
| 85 | HCl | 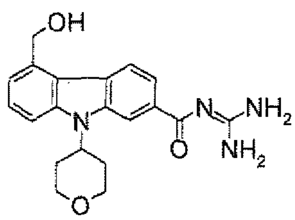 | | 93 | HCl | 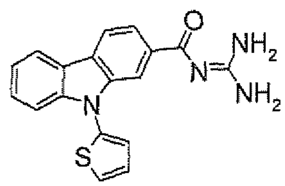 |
| 86 | HCl | 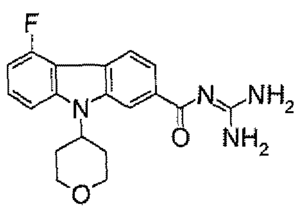 | | 94 | HCl | 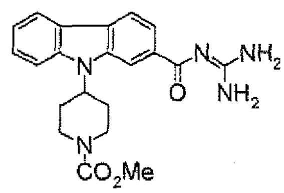 |
| 87 | HCl | 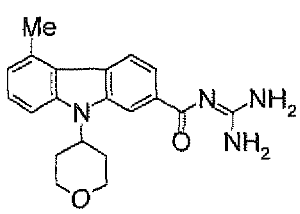 | | 95 | HCl | 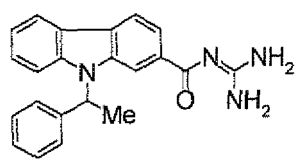 |
| | | | | 96 | HCl | 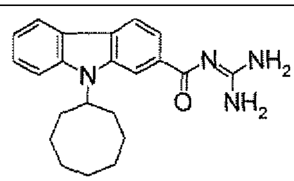 |

(continued)

| Ex | Sal | Str | | Ex | Sal | Str |
|---|---|---|---|---|---|---|
| 88 | 2HCl | 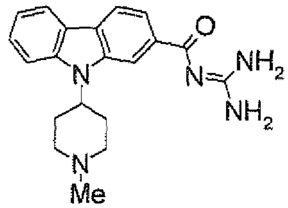 | | 97 | 2HCl | 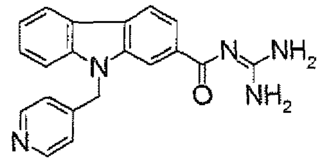 |
| 89 | 2HCl | 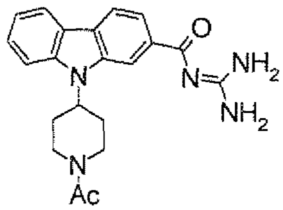 | | 98 | HCl | 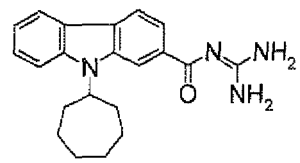 |
| 90 | HCl | 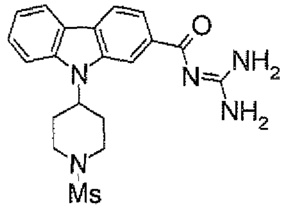 | | 99 | HCl | 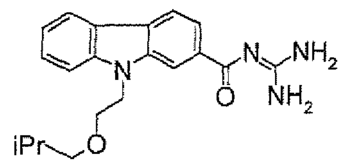 |
| 91 | 2HCl | 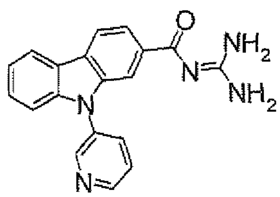 | | 100 | HCl | 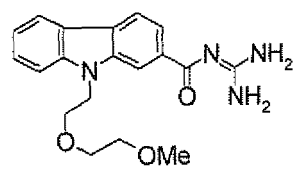 |

[Table 36]

| Ex | Sal | Str | | Ex | Sal | Str |
|---|---|---|---|---|---|---|
| 101 | Oxal | 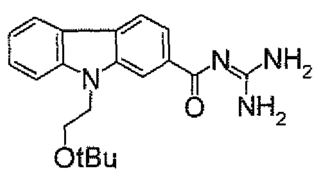 | | 109 | HCl | 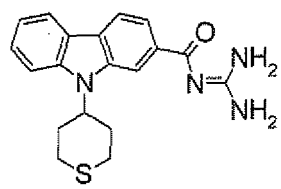 |
| 102 | 2HCl | 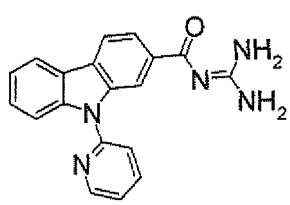 | | 110 | HCl | 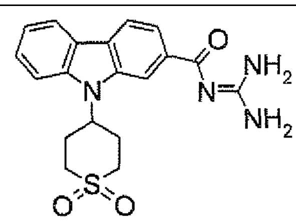 |

(continued)

| Ex | Sal | Str | Ex | Sal | Str |
|---|---|---|---|---|---|
| 103 | HCl | 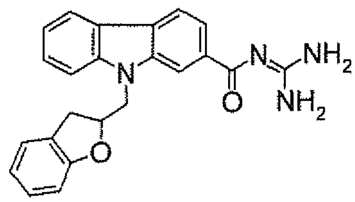 | 111 | 2HCl | 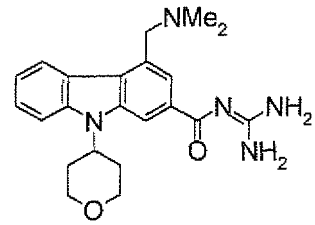 |
| 104 | HCl | 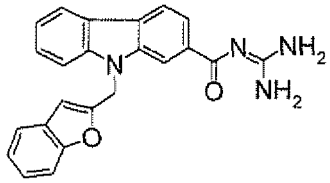 | 112 | HCl | 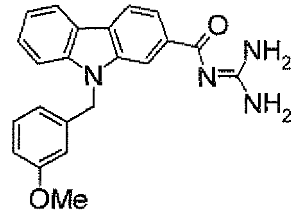 |
| 105 | HCl | 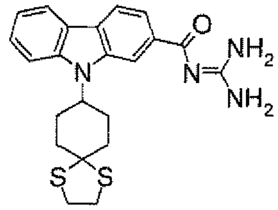 | 113 | HCl | 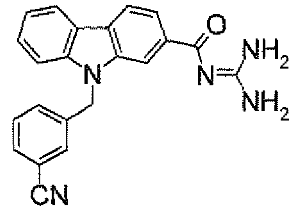 |
| 106 | HCl | 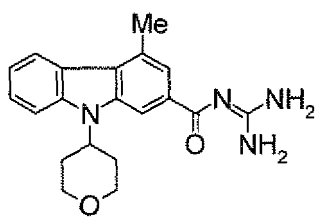 | 114 | HCl | 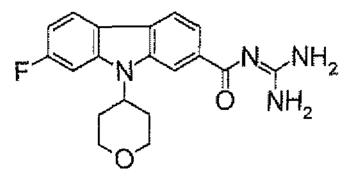 |
| 107 | HCl | 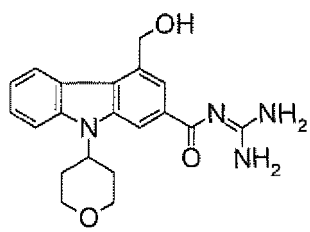 | 115 | HCl | 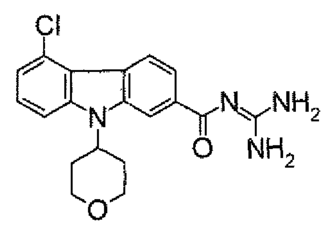 |
| 108 | 2HCl | 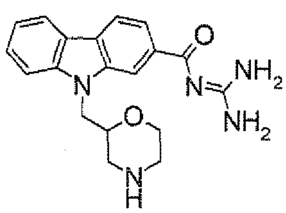 | 116 | HCl | 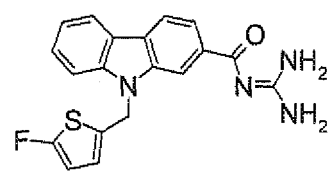 |

[Table 37]

| Ex | Sal | Str | Ex | Sal | Str |
|---|---|---|---|---|---|
| 117 | HCl | 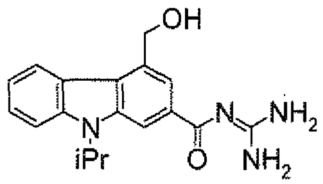 | 125 | HCl | 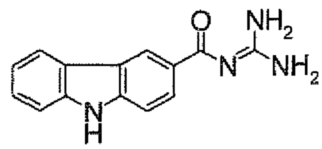 |
| 118 | HCl | 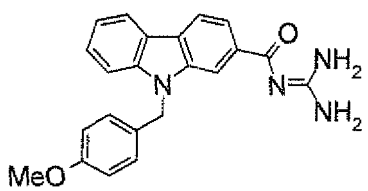 | 126 | HCl | 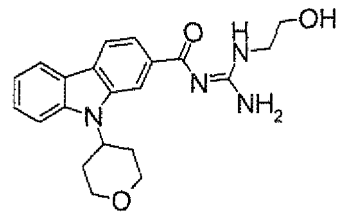 |
| 119 | HCl | 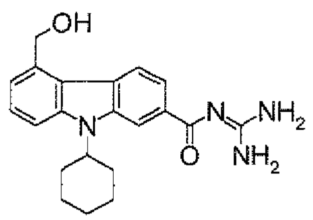 | 127 | 2HCl | 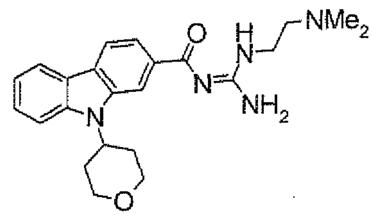 |
| 120 | HCl | 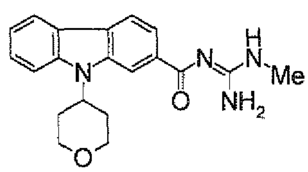 | 128 | HCl | 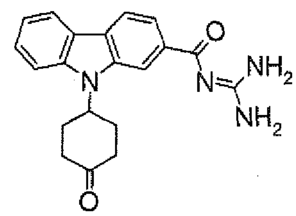 |
| 121 | HCl | 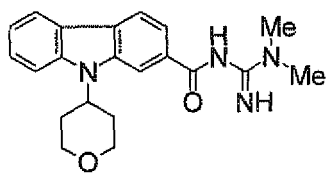 | 129 | HCl | 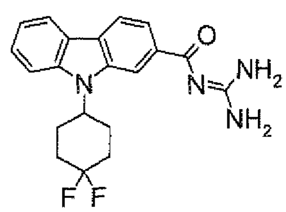 |
| 122 | HCl | 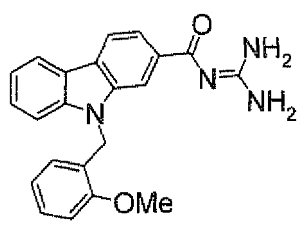 | 130 | 2HCl | 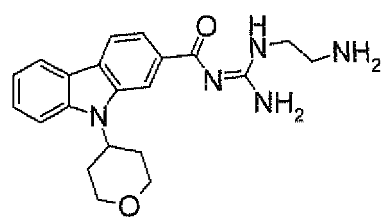 |

(continued)

| Ex | Sal | Str | | Ex | Sal | Str |
|---|---|---|---|---|---|---|
| 123 | HCl | 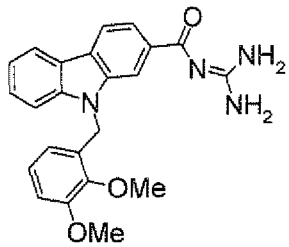 | | 131 | HCl | 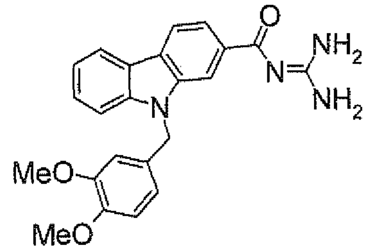 |
| 124 | HCl | 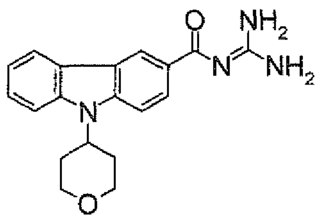 | | | | |

[Table 38]

| Ex | Sal | Str | | Ex | Sal | Str |
|---|---|---|---|---|---|---|
| 132 | HCl | 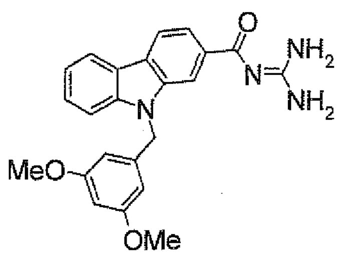 | | 139 | HCl | 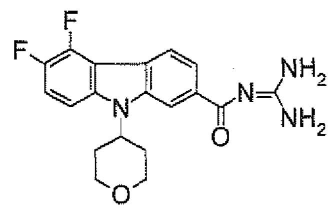 |
| 133 | HCl | 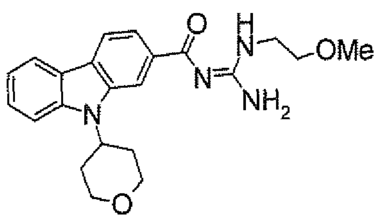 | | 140 | HCl | 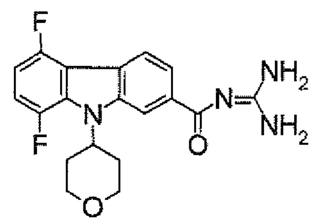 |
| 134 | HCl | 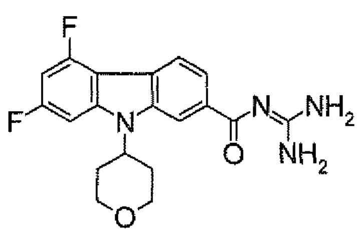 | | 141 | HCl | 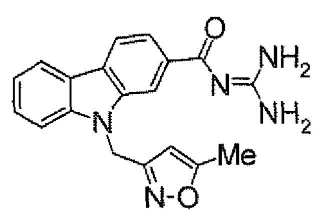 |

(continued)

| Ex | Sal | Str | | Ex | Sal | Str |
|---|---|---|---|---|---|---|
| 135 | HCl | 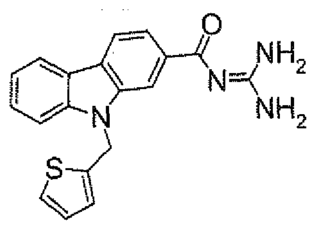 | | 142 | HCl | 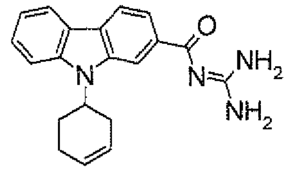 |
| | | | | 143 | HCl | 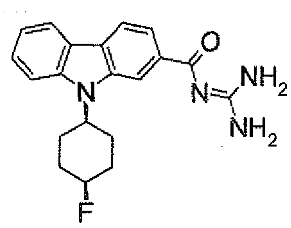 |
| 136 | HCl | 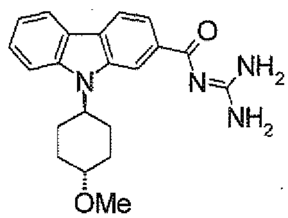 | | 144 | HCl | 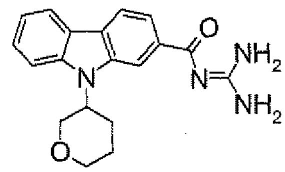 |
| 137 | HCl | 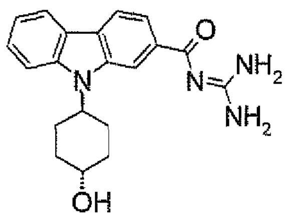 | | 145 | HCl | 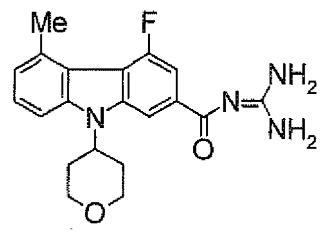 |
| 138 | HCl | 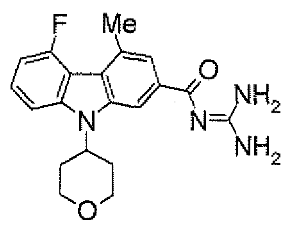 | | 146 | HCl | 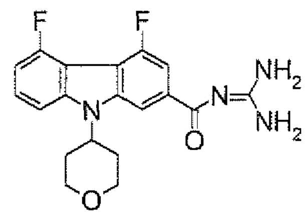 |

[Table 39]

| Ex | Sal | Str | | Ex | Sal | Str |
|---|---|---|---|---|---|---|
| 147 | HCl | 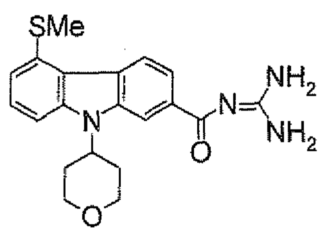 | | 154 | HCl | 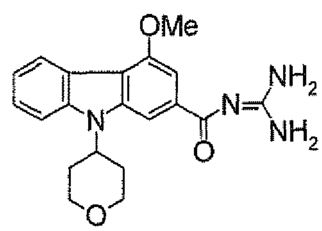 |

(continued)

| Ex | Sal | Str | | Ex | Sal | Str |
|---|---|---|---|---|---|---|
| 148 | HCl | 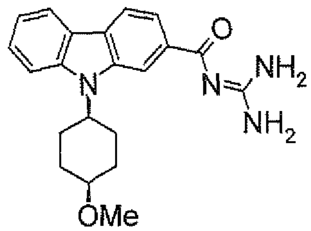 | | 155 | HCl | 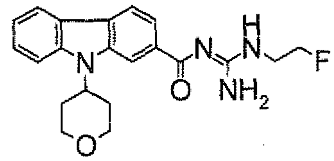 |
| 149 | HCl | 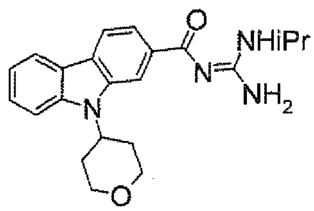 | | 156 | HCl | 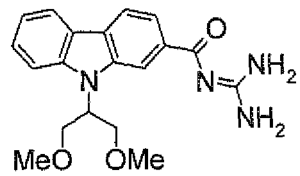 |
| 150 | HCl | 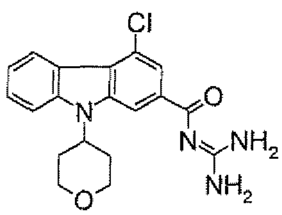 | | 157 | HCl | 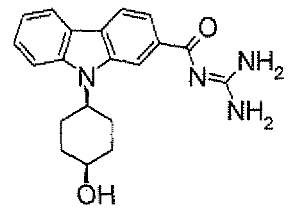 |
| | | | | 158 | HCl | 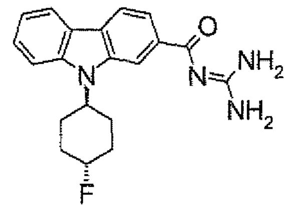 |
| 151 | HCl | 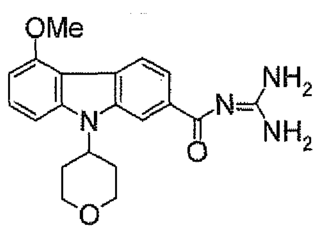 | | 159 | HCl | 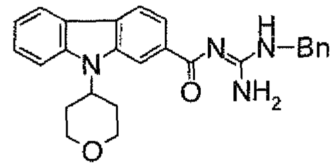 |
| 152 | HCl | 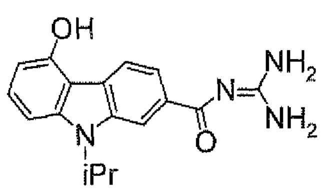 | | 160 | HCl | 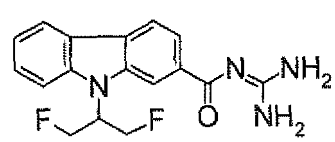 |

(continued)

| Ex | Sal | Str | | Ex | Sal | Str |
|---|---|---|---|---|---|---|
| 153 | HCl | 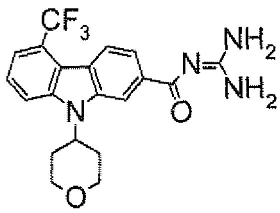 | | 161 | HCl | 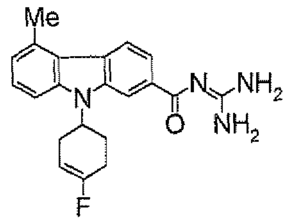 |
| | | | | 162 | HCl | 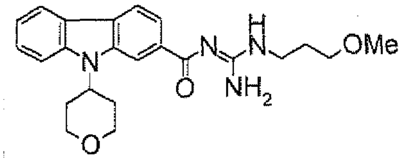 |

[Table 40]

| Ex | Sal | Str | | Ex | Sal | Str |
|---|---|---|---|---|---|---|
| 163 | HCl | 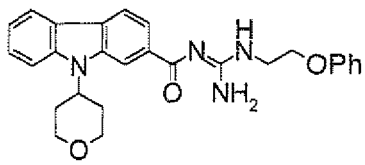 | | 173 | 2HCl | 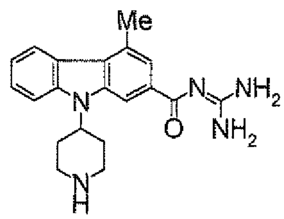 |
| 164 | HCl | 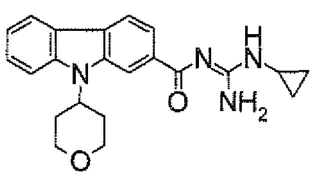 | | 174 | 2HCl | 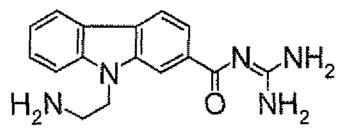 |
| 165 | HCl | 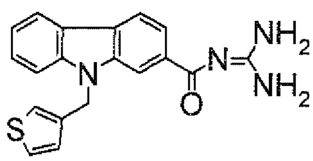 | | 175 | 2HCl | 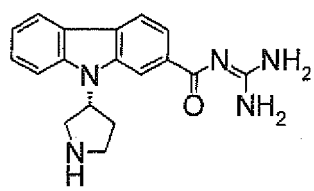 |
| 166 | HCl | 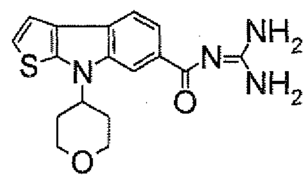 | | 176 | HCl | 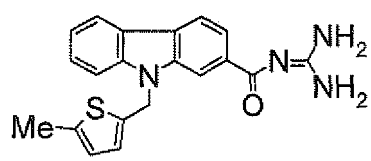 |
| 167 | 2HCl | 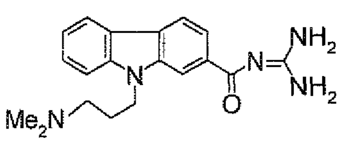 | | 177 | HCl | 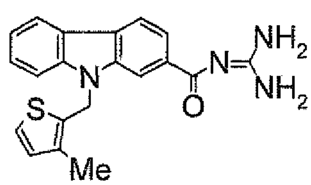 |

(continued)

| Ex | Sal | Str | | Ex | Sal | Str |
|---|---|---|---|---|---|---|
| 168 | 2HCl | 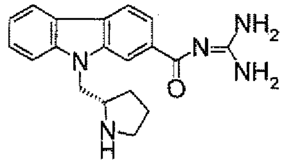 | | 178 | HCl | 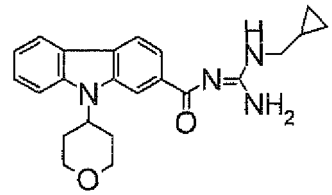 |
| 169 | Oxal | 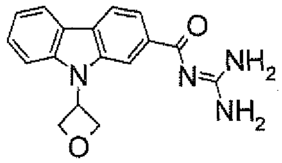 | | | | |
| 170 | 2HCl | 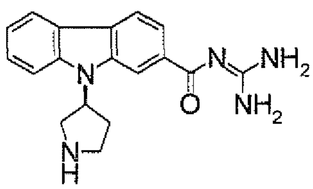 | | 179 | HCl | 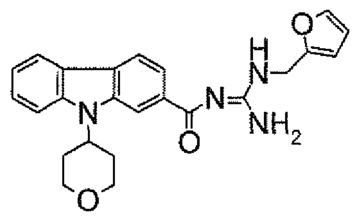 |
| 171 | 2HCl | 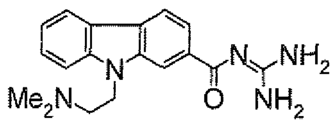 | | 180 | HCl | 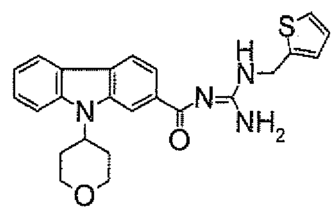 |
| 172 | 2HCl | 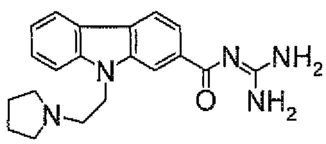 | | 181 | HCl | 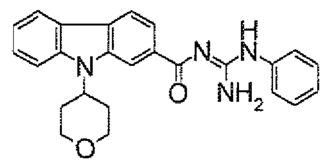 |

[Table 41]

| Ex | Sal | Str | | Ex | Sal | Str |
|---|---|---|---|---|---|---|
| 182 | HCl | 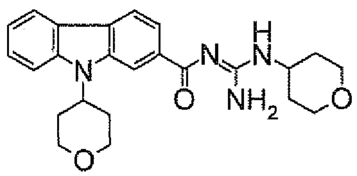 | | 191 | 2HCl | 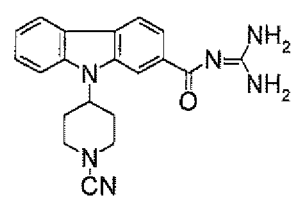 |
| 183 | 2HCl | 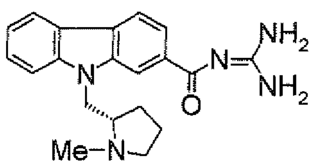 | | 192 | 2HCl | 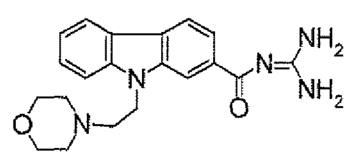 |

(continued)

| Ex | Sal | Str | | Ex | Sal | Str |
|---|---|---|---|---|---|---|
| 184 | 2HCl | 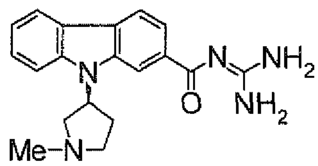 | | 193 | 2HCl | 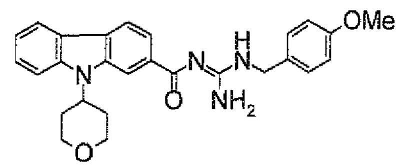 |
| 185 | 2HCl | 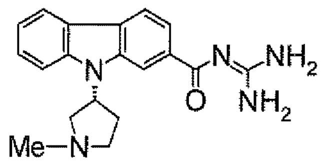 | | 194 | HCl | 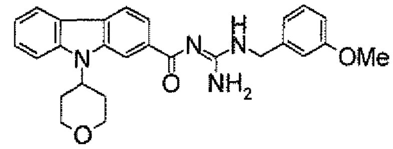 |
| 186 | HCl | 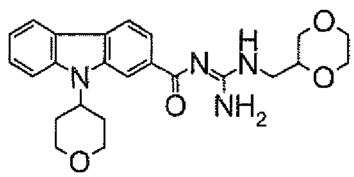 | | 195 | HCl | 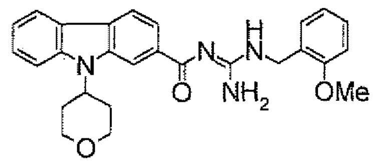 |
| 187 | HCl | 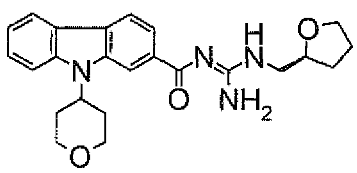 | | 196 | HCl | 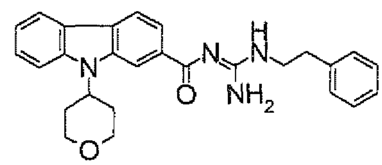 |
| 188 | HCl | 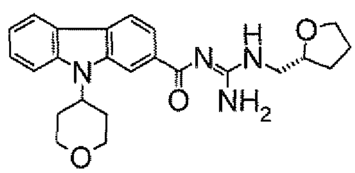 | | 197 | HCl | 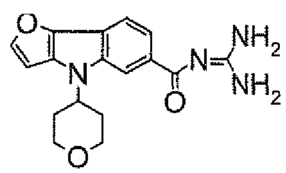 |
| 189 | 2HCl | 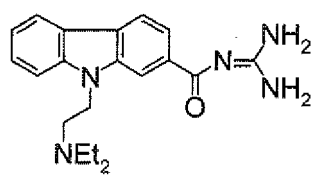 | | 198 | 2HCl | 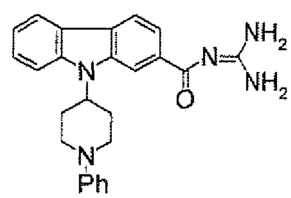 |
| 190 | 2HCl | 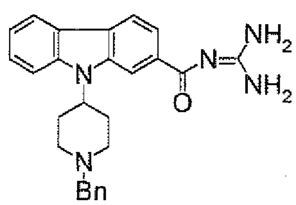 | | 199 | 2HCl | 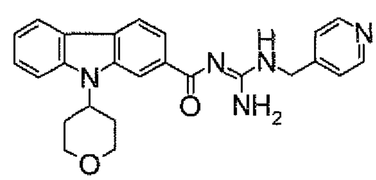 |

[Table 42]

| Ex | Sal | Str | Ex | Sal | Str |
|---|---|---|---|---|---|
| 200 | 2HCl | 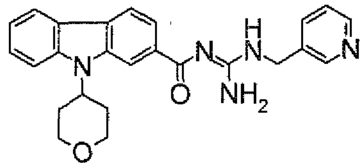 | 208 | 2HCl | 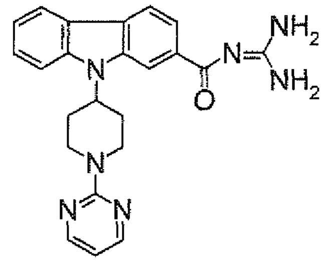 |
| 201 | 2HCl | 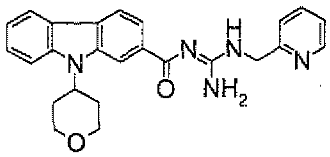 | 209 | HCl | 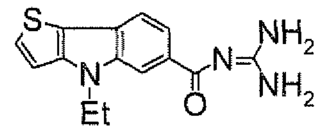 |
| 202 | 2HCl | 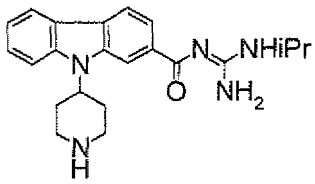 | 210 | HCl | 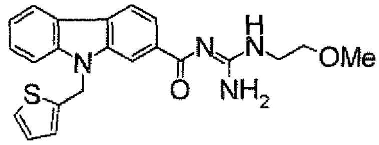 |
| 203 | 2HCl | 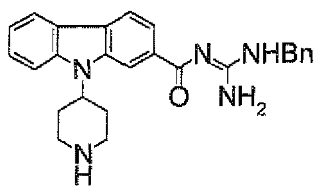 | 211 | HCl | 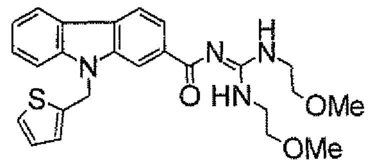 |
| 204 | HCl | 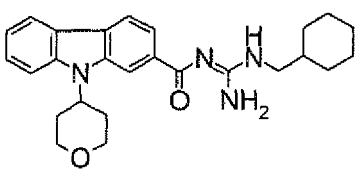 | 212 | HCl | 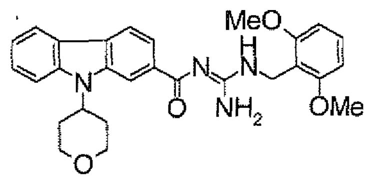 |
| 205 | HCl | 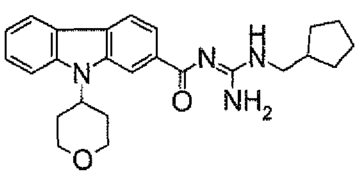 | 213 | HCl | 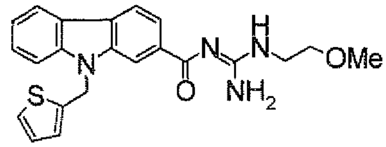 |
| 206 | HCl | 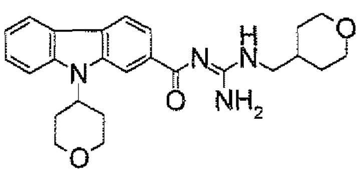 | 214 | HCl | 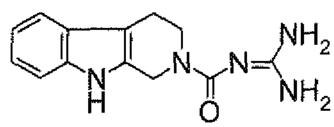 |
| 207 | HCl | 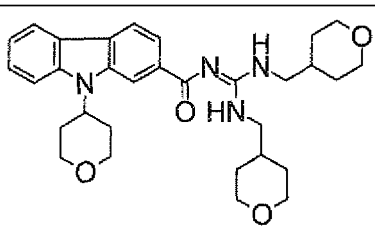 | 215 | HCl | 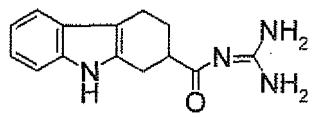 |

(continued)

| Ex | Sal | Str | | Ex | Sal | Str |
|---|---|---|---|---|---|---|
| | | | | 216 | Oxal | 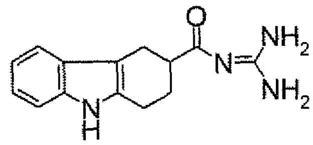 |

[Table 43]

| Ex | Sal | Str | | Ex | Sal | Str |
|---|---|---|---|---|---|---|
| 217 | Oxal | 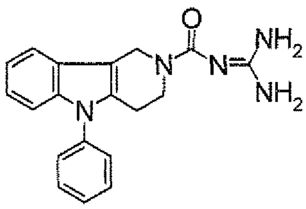 | | 225 | | 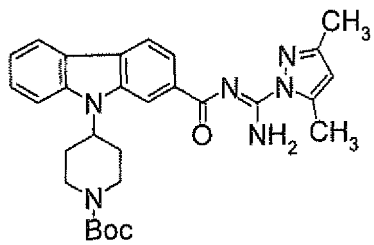 |
| 218 | HCl | 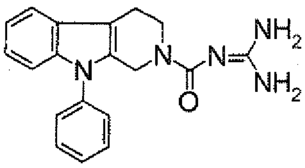 | | 226 | | 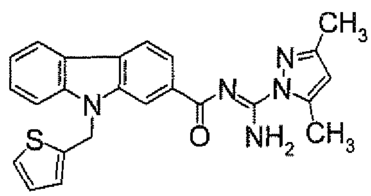 |
| 219 | | 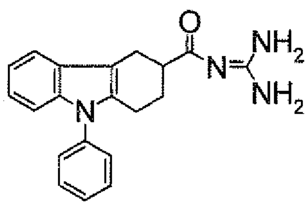 | | 227 | | 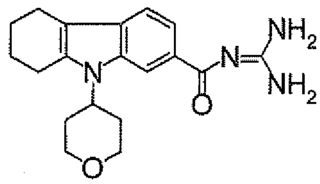 |

(continued)

| Ex | Sal | Str | | Ex | Sal | Str |
|---|---|---|---|---|---|---|
| 220 | Oxal | 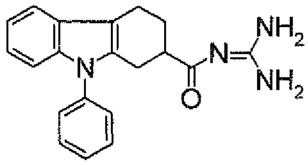 | | | | |
| 221 | HCl | 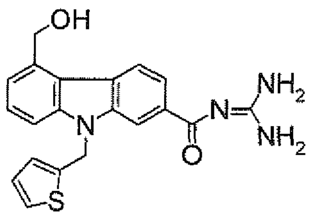 | | | | |
| 222 | Oxal | 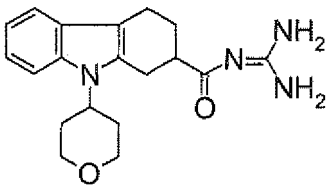 | | | | |
| 223 | Oxal | 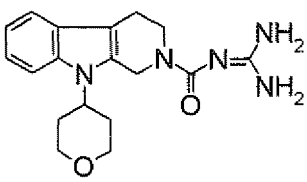 | | | | |
| 224 | | 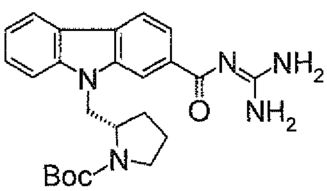 | | | | |

[Table 44]

| Ex | Syn | Dat (MASS) | | Ex | Syn | Dat (MASS) | | Ex | Syn | Dat (MASS) |
|---|---|---|---|---|---|---|---|---|---|---|
| 7 | 7 | FAB+:258 | | 103 | 1 | FAB+:385 | | 139 | 1 | FAB+: 373 |
| 8 | 8 | ESI+: 416 | | 104 | 1 | FAB+:383 | | 140 | 1 | FAB+: 373 |
| 9 | 9 | ESI+:406 | | 105 | 1 | FAB+:425 | | 141 | 1 | ESI+: 348 |
| 10 | 10 | FAB+: 365 | | 106 | 11 | FAB+: 351 | | 142 | 1 | FAB+: 333 |
| 11 | 11 | FAB+: 355 | | 107 | 11 | FAB+: 367 | | 143 | 1 | ESI+: 353 |
| 72 | 1 | FAB+: 323 | | 108 | 1,6 | ESI+: 352 | | 144 | 1 | FAB+: 337 |
| 73 | 1 | FAB+: 323 | | 109 | 1 | FAB+:353 | | 145 | 1 | FAB+: 369 |
| 74 | 1 | ESI+:373 | | 110 | 1 | Fay+:385 | | 146 | 1 | FAB+: 373 |

(continued)

| Ex | Syn | Dat (MASS) | | Ex | Syn | Dat (MASS) | | Ex | Syn | Dat (MASS) |
|---|---|---|---|---|---|---|---|---|---|---|
| 75 | 1 | FAB:383 | | 111 | 11 | ESI+:394 | | 147 | 1 | FAB+383 |
| 76 | 1 | FAB+:387 | | 112 | 1 | FAB+:373 | | 148 | 1 | FAB+: 365 |
| 77 | 1 | FAB+:339 | | 113 | 1 | FAB+:368 | | 149 | 9 | ESI+: 379 |
| 78 | 1 | FAB+: 351 | | 114 | 11 | ESI+: 355 | | 150 | 1 | ESI+: 371 |
| 79 | 1 | FAB+: 337 | | 115 | 11 | ESI+:371 | | 151 | 1 | FAB+: 367 |
| 80 | 1 | FAB+: 337 | | 116 | 1 | ESI+: 367 | | 152 | 1 | FAB+: 311 |
| 81 | 1 | FAB+: 325 | | 117 | 2 | FAB+: 325 | | 153 | 1 | FAB+: 405 |
| 82 | 1 | FAB+: 329 | | 118 | 1 | ESI+:373 | | 154 | 1 | FAB+: 367 |
| 83 | 11 | ESI+:344 | | 119 | 2 | ESI+: 365 | | 155 | 9 | ESI+: 383 |
| 84 | 11 | ESI+:344 | | 120 | 9 | FAB+: 351 | | 156 | 1 | ESI+: 355 |
| 85 | 2 | East+:367 | | 121 | 9 | ESI+:365 | | 157 | 1 | ESI+: 351 |
| 86 | 11 | ESI+:355 | | 122 | 1 | ESI+: 373 | | 158 | 1 | FAB+: 353 |
| 87 | 11 | EST+:351 | | 123 | 1 | Fay+: 403 | | 159 | 9 | FAB+: 427 |
| 88 | 1 | ESI+:350 | | 124 | 1 | ESI+: 337 | | 160 | 1 | FAB+: 331 |
| 89 | 1 | ESI+:378 | | 125 | 1 | E81+: 253 | | 161 | 1 | FAB+: 365 |
| 90 | 1 | E8I+: 414 | | 126 | 9 | FAB+: 381 | | 162 | 9 | FAB+: 409 |
| 91 | 1 | FAB+: 330 | | 127 | 9 | ESI+: 408 | | 163 | 9 | FAB+: 457 |
| 92 | 1 | FAB+: 335 | | 128 | 1 | ESI+: 349 | | 164 | 9 | FAB+: 377 |
| 93 | 1 | FAB+: 335 | | 129 | 1 | FAB+: 371 | | 165 | 1 | ESI+: 349 |
| 94 | 1 | EST+: 394 | | 130 | 9 | ESI+: 380 | | 166 | 1 | ESI+: 343 |
| 95 | 2 | FAB+: 357 | | 131 | 1 | ESI+: 403 | | 167 | 1 | FAB+:338 |
| 96 | 11 | FAB+: 363 | | 132 | 1 | ESI+: 403 | | 168 | 6 | FAB+:336 |
| 97 | 2 | ESI+:344 | | 133 | 9 | ESI+: 395 | | 169 | 1 | ESI+: 309 |
| 98 | 2 | Fay+: 349 | | 134 | 1 | FAB+: 373 | | 170 | 1 | FAB+: 322 |
| 99 | 1 | Fay+:353 | | 135 | 1 | ESI+: 349 | | 171 | 1 | ESI+: 310 |
| 100 | 1 | FAB+:355 | | 136 | 1 | Flab+: 365 | | 172 | 1 | ESI+: 350 |
| 101 | 1 | FAB+:353 | | 137 | 1 | ESI+: 351 | | 173 | 1 | ESI+: 350 |
| 102 | 1 | ESI+:330 | | 138 | 1 | FAB+:369 | | 174 | 1,6 | ESI+: 296 |

[Table 45]

| Ex | Syn | Dat (MASS) | | Ex | Syn | Dat (MASTS) |
|---|---|---|---|---|---|---|
| 175 | 1,6 | FAB+: 322 | | 211 | 9 | FAB+: 465 |
| 176 | 1 | FAB+: 363 | | 212 | 9 | ESI+: 487 |
| 177 | 1 | FAB+: 363 | | 213 | 9 | ESI+: 393 |
| 178 | 9 | ESI+:391 | | 214 | 7 | FAB+:258 |
| 179 | 9 | FAB+: 417 | | 215 | 1 | Fay+:257 |
| 180 | 9 | ESI+: 433 | | 216 | 1 | FAB+:257 |

(continued)

| Ex | Syn | Dat (MASS) | | Ex | Syn | Dat (MASTS) |
|---|---|---|---|---|---|---|
| 181 | 9 | FAB+:413 | | 217 | 7 | FAB+:334 |
| 182 | 9 | FAB+:421 | | 218 | 7 | FAB+:334 |
| 183 | 1 | FAB+:350 | | 219 | 1 | FAB+:333 |
| 184 | 1 | ESI+: 336 | | 220 | 1 | FAB+:333 |
| 185 | 1 | ESI+:336 | | 221 | 2 | ESI+:379 |
| 186 | 9 | FAB+: 437 | | 222 | 1 | ESI:341 |
| 187 | 9 | FAB+: 421 | | 223 | 7 | ESI+342 |
| 188 | 9 | FAB+:421 | | 224 | 1 | FAB+:436 |
| 189 | 1 | FAB+:352 | | 225 | 8 | FAB+: 515 |
| 190 | 1 | FAB+:426 | | 226 | 8 | FAB+: 428 |
| 191 | 1 | FAB+:375 | | 227 | 1 | ESI+:341 |
| 192 | 1 | FAB+:366 | | | | |
| 193 | 9 | FAB+: 457 | | | | |
| 194 | 9 | FAB+: 457 | | | | |
| 195 | 9 | FAB+: 457 | | | | |
| 196 | 9 | FAB+: 441 | | | | |
| 197 | 1 | ESI+:327 | | | | |
| 198 | 1 | FAB+:412 | | | | |
| 199 | 9 | ESI+:428 | | | | |
| 200 | 9 | ESI+:428 | | | | |
| 201 | 9 | ESI+:428 | | | | |
| 202 | 9,6 | FAB+: 378 | | | | |
| 203 | 9,6 | ESI+: 426 | | | | |
| 204 | 9 | FAB+: 433 | | | | |
| 205 | 9 | FAB+: 419 | | | | |
| 206 | 9 | FAB+: 435 | | | | |
| 207 | 9 | FAB+: 533 | | | | |
| 208 | 1 | ESI+:414 | | | | |
| 209 | 1 | FAB+: 287 | | | | |
| 210 | 9 | FAB+: 407 | | | | |

[Table 46]

| Ex | Dat (NMR-DMSOd$^6$) |
|---|---|
| 11 | 1.78-1.89 (2H, m), 2.56-2.71 (2H, m), 3.73 (2H, t, J = 11.3 Hz), 4.04-4.14 (2H, m), 5.23-5.35 (1H, m), 7.35 (1H, t, J = 7.5 Hz), 7.57-7.72 (2H, m), 7.93 (1H, d, J = 8.4 Hz), 8.20 (1H, d, J = 7.8 Hz), 8.54 (2H, brs), 8.74 (1H, s), 8.85 (2H, brs), 12.29 (1H, s) |

(continued)

| Ex | Dat (NMR-DMSOd6) |
|---|---|
| 74 | 4.43 (2H, t, J = 5.1 Hz), 4.94 (2H, t, J = 5.1 Hz), 6.77 (2H, d, J = 7.8 Hz), 6.84-6.88 (1H, m), 7.15-7.22 (2H, m), 7.26-7.32 (1H, m), 7.56-7.62 (2H, m), 7.79 (1H, d, J = 8.4 Hz), 7.95 (1H, dd, J = 8.4, 1.5 Hz), 8.26 (1H, d, J = 7.9 Hz), 8.34 (1H, d, J = 8.2 Hz), 8.54 (2H, brs), 8.74 (1H, d, J = 1.4 Hz), 8.90 (1H, brs), 12.24 (1H, brs) |
| 75 | 0.93 (6H, t, J = .70 Hz), 3.36-3.47 (4H, m), 3.97-4.01 (2H, m), 4.17-4.22 (2H, m), 5.20-5.30 (1H, m), 7.25-7.29 (1H, m), 7.50-7.55 (1H, m), 7.81 (1H, d, J = 8.2 Hz), 7.97 (1H, dd, J = 8.2,1.2 Hz), 8.27 (1H, d, J = 7.63 Hz), 8.36 (1H, d, J = 8.3 Hz), 8.53 (2H, brs), 8.63 (1H, s), 8.91 (2H, brs), 12.21 (1H, brs) |
| 76 | 2.99 (3H, s), 4.59 (1H, dd, J = 13.9,3.3 Hz), 4.77-4.86 (2H, m), 7.20-7.3 (5H, m), 7.48-7.56 (2H, m), 7.65 (1H, d, J = 8.4 Hz), 7.92 (1H, dd, J = 8.3,1.4 Hz), 8.24 (1H, d, J = 7.8 Hz), 8.32 (1H, d, J = 8.2 Hz), 8.55 (2H, brs), 8.57 (1H, s), 8.95 (2H, brs), 12.35 (1H, brs) |
| 77 | 1.08 (3H, d, J = 6.4 Hz), 1.83-2.05 (2H, m), 3.20 (3H, s), 3.21-3.30 (1H, m), 4.46-4.65 (2H, m), 7.26-7.31 (1H, m), 7.55-7.70 (2H, m), 7.94 (1H, dd, J= 8.1,1.5 Hz), 8.28 (1H, d, J = 8.3 Hz), 8.36 (1H, d, J = 8.3 Hz), 8.49 (2H, brs), 8.56 (1H, d, J = 1.4 Hz), 8.84 (2H, brs), 12.14 (1H, brs) |
| 82 | 7.3 (1H, t, J = 7.5 Hz), 7.44 (1H, d, J = 8.3 Hz), 7.54-7.62 (2H, m), 7.70-7.73 (4H, m), 8.02 (1H, s), 8.08 (1H, d, J = 8.3 Hz), 8.40 (1H, d, J = 7.8 Hz), 8.48-8.68 (5H. m), 11.94 (1H, brs) |
| 84 | 6.03 (2H, s), 7.34 (1H, t, J = 7.5 Hz), 7.53-7.62 (1H, m), 7.75-7.85 (2H, m), 8.02 (1H, dd, J = 8.2, 1.1 Hz), 8.11 (1H, d, J = 8.1 Hz), 8.34 (1H, d, J = 7.8 Hz), 8.41 (1H, d, J = 8.2 Hz), 8.57-8.81 (3H, m), 8.84-9.06 (4H, m), 12.40 (1H, s) |
| 86 | 1.77-1.90 (2H, m), 2.54-2.70 (2H, m), 3.66-3.80 (2H, m), 4.03-4.13 (2H, m), 5.23-5.34 (1H, m), 7.10 (1H, dd, J = 10.2, 8.1 Hz), 7.52-7.60 (1H, m), 7.72 (1H, d, J = 8.4 Hz), 7.96 (1H, dd, J = 8.2,1.3 Hz), 8.26 (1H, d, J = 8.1 Hz), 8.52 (2H, brs), 8.87 (3H, brs), 12.23 (1H, s) |
| 93 | 7.31-7.34 (1H, m), 7.41 (1H, m), 7.49-7.62 (3H, m), 7.77-7.79 (1H, m), 8.10 (1H, s), 8.18-5.20 (1 H, m), 8.38 (1H, d, J = 7.8 Hz), 8.47 (1H, d, J = 8.2 Hz), 8.61 (2H, brs), 8.80 (2H, brs), 12.15 (1H, brs) |
| 99 | 0.62 (6H, d, J = 6.8 Hz), 1.49-1.63 (1H, m), 3.08 (2H, d, J = 6.4 Hz), 3.80 (2H, t, J = 5.1 Hz), 4.70 (2H, t, J = 5.1 Hz), 7.24-7.30 (1H, m), 7.53-7.59 (1H, m), 7.72 (1H, d, J = 8.4 Hz), 7.91 (1H, dd, J = 8.4,1.5 Hz), 8.26 (1H, d, J = 7.9 Hz), 8.35 (1H, d, J = 8.2 Hz), 8.47 (2H, brs), 8.61 (1H, d, J = 1.1 Hz), 8.80 (2H, brs), 12.06 (1H, brs) |
| 100 | 3.07 (3H, s), 3.24-3.30 (2H, m), 3.42-3.50 (2H, m), 3.85 (2H, t, J = 5.4 Hz), 4.69 (2H, t, J = 5.4 Hz), 7.24-7.31 (1H, m), 7.58-7.59 (1H, m), 7.72 (1H, d, J = 8.2 Hz), 7.91 (1H, dd, J = 8.1,1.5 Hz), 8.26 (1H, d, J = 7.8 Hz), 8.35 (1H, d, J = 8.1 Hz), 8.50 (2H, brs), 8.58 (1H, d, J = 1.2 Hz), 8.80 (2H, brs), 12.06 (1H, brs) |
| 101 | 0.90 (9H, s), 3.70 (2H, t, J = 5.4 Hz), 4.53 (2H, t, J = 5.4 Hz), 7.20-7.26 (1H, m), 7.47-7.53 (1H, m), 7.67 (1H, d, J = 8.3 Hz), 7.91 (1H, dd, J = 8.3,1.1 Hz), 8.20 (2H, t, J = 8.3 Hz), 8.33 (1H, d, J = 1.0 Hz) |

[Table 47]

| Ex | Dat (NMR-DMSOd6) |
|---|---|
| 103 | 3.85 (1H, dd, J = 16.0,7.0 Hz), 3.47 (1H, dd, J = 16.0,9.1 Hz), 4.79 (2H, d, J = 5.7 Hz), 5.29-5.39 (1H, m), 6.62 (1H, d, J = 7.9 Hz), 6.79-6.85 (1H, m), 7.03-7.09 (1H, m), 7.22 (1H, d, J = 7.2 Hz), 7.29 (1H, t, J = 7.6 Hz), 7.54-7.56 (1H, m), 7.69 (1H, d, J = 8.4 Hz), 7.94 (1H, dd, J = 8.2,1.4 Hz), 8.27 (1H, d, J = 7.6 Hz), 8.37 (1H, d, J = 8.4 Hz), 8.48 (2H, brs), 8.69 (1H, d, J = 1.0 Hz), 8.82 (2H, brs), 12.11 (1H, brs) |
| 104 | 6.01 (2H, s), 7.04 (1H, s), 7.15-7.24 (2H, m), 7.29-7.35 (1H, m), 7.40-7.44 (1H, m), 7.54-7.64 (2H, m), 7.91 (1H, d, J = 8.4 Hz), 7.99 (1H, dd, J = 8.4,1.5 Hz), 8.30 (1H, d, J = 7.7 Hz), 8.38 (1H, d, J = 8.4 Hz), 8.51 (2H, brs), 8.86 (2H, brs), 8.90 (1H, s), 12.20 (1H, brs) |
| 105 | 1.86-2.02 (2H, m), 2.17-2.46 (4H, m), 2.53-2.71 (2H, m), 3.41 (4H, s), 5.01-5.20 (1H, m), 7.28 (1H, t, J = 7.5 Hz), 7.55-7.71 (1H, m), 7.76 (1H, d, J = 8.4 Hz), 7.92 (1H, d, J = 8.4 Hz), 8.30 (1H, d, J = 7.6 Hz), 8.36 (1H, d, J = 8.3 Hz), 8.49 (2H, brs), 8.69-9.00 (3H, brs), 12.08 (1H, brs) |

(continued)

| Ex | Dat (NMR-DMSOd6) |
|---|---|
| 106 | 1.73-1.86 (2H, m), 2.56-2.72 (2H, m), 2.90 (3H, s), 3.75 (2H, t, J = 11 Hz), 4.08 (2H, dd, J = 11.2, 4.1 Hz), 5.23-5.36 (1H, m), 7.30 (1H, t, J = 7.5 Hz), 7.50-7.60 (1H, m), 7.75 (1H, s), 7.88 (1H, d, J = 8.5 Hz), 8.28 (1H, d, J = 7.8 Hz), 8.53 (2H, brs), 8.74 (1H, s), 8.94 (2H, brs), 12.24 (1H, s) |
| 109 | 2.11-2.24 (2H, m), 2.57-2.73 (2H, m), 2.75-2.90 (2H, m), 3.05-3.22 (2H, m), 4.92-5.11 (1H, m), 7.28 (1H, t, J = 7.3 Hz), 7.50-7.60 (1H, m), 7.83 (1H, d, J = 8.4 Hz), 7.90 (1H, d, J = 8.1 Hz), 8.29 (1H, d, J = 7.8 Hz), 8.37 (1H, d, J = 8.3 Hz), 8.45 (2H, brs), 8.56-9.01 (3H, brs), 12.04 (1H, brs) |
| 110 | 2.16-2.33 (2H, m), 2.92-3.12 (2H, m), 3.25-3.42 (2H, m), 3.59-3.74 (2H, m), 5.39-5.50 (2H, m), 7.31 (1H, t, J = 7.3 Hz), 7.57-7.65 (1H, m), 7.69-7.77 (1H, m), 8.32 (1H, d, J = 8.4 Hz), 8.38 (1H, d, J = 8.4 Hz), 8.53 (3H, brs), 8.85 (2H, brs), 12.08 (1H, brs) |
| 112 | 3.68 (3H, s), 5.78 (2H, s), 6.72 (1H, d, J = 7.6 Hz), 6.79 (1H, dd, J = 8.2,2.0 Hz), 6.86-6.90 (1H, m), 7.16 (1H, t, J = 7.9 Hz), 7.27-7.33 (1H, m), 7.52-7.60 (1H, m), 7.72 (1H, d, J = 8.3 Hz), 7.97 (1H, dd, J = 8.3,1.5 Hz), 8.30 (1H, d, J = 7.9 Hz), 8.39 (1H, d, J = 8.3 Hz), 8.48 (2H, brs), 8.72 (1H, d, J = 1.0 Hz), 8.82 (2H, brs), 12.15 (1H, brs) |
| 113 | 5.89 (2H, s), 7.29-7.36 (1H, m), 7.41-7.52 (2H, m), 7.53-7.61 (1H, m), 7.67-7.80 (3H, m), 7.97 (1H, dd, J = 8.3,1.5 Hz), 8.32 (1H, d, J = 7.9 Hz), 8.38-8.55 (2H, brs), 8.42 (1H, d, J = 8.0 Hz), 8.68 (1H, s), 8.72 (2H, brs), 12.00 (1H, brs) |
| 118 | 3.66 (3H, s), 5.74 (2H, s), 6.79-6.86 (2H, m), 7.18-7.34 (3H, m), 7.50-7.60 (1H, m), 7.74 (1H, d, J = 8.5 Hz), 7.96 (1H, dd, J = 8.2,1.5 Hz), 8.29 (1H, d, J = 7.8 Hz), 8.38 (1H, d, J = 8.2 Hz), 8.51 (2H, brs), 8.77 (1H, d, J = 1.2 Hz), 8.87 (2H, brs) |
| 120 | 1.76-1.88 (2H, m), 2.56-2.71 (2H, m), 2.98 (3H, d, J = 5.0 Hz), 3.68-3.80 (2H, m), 4.09 (2H, dd, J = 11.2, 3.9 Hz), 5.19-5.31 (1H, m), 7.24-7.32 (1H, m), 7.51-7.60 (1H, m), 7.85 (1H, d, J = 8.5 Hz), 7.92 (1H, d, J = 8.2 Hz), 8.30 (1H, d, J = 7.7 Hz), 8.37 (1H, d, J = 8.2 Hz), 8.81 (2H, brs), 9.22 (1H, brs), 9.56-9.66 (1H, m), 12.17 (1H, s) |
| 125 | 7.23-7.32 (1H, m), 7.44-7.52 (1H, m), 7.58 (1H, d, J = 8.1 Hz), 7.63 (1H, d, J = 8.6 Hz), 8.18 (1H, dd, J = 8.6, 1.9 Hz), 8.21 (1H, d, J = 7.8 Hz), 8.49 (2H, brs), 8.87 (2H, brs), 9.24 (1H, d, J = 1.6 Hz), 11.90 (1H, s) |
| 129 | 1.96-1.99 (2H, m), 2.26-2.62 (6H, m), 5.21-5.32 (1H, m), 7.29 (1H, t, J = 7.5 Hz), 7.57 (1H, t, J = 7.8 Hz), 7.72 (1H, d, J = 7.5 Hz), 7.93 (1H, d, J = 8.4 Hz), 8.31 (1H, d, J = 7.8 Hz), 8.38 (1H, d, J = 7.2 Hz), 8.50 (2H, brs), 8.74-8.93 (3H, m), 12.10 (1H, brs) |

[Table 48]

| Ex | Dat (NMR-DMSOd6) |
|---|---|
| 133 | 1.75-1.88 (2H, m), 2.56-2.71 (2H, m), 3.35 (3H, s), 3.53-3.64 (4H, m), 3.67-3.79 (2H, m), 4.03-4.14 (2H, m), 5.21-5.34 (1H, m), 7.25-7.32 (1H, m), 7.52-7.58 (1H, m), 7.85 (1H, d, J = 8.5 Hz), 7.95 (1H, d, J = 9.4 Hz), 8.30 (1H, d, J = 7.6 Hz), 8.37 (1H, d, J = 8.2 Hz), 8.85 (1H, brs), 8.99 (1H, brs), 9.37 (1H, brs), 9.73 (1H, brs), 12.27 (1H, brs) |
| 135 | 6.01 (2H, s), 6.91-6.96 (1H, m), 7.27-7.37 (3H, m), 7.56-7.63 (1H, m), 7.85 (1H, d, J = 8.3 Hz), 7.99 (1H, dd, J = 8.3, 1.3 Hz), 8.29 (1H, d, J = 7.7 Hz), 8.37 (1H, d, J = 8.2 Hz), 8.54 (2H, brs), 8.84-8.98 (3H, m), 12.25 (1H, brs) |
| 138 | 1.73-1.85 (2H, m), 2.57-2.73 (2H, m), 2.89 (3H, d, J = 7.3 Hz), 3.71-3.82 (2H, m), 4.02-4.13 (2H, m), 5.26-5.43 (1H, m), 7.02-7.12 (1H, m), 7.51-7.60 (1H, m), 7.73 (1H, d, J = 8.3 Hz), 7.77 (1H, s), 8.53 (2H, brs), 8.77 (1H, s), 8.92 (2H, brs), 12.27 (1H, brs) |
| 141 | 2.28 (3H, s), 5.83 (2H, s), 6.07 (1H, s), 7.27-7.35 (1H, m), 7.54-7.62 (1H, m), 7.74 (1H, d, J = 8.3 Hz), 8.01 (1H, d, J = 8.2 Hz), 8.27-8.32 (1H, m), 8.38 (1H, d, J = 8.2 Hz), 8.56 (2H, brs), 8.77 (1H, s), 8.87 (2H, brs) |
| 144 | 1.79-2.06 (3H, m), 2.64-2.80 (1H, m), 3.71-3.82 (1H, m), 3.85-4.00 (2H, m), 4.29-4.40 (1H, m), 4.92-5.07 (1H, m), 7.28 (1H, d, J = 8.0 Hz), 7.50-7.61 (1H, m), 7.93 (2H, d, J = 8.3 Hz), 8.30 (1H, d, J = 7.6 Hz), 8.37 (1H, d, J = 8.2 Hz), 8.52 (2H, brs), 8.70 (1H, s), 8.91 (2H, brs), 12.29 (1H, brs) |

(continued)

| Ex | Dat (NMR-DMSOd6) |
|---|---|
| 145 | 1.75-1.85 (2H, m), 2.59-2.73 (2H, m), 2.84 (3H, d, J = 7.5 Hz), 3.70-3.82 (2H, m), 4.02-4.13 (2H, m), 5.27-5.42 (1H, m), 7.13 (1H, d, J = 7.3 Hz), 7.46-7.5 (1H, m), 7.64 (1H, d, J = 12.6 Hz), 7.76 (1H, d, J= 8.5 Hz), 8.53 (2H, brs), 8.76 (1H, s), 8.85 (2H, brs), 12.29 (1H, brs) |
| 146 | 1.78-1.92 (2H, m), 2.56-2.72 (2H, m), 3.69-3.81 (2H, m), 3.97-4.18 (2H, m), 5.26-5.43 (1H, m), 7.13 (1H, dd, J = 10.5,8.2 Hz), 7.58-7.66 (1H, m), 7.69 (1H, d, J = 10.3 Hz), 7.77 (1H, d, J = 8.5 Hz), 8.56 (2H, brs), 8.81 (1H, s), 8.86 (2H, brs), 12.38 (1H, brs) |
| 147 | 1.74-1.87 (2H, m), 2.56-2.74 (2H, m), 2.70 (3H, s), 3.68-3.79 (2H, m), 4.02-4.13 (2H, m), 5.20-5.37 (1H, m), 7.16 (1H, d, J = 7.7 Hz), 7.52-7.58 (1H, m), 7.69 (1H, d, J = 8.4 Hz), 7.95 (1H, dd, J = 8.4,1.3 Hz), 8.48 (2H, brs), 8.56 (1H, d, J = 8.3 Hz), 8.79 (1H, s), 8.80 (2H, brs), 12.13 (1H, brs) |
| 148 | 1.63-1.66 (2H, m), 1.76-1.82 (2H, m), 2.09-2.12 (2H, m), 2.56-2.65 (2H, m), 3.38 (3H, s), 3.58 (1H, m), 4.98-5.13 (1H, m), 7.25-7.29 (1H, m), 7.54-7.58 (1H, m), 7.71 (1H, d, J = 8.5 Hz), 7.94 (1H, d, J = 8.3 Hz), 8.30 (1H, d, J = 7.7 Hz), 8.36 (1H, d, J = 8.2 Hz), 8.52 (2H, brs), 8.82 (1H, brs), 8.92 (2H, brs), 9.10 (1H, brs) |
| 149 | 1.27 (3H, s), 1.29 (3H, s), 1.75-1.86 (2H, m), 2.56-2.74 (2H, m), 3.65-3.82 (2H, m), 3.90-4.03 (1H, m), 4.04-4.18 (2H, m), 5.18-5.34 (1H, m), 7.22-7.32 (1H, m), 7.51-7.59 (1H, m), 7.85 (1H, d, J = 8.4 Hz), 7.93 (1H, d, J = 8.2 Hz), 8.26-8.33 (1H, m), 8.36 (1H, d, J = 8.2 Hz), 8.85 (1H, s), 9.02 (1H, s), 9.32 (1H, s), 9.59-9.76 (1H, m), 12.12 (1H, m) |
| 150 | 1.75-1.90 (2H, m), 2.58-2.73 (2H, m), 3.67-3.83 (2H, m), 4.05-4.15 (2H, m), 5.28-5.42 (1H, m), 7.33-7.41 (1H, m), 7.60-7.68 (1H, m), 7.90-7.98 (2H, m), 8.48-8.68 (3H, m), 8.77-9.00 (3H, m), 12.38 (1H, brs) |
| 151 | 1.75-1.87 (2H, m), 2.53-2.71 (2H, m), 3.66-3.80 (2H, m), 4.01-4.14 (2H, m), 4.06 (3H, s), 5.16-5.32 (1H, m), 6.83 (1H, d, J = 7.8 Hz), 7.39-7.54 (2H, m), 7.91-7.95 (1H, m), 8.33 (1H, d, J = 8.3 Hz), 8.52 (2H, brs), 8.81 (1H, s), 8.92 (2H, brs), 12.22 (1H, brs) |

[Table 49]

| Ex | Dart (NMR-DMSOd6) |
|---|---|
| 152 | 1.66 (6H, d, J = 6.9 Hz), 5.15-5.29 (1H, m), 6.67 (1H, d, J = 7.8 Hz), 7.19 (1H, d, J = 8.2 Hz), 7.33 (1H, t, J = 8.1 Hz), 7.89 (1H, dd, J = 7.1,1.2 Hz), 8.34 (1H, d, J = 8.2 Hz), 8.48 (2H, brs), 8.55 (1H, s), 8.85 (2H, brs), 10.46 (1H, s), 12.07 (1H, brs) |
| 158 | 1.81-1.96 (4H, m), 2.15-2.27 (2H, m), 2.56-2.70 (2H, m), 4.90-5.14 (2H, m), 7.25-7.29 (1H, m), 7.51-7.55 (1H, m), 7.85-7.90 (2H, m), 8.27-8.85 (7H, m), 12.01 (1H, brs) |
| 159 | 180-1.83 (2H, m), 2.59-2.67 (2H, m), 3.70-3.75 (2H, m), 4.06-4.10 (2H, m), 4.57 (2H, d, J = 6.2 Hz), 5.23-5.29 (1H, m), 7.28 (1H, t, J = 7.5 Hz), 7.34-7.38 (1H, m), 7.41-7.47 (4H, m), 7.55 (1H, t, J = 7.3 Hz), 7.85 (1H, d, J = 8.5 Hz), 7.95 (1H, d, J = 9.0 Hz), 8.30 (1H, d, J = 7.7 Hz), 8.37 (1H, d, J = 8.2 Hz), 8.87 (1H, brs), 9.15 (1H, brs), 9.45 (1H, brs), 10.10-10.13 (1H, m), 12.3 (1H, brs) |
| 160 | 4.72-5.42 (4H, m), 5.63-5.82 (1H, m), 7.29-7.36 (1H, m), 7.55-7.61 (1H, m), 7.73-7.85 (1H, m), 7.95-8.02 (1H, m), 8.28-8.33 (1H, m), 8.37-8.43 (1H, m), 8.52 (2H, brs), 8.70 (1H, s), 8.86 (2H, brs), 12.19-12.22 (1H, m) |
| 161 | 1.92-2.01 (2H, m), 2,28-2.43 (2H, m), 2.77-2.98 (4H, m), 3.10 (1H, m), 5.23 (1H, m), 5.33-5.42 (1H, m), 7.08 (1H, d, J = 7.3 Hz), 7.42-7.46 (1H, m), 7.67 (1H, d, J = 8.4 Hz), 7.93-7.95 (1H, m), 8.34 (1H, d, J = 8.4 Hz), 8.52 (2H, brs), 8.72 (1H, brs), 8.89 (2H, brs), 12.25 (1H, brs) |
| 162 | 1.80-1.90 (4H, m), 2.59-2.67 (2H, m), 3.29 (3H, s), 3.41-3.50 (4H, m), 3,71-3.76 (2H, m), 4.07-4.10 (2H, m), 5.20-5.35 (1H, m), 7.26-7.30 (1H, m), 7.53-7.57 (1H, m), 7.85 (1H, d, J = 8.4 Hz), 7.94 (1H, d, J = 9.3 Hz), 8.30 (1H, d, J = 7.6 Hz), 8.37 (1H, d, J = 8.3 Hz), 8.86 (1H, brs), 8.94 (1H, brs), 9.30 (1H, brs), 9.75-9.83 (1H, m), 12.21 (1H, brs) |
| 163 | 1.80-1.83 (2H, m), 2.59-2.67 (2H, m), 3.70-3.76 (2H, m), 3.81-3.85 (2H, m), 4.00-4.10 (2H, m), 4.21-4.24 (2H, m), 5.20-5.33 (1H, m), 6.96-7.03 (3H, m), 7.26-7.35 (3H, m), 7.53-7.57 (1H, m), 7.84 (1H, d, J = 8.6 Hz), 7.95 (1H, d, J = 8.4 Hz), 8.30 (1H, d, J = 7.6 Hz), 8.36 (1H, d, J = 8.3 Hz), 8.90 (1H, brs), 9.16 (1H, brs), 9.50 (1H, brs), 9.92-10.02 (1H, m), 12.42 (1H, brs) |

(continued)

| Ex | Dart (NMR-DMSOd6) |
|---|---|
| 164 | 0.74-0.78 (2H, m), 0.91-0.96 (2H, m), 1.80-1.82 (2H, m), 2.58-2.67 (2H, m), 2.73-2.75 (1H, m), 3.70-3.76 (2H, m), 4.06-4.10 (2H, m), 5.22-5.2 (1H, m), 7.28 (1H, t, J = 7.5 Hz), 7.55 (1H, t, J = 7.3 Hz), 7.85 (1H, d, J = 8.5 Hz), 7.93 (1H, d, J = 8.3 Hz), 8.30 (1H, d, J = 7.7 Hz), 8.36 (1H, d, J = 8.3 Hz), 8.83 (1H, brs), 9.09 (1H, brs), 9.41 (1H, brs), 9.86 (1H, brs), 12.18 (1H, brs) |
| 166 | 1.90-2.04 (2H, m), 2.11-2.27 (2H, m), 3.64-3.76 (2H, m), 4.00-4.13 (2H, m), 5.18-5.31 (1H, m), 7.25 (1H, d, J = 5.4 Hz), 7.57 (1H, d, J = 5.4 Hz), 7.88 (1H, d, J = 8.3 Hz), 8.00 (1H, d, J = 8.4 Hz), 8.49 (2H, brs), 8.94 (2H, brs), 9.00 (1H, s), 12.22 (1H, brs) |
| 167 | 2.19-2.34 (2H, m), 2.74 (6H, s), 3.22-3.39 (2H, m), 4.53-4.70 (2H, m), 7.31 (1H, t, J = 7.8 Hz), 7.63-7.57 (1H, m), 7.90 (1H, d, J = 8.4 Hz), 7.94 (1H, dd, J = 8.3,1.5 Hz), 8.29 (1H, d, J = 7.8 Hz), 8.36 (1H, d, J = 8.3 Hz), 8.67 (2H, brs), 8.94 (1H, s), 9.04 (2H, brs), 10.35 (1H, brs), 12.43 (1H, brs) |
| 168 | 1.79-1.98 (2H, m), 1.99-2.14 (1H, m), 2.16-2.28 (1H, m), 3.02-3.18 (1H, m), 3.28-3.47 (1H, m), 4.04-4.20 (1H, m), 4.79-4.99 (2H, m), 7.33 (1H, t, J = 7.6 Hz), 7.56-7.64 (1H, m), 7.94-8.02 (2H, m), 8.31 (1H, d, J = 7.8 Hz), 8.39 (1H, d, J = 8.2 Hz), 8.69 (2H, brs), 8.90 (1H, brs), 8.93 (2H, brs), 9.20 (1H, brs), 9.35 (1H, brs), 12.32 (1H, brs) |

[Table 50]

| Ex | Dat (NMR-DMSOd6) |
|---|---|
| 175 | 2.33-2.45 (1H, m), 2.73-2.80 (1H, m), 3.30-3.37 (1H, m), 3.65-3.97 (3H, m), 5.88-5.95 (1H, m), 7.34 (1H, t, J = 7.5 Hz), 7.60 (1H, t, J = 7.3 Hz), 7.90 (1H, d, J = 8.5 Hz), 7.97 (1H, d, J = 8.3 Hz), 8.33 (1H, d, J =7.7 Hz), 8.41 (1H, d, J = 8.3 Hz), 8.60 (2H, brs), 8.71 (1H, s), 8.88 (2H, brs), 9.57 (2H, brs), 12.16 (1H, brs) |
| 177 | 2.38 (3H, s), 5.91 (2H, s), 6.84 (1H, d, J = 5.1 Hz), 7.18 (1H, d, J = 5.1 Hz), 7.25-7.33 (1H, m), 7.52-7.60 (1H, m), 7.68 (1H, d, J = 8.4 Hz), 8.02 (1H, dd, J = 8.2, 1,2 Hz), 8.28-8.34 (1H, m), 8.38 (1H, d, J = 8.3 Hz), 8.54 (2H, brs), 8.67 (1H, s), 8.86 (2H, brs), 12.21 (1H, brs) |
| 178 | 0.32-0.39 (2H, m), 0.54-0.61 (2H, m), 1.09-1.22 (1H, m), 1.75-1.88 (2H, m), 2.56-2.70 (2H, m), 3.22-3.32 (2H, m), 3.66-3.80 (2H, m), 4.02-4.14 (2H, m), 5.21-5.34 (1H, m), 7.28 (1H, t, J = 7.5 Hz), 7.51-7.59 (1H, m), 7.85 (1H, d, J = 8.4 Hz), 7.92-7.99 (1H, m), 8.30 (1H, d, J = 7.7 Hz), 8.37 (1H, d, J = 8.2 Hz), 8.89 (1H, brs), 8.97 (1H, s), 9.33 (1H, brs), 9.80 (1H, brs), 12.23 (1H, brs) |
| 179 | 1.74-1.88 (2H, m), 2.56-2.70 (2H, m), 3.64-3.80 (2H, m), 4.01-4.14 (2H, m), 4.65-4.76 (2H, m), 5.18-5.32 (1H, m), 6.46-6.52 (1H, m), 6.54-6.59 (1H, m), 7.28 (1H, t, J = 7.5 Hz), 7.50-7.59 (1H, m), 7.70-7.75 (1H, m), 7.85 (1H, d, J = 8.5 Hz), 7.93 (1H, d, J = 8.3 Hz), 8.30 (1H, d, J = 7.8 Hz), 8.36 (1H, d, J = 8.2 Hz), 8.85 (1H, s), 9.24 (1H, brs), 9.53 (1H, brs), 10.00-10.09 (1H, m), 12.30 (1H, brs) |
| 180 | 1.74-1.88 (2H, m), 2.56-2.71 (2H, m), 3.64-3.80 (2H, m), 4.01-4.15 (2H, m), 4.80-4.93 (2H, m), 5.18-5.33 (1H, m), 7.02-7.10 (1H, m), 7.22-7.32 (2H, m), 7.51-7.60 (2H, m), 7.85 (1H, d, J = 8.5 Hz), 7.93 (1H, d, J = 8.4 Hz), 8.30 (1H, d, J = 7.8 Hz), 8.36 (1H, d, J = 8.2 Hz), 8.86 (1H, brs), 9.23 (1H, brs), 9.52 (1H, brs), 10.06-10.20 (1H, m), 12.30 (1H, brs) |
| 181 | 1.76-1.92 (2H,m), 2.56-2.76 (2H, m), 3.66-3.82 (2H, m), 4.03-4.19 (2H, m), 5.13-5.37 (1H, m), 7.30 (1H, t, J = 7.5 Hz), 7.41-7.50 (3H, m), 7.52-7.61 (3H, m), 7.86 (1H, d, J = 8.3 Hz), 7.97 (1H, dd, J = 8.2,1.2 Hz), 8.31 (1H, d, J = 7.8 Hz), 8.39 (1H, d, J = 8.2 Hz), 8.81 (1H, brs), 8.96 (1H, brs), 9.43 (1H, brs), 11.41 (1H, brs), 12.31 (1H, brs) |
| 182 | 1.51-1.67 (2H, m), 1.76-1.87 (2H, m), 1.89-2.00 (2H, m), 2.55-2.72 (2H, m), 3.38-3.50 (2H, m), 3.68-3.79 (2H, m), 3.85-3.99 (3H, m), 4.01-4.14 (2H, m), 5.20-5.33 (1H, m), 7.28 (1H, t, J = 7.5 Hz), 7.52-7.58 (1H, m), 7.85 (1H, d, J = 8.4 Hz), 7.93 (1H, d, J = 8.3 Hz), 8.30 (1H, d, J = 7.7 Hz), 8.36 (1H, d, J = 8.2 Hz), 8.84 (1H, brs), 9.17 (1 H, brs), 9.37 (1H, brs), 9.78 (1H, d, J = 7.1 Hz), 12.12 (1H, brs) |
| 183 | 1.71-2.19 (4H, m), 3.02-3.17 (1H, m), 2.80 (3H, d, J = 3.3 Hz), 3.63-3.79 (1H, m), 4.25-4.43 (1H, m), 4.88-5.15 (2H, m), 7.34 (1H, t, J = 7.9 Hz), 7.59-7.67 (1H, m), 7.95 (2H, d, J = 8.1 Hz), 8.31 (1H, d, J = 7.6 Hz), 8.40 (1H, d, J = 8.1 Hz), 8.61 (2H, brs), 8.78 (1H, s), 8.95 (2H, brs), 11.11 (1H, brs), 12.42 (1H, brs) |

(continued)

| Ex | Dat (NMR-DMSOd6) |
|---|---|
| 186 | 1.76-1.88 (2H, m), 2.57-2.71 (2H, m), 3.30-3.46 (2H, m), 3.47-3.57 (2H, m), 3.60-3.88 (7H, m), 4.04-4.14 (2H, m), 5.22-5.33 (1H, m), 7.28 (1H, t, J = 7.5 Hz), 7.52-7.58 (1H, m), 7.85 (1H, d, J = 8.4 Hz), 7.96 (1H, d, J = 8.3 Hz), 8.30 (1H, d, J = 7.7 Hz), 8.37 (1H, d, J = 8.3 Hz), 8.87 (1H, s), 9.03 (1H, brs), 9.43 (1H, brs), 9.72-9.82 (1H, m), 12.33 (1H, s) |
| 189 | 1.27 (6H, t, J = 7.2 Hz), 3.20-3.46 (4H, m), 3.50-3.66 (2H, m), 4.94-5.07 (2H, m), 7.34 (1H, t, J = 7.4 Hz), 7.59-7.67 (1H, m), 7.90-7.97 (2H, m), 8.31 (1H, d, J = 7.8 Hz), 8.39 (1H, d, J = 8.2 Hz), 8.58 (2H, brs), 8.84 (1H, s), 8.90 (2H, brs), 10.58 (1H, brs),12.37 (1H, brs) |

[Table 51]

| Ex | Dat (NMR-DMSOd6) |
|---|---|
| 190 | 1.96-2.13 (2H, m), 3.10-3.43 (4H, m), 3.55-3.72 (2H, m), 4.45 (2H, m), 5.25-5.41 (1H, m), 7.29 (1H, t, J = 7.4 Hz), 7.46-7.59 (4H, m), 7.69-7.77 (2H, m), 7.92-7.97 (1H, m), 8.06-8.18 (1H, m), 8.29 (1H, d, J = 7.8 Hz), 8.36 (1H, d, J = 8.2 Hz), 8.69 (2H, brs), 8.90 (3H, brs), 11.12(1H, brs), 12.02 (1H, brs) |
| 191 | 1.95-2.20 (2H, m), 2.98-3.22 (2H, m), 3.23-3.46 (2H, m), 3.54-3.75 (2H, m), 4.59 (2H, s), 5.28-5.43 (1H, m), 7.30 (1H, t, J = 7.5 Hz), 7.52-7.58 (1H, m), 7.95 (1H, dd, J = 8.2,1.3 Hz), 8.08 (1H, d, J = 8.3 Hz), 8.31 (1H, d, J = 7.5 Hz), 8.37 (1H, d, J = 8.1 Hz), 8.66 (2H, brs), 8.88 (3H, brs), 12.20 (1H, brs) |
| 192 | 3.29-3.44 (2H, m), 3.54-3.75 (4H, m), 3.77-3.91 (2H, m), 3.96-4.12 (2H, m), 4.95-5.08 (2H, m), 7.34 (1H, t, J = 7.5 Hz), 7.62 (1H, t, J = 7.6 Hz), 7.90-7.98 (2H, m), 8.30 (1H, d, J = 7.8 Hz), 8.38 (1H, d, J = 8.2 Hz), 8.64 (2H, brs), 8.80 (1H, s), 8.94 (2H, brs), 11.68 (1H, brs), 12.40 (1H,s) |
| 193 | 1.80-1.82 (2H, m), 2.59-2.67 (2H, m), 3.70-3.77 (5H, m), 4.06-4.09 (2H, m), 4.59 (2H, d, J = 5.9 Hz), 5.22-5.34 (1H, m), 6.96-6.70 (2H, m), 7.27-7.30 (1H, m), 7.39-7.42 (2H, m), 7.53-7.57 (1H, m), 7.85 (1H, d, J = 8.5 Hz), 7.95 (1H, d, J = 9.2 Hz), 8.30 (1H, d, J = 7.7 Hz), 8.36 (1H, d, J = 8.2 Hz), 8.92 (1H, brs), 9.16 (1H, brs), 949 (1H, brs), 10.1 (1H,brs), 12.4(1H,brs) |
| 194 | 1.80-1.83 (2H, m), 2.59-2.67 (2H, m), 3.70-3.75 (2H, m), 3.79 (3H, s), 4.06-4.10 (2H, m), 4.64 (2H, d, J = 4.0 Hz), 5.19-5.32 (1H, m), 6.92-6.94 (1H, m), 7.01-7.05 (2H, m), 7.26-7.37 (2H, m), 7.53-7.57 (1H, m), 7.85 (1H, d, J = 8.4 Hz), 7.95 (1H, d, J = 8.2 Hz), 8.30 (1H, d, J = 7.7 Hz), 8.37 (1H, d, J = 8.3 Hz), 8.86 (1H, brs), 9.13 (1H, brs), 9.44 (1H, brs), 10.10 (1H, brs), 12.29 (1H, brs) |
| 195 | 1.80-1.82 (2H, m), 2.59-2.67 (2H, m), 3.70-3.75 (2H, m), 3.89 (3H, s), 4.02-4.09 (2H, m), 4.61 (2H, d, J = 5.9 Hz), 5.22-5.34 (1H, m), 6.70-7.11 (2H, m), 7.26-7.30 (1H, m), 7.35-7.43 (2H, m), 7.53-7.57 (1H, m), 7.85 (1H, d, J = 8.5 Hz), 7.96 (1H, d, J = 7.2 Hz), 8.30 (1H, d, J = 7.6 Hz), 8.36 (1H, d, J = 8.3 Hz), 8.92 (1H, brs), 9.14 (1H, brs), 9.49 (1H, brs), 10.00-10.03 (1H, m), 12.44 (1H, brs) |
| 197 | 1.92-2.02 (2H, m), 2.07-2.20 (2H, m), 3.64-3.74 (2H, m), 4.00-4.08 (2H, m), 5.04-5.16 (1H, m), 7.16 (1H, d, J = 2.3 Hz), 7.82 (2H, s), 8.02 (1H, d, J = 2.0 Hz), 8.46 (2H, brs), 8.94 (2H, brs), 8.98 (1H, s), 12.19 (1H, s) |
| 198 | 2.05-2.21 (2H, m), 3.15-3.55 (2H, m), 3.61-4.09 (4H, m), 5.47-5.69 (1H, m), 7.32 (1H, t, J = 7.5 Hz), 7.53-7.89 (6H, m), 7.98 (1H, dd, J = 8.1,1.0 Hz), 8.03-8.12 (1H, m), 8.32 (1H, d, J = 7.8 Hz), 8.39 (1H, d, J = 8.2 Hz), 8.65 (2H, brs), 8.92 (2H, brs), 9.00 (1H, s), 12.04 (1H, brs) |
| 208 | 1.93-2.05 (2H, m), 3.22-3.43 (2H, m), 3.73-4.14 (2H, m), 4.88-5.04 (2H, m), 5.30-5.49 (1H, m), 6.73 (1H, t, J = 5.0 Hz), 7.26 (1H, t, J = 7.6 Hz), 7.47-7.76 (2H, m), 7.94 (1H, dd, J = 8.2,1.2 Hz), 8.29 (1H, d, J = 7.6 Hz), 8.36 (1H, d, J = 8.0 Hz), 8.46 (2H, d, J = 4.6 Hz), 8.54 (2H, brs), 8.95 (3H, brs), 12.29 (1H, brs) |

(Test Examples)

The pharmacological activity of the compound (I) that is an active ingredient of the pharmaceutical of the present invention was confirmed in the following test. Test Example 1 Acquisition of an HEK293 cell forcibly expressing a human $5\text{-}HT_{5A}$ receptor

An ORF of a human $5\text{-}HT_{5A}$ receptor (Genbank AF498985) was cloned from a human hippocampal cDNA library, and then inserted into a pCR2.1 vector (Invitrogen), and *Escherichia coli* having the plasmid was mass cultured. Next, the

human 5-$HT_{5A}$ receptor full-length cDNA sequence was analyzed, recombined into a pCDNA3.1 vector (Invitrogen) as an expression vector, and mass cultured. A human embryonic kidney-induced cell HEK293 cell (ATCC) was seeded, and the resulting expression plasmid (1 μg) above was added thereto together with LIPOFECTAMINE 2000 (Invitrogen; 2 μl), a gene was introduced into the HEK293 cell, and then Geneticin (G418 sulfate 500 μg/ml; Kanto Chemical Co., Inc.) was used as a drug-resistant marker to screen the expressing cell. Thus prepared gene-expressing recombinant cell was cultured in a D-MEM, 10% FCS, 1% Pc./Sm., 500 μg/ml G418 culture medium for 3 days. This experimental operation was conducted in accordance with a gene operation experiment manual of a known method (Sambrook, J. et al, Molecular Cloning-A Laboratory Manual", Cold Spring Harabor Laboratory, NY, 1989), etc., an instruction appended in a reagent or the like.

Test Example 2 Test of human 5-$HT_{5A}$ receptor binding inhibition

(1) Preparation of a membrane from an HEK293 cell forcibly expressing a human 5-$HT_{5A}$ receptor

An HEK293 cell forcibly expressing a human 5-$HT_{5A}$ receptor was cultured in an F500 plate, and scraped for collection using a scraper. After centrifugation, the precipitates were collected and an incubation buffer (50 mM Tris (HCl) PH 7.4, 10 mM $MgSO_4$, 0.5 mM EDTA) was added thereto. After homogenization, it was further centrifuged, an incubation buffer was added to the precipitate, and the mixture was well suspended. These operations were repeatedly conducted, the protein concentration was then measured, and the preparation of a membrane was completed.

(2) Experiment on human 5-$HT_{5A}$ receptor binding inhibition

The compound to be tested (0.3 to 300 nM) and a 100 μM 5-CT solution in DMSO were added to a 96-well plate at 2 μl/well. The number of the wells to be measured under the same condition in one experiment was set at 2, and an average value thereof was used. It was suspended in an incubation buffer, and a HEK293 cell membrane forcibly expressing a human 5-$HT_{5A}$ receptor that had been prepared at 200 μg/ml was added thereto at 100 μl/well. The mixture was incubated at room temperature for 15 minutes, and a [$^3$H]5-CT solution (2 nM [$^3$H]5-CT, an incubation buffer) was then added thereto at 100 μl/well.
Separate from this, 100 μl of the solution was dispersed to a liquid scintillation vial, 2 ml of Aquasol II (registered trademark) was added thereto, followed by stirring, and the radioactivity was then measured with a liquid scintillation counter. The solution was incubated at 37˚C for 60 minutes. The reaction liquid was sucked to a 96-well GF/C filter plate that had been preliminarily treated with 0.2% polyethyleneimine, and washed six times with an ice-cooled 50 mM Tris (pH 7.5) buffer. The GF/C filter plate was dried.
MicroscintTMPS (registered trademark) was added thereto at 40 μl/well. The radioactivity remaining on the GF/C filter plate was measured in a top counter.
For the inhibitory activity for the binding of the [$^3$H]5-CT by the compound to be tested in each experiment, $IC_{50}$ value was calculated by taking the radioactivity when only DMSO was added as 0% inhibition, and the radioactivity when 1 μM 5-CT was added as 100% inhibition. Apart from this, a Ki value was calculated from the Kd value of the [$^3$H]5-CT that had been determined by Scatchard analysis.

$$Ki = IC_{50} \text{ (1 + Concentration of the ligands added/Kd (4.95 nM))}$$

As a result of this test, it was proved that the compound (I) that is an active ingredient of the pharmaceutical of the present invention has strong human 5-$HT_{5A}$ receptor binding inhibition.
Hereinbelow, the Example numbers and the Ki values (the numbers in parenthesis: nM) of the compounds exhibiting strong activity are exemplified.
Examples 1 (0.69), 2 (2.8), 25 (0.51), 27 (0.66), 28 (4.5), 37 (8.3), 86 (0.56), 102 (5.3), 106 (0.27), 120 (2.2), 159 (1.6)
In addition, the Example numbers of the compound exhibiting Ki values of 50 nM or less are exemplified below.
Examples 6, 11, 22, 24, 26, 59, 65, 114, 115, 116, 126, 129, 135, 138, 140, 141, 143, 144, 145, 146, 147, 148, 149, 150, 152, 160, 161, 162, 164, 175, 177, 178, 179, 180, 181, 182, 186, 187, 188, 191, 193, 194, 195, 196, 198, 204, 205, 206, 210, 212, 218, 220, 227
From above, it was confirmed that the compound (I) has a 5-$HT_{5A}$ receptor affinity.

Test Example 3 Evaluation of various drugs relative to a drug for increasing the kinetic momentum of mice (methane phetamine, MK-801) (Method for measuring a kinetic momentum by discharge of an infrared ray)

The effect of improving the positive symptoms and the negative symptoms of schizophrenia by the compound (I) was evaluated by measuring the kinetic momentum that had been suppressed with administration of the compound in a model causing the symptoms by methane phetamine (which is hereinafter simply referred to as MAP) and M-801.

(1) Animals

Species: Male ICR mouse/number of animals (number of animals per group): 8 to 12 animals per group
Week-old in use: 4-6 week-old
Supplier or producer: Japan SLC, Inc.

(2) Procedure for operation

The animal was left in a laboratory for 1 hour or longer to be acclimated to the environment, and the animal was taken from the feeding cage, orally administered with a compound to be tested, and then returned to the feeding cage. After 30 minutes, it was put into a cage for measurement, and the kinetic momentum of just the compound to be tested was measured. Further, after 30 minutes, the animal was taken out, and intraperitoneally administered with a drug for increasing kinetic momentum (MAP; 1 mg/kg or MK-801; 0.3 mg/kg, all dissolved in physiological saline), and its kinetic momentum was measured for a certain time (60 minutes) using a device for measuring the kinetic momentum by means of an infrared ray sensor (CompACT AMS, Muromachi Kikai Co., Ltd.). Also, the test was carried out under non-fasting.

(3) Analysis

The 60 minutes of measurements was classified into three groups: a first half 30 minutes, a second half 3 0 minutes, and a total 60 minutes. For a normal mouse (a mouse administered with physiological saline) and a mouse administered with the drug for increasing kinetic momentum, a Student's T test was used for evaluation in each interval. For the group administered with the compound to be tested, a solvent (vehicle) group and a Dunnett's T test were carried out and evaluated. For the evaluation, in case where there was a significant ($P<0.05$) difference for the total 60 minutes, it was considered to be effective.

As a result of this test, it was proven that the compound (I) inhibits the overactivity induced by MAP or MK-801. For example, the compounds of Examples 6, 25, 86, 106, and 135, and the compound of Example 65 significantly inhibited the MAP-induced overactivity at doses of 0.01 mg/kg and doses of 0.003 mg/kg, respectively. On the other hand, olanzapine as a known compound significantly inhibited the MAP-induced overactivity at doses of 0.3 mg/kg.
Furthermore, the compounds of Examples 6, 25, 37, 65, 86, 135, 138, 146, and 178, and the compounds of Examples 106 and 194 significantly inhibited the MK-801-induced overactivity at doses of 0.01 mg/kg and doses of 0.03 mg/kg, respectively. The compounds of Examples 22, 24, 129, 150, and 161 significantly inhibited the MK-801-induced overactivity at doses of 0.1 mg/kg. On the other hand, clozapine as a known compound significantly inhibited the MK-801-induced overactivity at doses of 0.3 mg/kg.
From above, it was confirmed that the compound (I) has the effect of improving the positive symptoms and the negative symptoms of schizophrenia. Furthermore, since the compound (I) inhibited the MAP-induced overactivity, it is also supposed that the compound (I) is effective for bipolar disorders and attention deficit hyperactivity disorders.

Test Example 4 An improvement effect for scopolamine-induced or MK-801-induced spontaneous alternation behavior in mice

An improvement effect of the compound (I) for cognitive impairment was evaluated by the above-described well-known test method as a model of a short-term learning disorder.

(1) Animals

Species: Male ddY mice/number of animals (number of animals per group): 6 to 10 animals per group

Week-old in use: 5 week-old
Supplier or producer: Japan SLC, Inc.

(2) Measurement method

A mouse was introduced into the laboratory 1 hour before starting the test. The mouse was placed at one end of an arm in a Y-maze having equal lengths of arms in three directions, and was able freely explored for 8 minutes with the number of the entries into the arms were counted. Furthermore, a spontaneous alternation behavior was defined as consecutive entries into each of the three arms, and an alternation rate was defined as the percentage of the number of time of this behavior relative to the total number of the entries, and calculated by the following equation.

$$\text{Alternation rate (\%)} = (\text{number of spontaneous alternation behaviors/total number of entries} - 2) \times 100.$$

The compound to be tested was orally administered 50 minutes before the initiation of the test, and 30 minutes later, 0.5 mg/kg of scopolamine or 0.15 mg/kg of MK-801 (in the normal group, physiological saline) was intraperitoneally administered. Furthermore, for the normal group (the group administered with physiological saline) and the control group (the group administered with 0.5 mg/kg scopolamine or 0.15 mg/kg MK-801), a solvent (vehicle) was orally administered when the compound to be tested was administered. For the normal group, physiological saline was intraperitoneally administered when scopolamine was administered.

(3) Data analysis

The alternation rate (%) is expressed as an average value in each group (mean±SE). In regard to the alternation rate (%), in the case where a significant difference between the normal group and the control group (Student's T test) was found, it was considered that there was an establishment of learning disorder by the administration of scopolamine or MK-801. By carrying out the Dunnett evaluation for the group administered with the compound to be tested relative to the control group, the presence or absence of the learning disorder action of the compound to be tested was determined.
In each evaluation, it was considered that there was a tendency at $p<0.10$, and there was a significant difference at $p<0.05$.
As a result of this test, it was proven that the compound (I) inhibits the scopolamine-induced spontaneous alternation behavior disorder. For example, the compounds of Examples 86 and 106, the compounds of Examples 6,25, 65, and 135, and the compounds of Examples 26 and 59 significantly inhibited the scopolamine-induced spontaneous alternation behavior disorder at doses of 0.0001 mg/kg, doses of 0.003 mg/kg, and doses of 0.03 mg/kg, respectively.
On the other hand, donepezil as a known compound significantly inhibited the scopolamine-induced spontaneous alternation behavior disorder at doses of 0.25 mg/kg.
The compound of Example 25 significantly improved the MK-801-induced spontaneous alternation behavior disorder at doses of 0.003 mg/kg.
From above, it was confirmed that the compound (I) has an effect on cognitive impairment.

Test Example 5 An improvement effect for a disorder of PCP-induced prepulse inhibition (PPI) in a rat

A startle amplitude occurs in humans to which an sound stimulus has been given, but in healthy human, this startle amplitude is inhibited by the giving of a weak sound stimulus that precedes the sound stimulus. For a patient with schizophrenia, the inhibitory function similarly declined. It is known that when a rat is administered with PCP (phencyclidine), there is a symptom similar to the negative symptom of schizophrenia in humans. Using this model, the improvement effect of the compound (I) for the information processing disorder included in cognitive impairment of schizophrenia was evaluated.

(1) Animals to be used

Species: Male Wistar rat/number of the animals (number of animals per group): 12 animals per group
Week-old in use: 7 to 10 week-old
Supplier or producer: Charles River Laboratories Japan Inc.

(2) Instruments to be used

Startle amplitude measuring device for small animals: an SR-LAB ABS system (manufactured by San Diego Instruments)

Software: SR-LAB Startle Reflex System (manufactured by San Diego Instruments)

An animal holder, to which a Plexiglas-made cylinder for animal storage having a diameter of 8.2 cm was attached, was positioned in the upper part of a Plexiglas-made frame in a measurement box. In the measurement box, a sound-insulating treatment and ventilation (FAN) were carried out. Sound was administered by a speaker attached to the 24 cm upper part of the cylinder. The movement of the animals in the cylinder was detected by a transducer attached in the lower part of the frame and recorded by a microcomputer via an interface.

(3) Measurement method

The experiment was initiated after the animals were put into the chamber for measurement and had spent 10 minutes adapting to the measurement environment. Basically, at 35 minutes after the compound to be tested was orally administered, 1 mg/kg of PCP was subcutaneously administered (1 ml/kg). Five minutes later, the rats were put into a chamber for measurement, allowed to adapt for 10 minutes, and the measurement was then initiated. A white noise of 65 dB (for all frequencies, a disordered noise having a constant energy per unit band) used as a background noise was always played through the break periods and the sessions. The three types of trials as shown below were carried out in a random order 10 times for each type with 30 times in total. Each trial was carried out at a pseudo-random interval of 20 to 60 seconds with an average of 40 seconds. A pulse was defined as a white noise of 120 dB, 20 msec, and a prepulse was defined as a white noise of 70, 80 dB, 20 msec.

1) Only a pulse (120 dB, 20 msec) is given (simply referred to as a P-alone trial).
2) A pulse is given at 100 msec after the initiation of prepulse of 70 dB, 20 msec (simply referred to as a PP70 & P trial).
3) A pulse is given at 100 msec after the initiation of prepulse of 80 dB, 20 msec (simply referred to as a PP80 & P trial).

The startle amplitude of the animal was measured for 100 msec from the initiation of the pulse, and the maximum value was taken as a "maximum startle amplitude (Vmax))". The "maximum startle amplitude" for the ten times for each of the three types of trials were averaged, and taken as a "startle amplitude (simply referred to as SA)" under the stimulation condition.
The % prepulse inhibition (% PPI) was calculated in the following equation in the PP80 & P trial of 3) above.

% Prepulse inhibition (% PPI) = (Startle amplitude at P-alone trial (SA) - Startle amplitude (SA) at a PP80 & P trial)/Startle amplitude at P alone trial × 100

The experiment was regulated by means of a computer, and data were taken.

(4) Data organization:

The measured value was expressed as an average value (mean$\pm$SE). First, the startle amplitudes (SA) were statistically analyzed. In case where SA in the PP80 & P trial was significantly inhibited, as compared with SA in the P alone trial of the normal group (the group administered with physiological saline) (evaluated by means of a Paired t-test), it was taken that the experiment had passed, and the subsequent analysis was carried out. For the measured data of % PPI, the normal group and the control group (the group administered with PCP) were compared by a Student t-test, and for the control group and the group administered with the compound to be tested were compared using a Dunnett evaluation. In each of the evaluations, it was considered that there was a significant difference if $p<0.05$. The effect of the compound to be tested was assessed with % PPI.
As a result of this test, it was proven that the compound (I) improves the disorder of PCP prepulse inhibition (PPI). For example, the compound of Example 25 and the compound of Examples 65 significantly improved the disorder of PCP prepulse inhibition (PPI) at doses of 0.03 and 0.1 mg/kg and 0.1 and 0.3 mg/kg, respectively. On the other hand, quetiapine as a known compound significantly improved the PCP-induced PPI at doses of 10 mg/kg.
From above, it was confirmed that the compound (I) also has an effect on information processing disorders included in the cognitive impairment of schizophrenia.

Test Example 6 Evaluation of a drug for water maze learning disorders in old rats

The improvement effect of the compound (I) for dementia was evaluated by a known water maze learning disorder model used as a pathophysiology model. Specifically, it was evaluated in accordance with the method as described in "J Pharmacol Exp Ther, 1996; 279: 1157-73, Yamazaki M. et al.".
As a result of this test, it was proven that the compound (I) improves water maze learning disorders in old rats. For example, the compound of Example 25 significantly improved water maze learning disorders in old rats at doses of 0.01 and 0.03 mg/kg.
From above, it was confirmed that the compound (I) has an effect on dementia.
In these tests, the compound of the present invention was not associated with side effects such as a sedation action and the like, that have been reported for the conventional compounds and exhibited improving actions.
From the above-described test results, it can be confirmed that the pharmaceutical composition of the present invention is effective for treating or preventing a 5-$HT_{5A}$ receptor-related disease, particularly for treating or preventing dementia, schizophrenia, bipolar disorder, or attention deficit hyperactivity disorder, neurosis (anxiety disorder, panic disorder, obsessive-compulsive disorder or the like), autism, mood disorder (depressive disorder), neurodegenerative disease, brain infarction, and inter alia, for treating or preventing a memory-related functional disorder such as dementia and a cognitive impairment in schizophrenia.
The pharmaceutical composition of the present invention is excellent in terms of safety when compared with the conventional compound, and is expected to be a novel and effective agent for treating the above-described diseases.
A preparation containing one or two or more kinds of the compound (I) or a salt thereof as an active ingredient can be prepared in accordance with methods that are usually used in the art using a pharmaceutically acceptable carrier, excipient and the like.
The administration can be carried out in any form of oral administration via tablets, pills, capsules, granules, powders, liquid preparations or the like, or parenteral administration via injections such as intraarticular, intravenous, or intramuscular injections, suppositories, ophthalmic solutions, ophthalmic ointments, percutaneous liquid preparations, ointments, percutaneous patches, transmucosal liquid preparations, transmucosal patches, inhalations and the like.
Regarding the solid composition for oral administration according to the present invention, tablets, powders, granules or the like are used. In such a solid composition, one or two or more kinds of active ingredients are mixed with at least one inert excipient such as lactose, mannitol, glucose, hydroxypropylcellulose, microcrystalline cellulose, starch, polyvinyl pyrrolidone, and/or magnesium aluminometasilicate. According to a conventional method, the composition may contain inert additives such as a lubricant such as magnesium stearate, a disintegrator such as carboxymethylstarch sodium, a stabilizing agent and a dissolution aid. As occasion demands, the tablets or the pills may be coated with sugar, or a film of a gastric or enteric material.
The liquid composition for oral administration includes pharmaceutically acceptable emulsions, soluble liquid preparations, suspensions, syrups, elixirs or the like, and contains a generally used inert diluent such as purified water or ethanol. In addition to the inert diluent, this liquid composition may contain an auxiliary agent such as a solubilizing agent, a moistening agent, and a suspending agent, a sweetener, a flavor, an aroma, and an antiseptic.
Injections for parenteral administration include sterile aqueous or non-aqueous soluble liquid preparations, suspensions and emulsions. The aqueous solvent includes, for example, distilled water for injection and physiological saline. Examples of the non-aqueous solvent include propylene glycol, polyethylene glycol, plant oils such as olive oil, alcohols such as ethanol, Polysorbate 80 (Japanese Pharmacopeia) and the like. Such a composition may further contain a tonicity agent, an antiseptic, a moistening agent, an emulsifying agent, a dispersing agent, a stabilizing agent, or a dissolution aid. These are sterilized, for example, by filtration through a bacteria retaining filter, blending of a bactericide, or irradiation. In addition, these can also be used by producing a sterile solid composition, and dissolving or suspending it in sterile water or a sterile solvent for injection prior to its use.
The drug for external use includes ointments, plasters, creams, jellies, cataplasms, sprays, lotions, opthalmic sulutions, opthalmic ointments and the like. The drug contains generally used ointment bases, lotion bases, aqueous or non-aqueous liquid preparations, suspensions, emulsions and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate and the like.
Regarding the transmucosal agents such as an inhalations and a transnasal agent, those in the form of a solid, liquid, or semi-solid state are used, and may be prepared in accordance with a conventionally known method. For example, a known excipient, and also a pH adjusting agent, an antiseptic, a surfactant, a lubricant, a stabilizing agent, a viscosity increasing agent or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device. The dry powder inhaler or the like may be for single or multiple administration use, and a dry powder or a powder-containing capsule may be used. Alternatively,

this may be in a form such as a pressurized aerosol spray which uses an appropriate propellant, for example, a suitable gas such as chlorofluoroalkane, hydrofluoroalkane, carbon dioxide and the like, or other forms.

In oral administration, the daily dose is generally from about 0.0001 to 100 mg/kg, preferably from 0.0001 to 10 mg/kg, and even more preferably from 0.0001 to 1 mg/kg, in regard to body weight, administered in one portion or divided in 2 to 4 portions. In the case of intravenous administration, the daily dose is suitably administered from about 0.00001 to 1 mg/kg in regard to body weight, once a day or divided up and taken two or more times a day. In addition, a drug for external use or a transmucosal agent is administered at doses from about 0.0001 to 10 mg/kg per body weight, once a day or divided up and taken two or more times a day. The dose is appropriately decided in response to individual cases by taking into consideration the symptoms, the age, and the gender of the subject and the like. The content of the active ingredient in the preparation is from 0.0001 to 50%, and more preferably from 0.001 to 50%.

The compound that is an active ingredient of the pharmaceutical of the present invention can be used in combination with drugs used for treating or preventing the diseases for which the compound is considered to be effective. The combined preparation may be administered simultaneously, or separately one after the other or at desired time intervals. The preparations to be co-administered may be a blend or may be prepared individually.

INDUSTRIAL AVAILABILITY

The compound that is an active ingredient of the pharmaceutical of the present invention has advantages in that it has a potent 5-$HT_{5A}$ receptor modulating action, and has an excellent pharmacological action based thereon. The pharmaceutical composition of the present invention is useful for treating or preventing a 5-$HT_{5A}$ receptor-related disease, and particularly, for treating or preventing dementia, schizophrenia, bipolar disorder, or attention deficit hyperactivity disorder. The compound that is an active ingredient of the pharmaceutical of the present invention is useful for improvement of memory-related functional disorders such as dementia and a cognitive impairment in schizophrenia.

## Claims

**1.** A 5-$HT_{5A}$ receptor modulator comprising a compound represented by the following general formula (I) or a salt thereof as an active ingredient.

[Chem. 18]

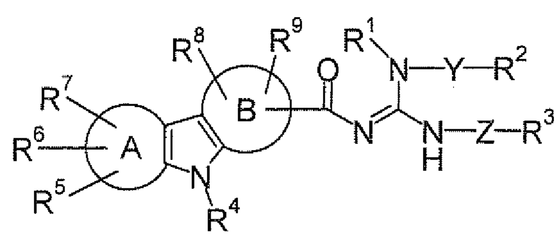

(I)

(the symbols in the formula represent the following meanings:

$R^1$: H, lower alkyl, halogeno-lower alkyl, $C_{2-6}$ alkylene-$OR^a$, or $C_{2-6}$ alkylene-NR $^aR^b$,
$R^2$ and $R^3$: the same as or different from each other, each representing H, -$OR^a$, -$NR^aR^b$, phenyl, cycloalkyl, or a monocyclic heterocyclic group, or $R^2$ together with $R^1$ and with a nitrogen atom may form a monocyclic nitrogen-containing heterocyclic group, wherein phenyl, cycloalkyl, the monocyclic heterocyclic group, and the monocyclic nitrogen-containing heterocyclic group may be substituted with lower alkyl or -$OR^a$,
$R^a$ and $R^b$: the same as or different from each other, each representing H or lower alkyl,
$R^4$: lower alkyl which may be substituted with one or two groups selected from the groups represented by Group G, H, -C(O)$R^a$, -$S(O)_p$-lower alkyl, -C(O)$NR^aR^b$, or -L-X,
Group G: -$NR^aR^b$, -$OR^a$, or -O-lower alkylene-$OR^a$,
L: a bond, -C(O)-, -$S(O)_p$-, lower alkylene, or lower alkylene-O-lower alkylene, wherein lower alkylene may be substituted with -$OR^a$,
X: a heterocyclic group, aryl, cycloalkyl, or cycloalkenyl, wherein the ring group represented by X may be substituted with one or two groups selected from lower alkyl, halogen, -$OR^a$, -C(O)$R^a$, -$CO_2R^a$, -$S(O)_p$-lower

alkyl, -CN, lower alkylene-CN, benzhydryl, phenyl, monocyclic heteroaryl, and oxo,
p: 0, 1, or 2,

[Chem. 19]

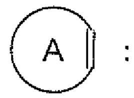

a benzene, thiophene, furan, cyclohexene, or tetrahydropyridine ring,
$R^5$, $R^6$, and $R^7$: the same as or different from each other, each representing H, lower alkyl, lower alkenyl, halogen, -O-halogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^a$, $-C(O)NR^aR^b$, lower alkylene-$OR^a$, or lower alkylene-$NR^aR^b$,

[Chem. 20]

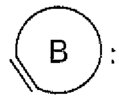

a benzene, cyclohexene or tetrahydropyridine ring,
$R^8$ and $R^9$: the same as or different from each other, each representing H, lower alkyl, lower alkenyl, halogen, -O-halogeno-lower alkyl, -CN, $-NO_2$, $-OR^a$, $-OC(O)R^a$, $-NR^aR^b$, $-NR^a-C(O)R^b$, $-NR^a-S(O)_2$-lower alkyl, -SH, $-S(O)_p$-lower alkyl, $-S(O)_2-NR^aR^b$, $-C(O)R^a$, $-CO_2R^a$, $-C(O)NR^aR^b$, lower alkylene-$OR^a$, or lower alkylene-$NR^aR^b$, and
Y and Z: the same as or different from each other, each representing a bond, lower alkylene, or lower alkylene-O-).

**2.** The 5-$HT_{5A}$ receptor modulator as described in claim 1, wherein A is a benzene ring and B is a benzene ring.

**3.** The 5-$HT_{5A}$ receptor modulator as described in claim 2, wherein $R^4$ is -L-X, in which L is a bond or $C_{1-4}$ alkylene and X is a ring group selected from a monocyclic heterocyclic group, phenyl, and cycloalkyl.

**4.** The 5-$HT_{5A}$ receptor modulator as described in claim 2, wherein $R^4$ is lower alkyl or $-C(O)R^a$.

**5.** The 5-$HT_{5A}$ receptor modulator as described in claim 1, wherein the compound represented by the general formula (I) is selected from the group consisting of 9-cyclobutyl-N-(diaminomethylene)-9H-carbazole-2-carbaxamide, N-(diaminomethylene)-9-piperidin-4-yl-9H-carbazole-2-carboxamide, 9-cyclohexyl-N-(diaminomethylene)-9H-carbazole-2-carboxamide, N-(diaminomethylene)-9-(tetrahydro-2H-pyran-4-yl)-9H-carbazole-2-carboxamide,9-acetyl-N-(diaminomethylene)-9H-carbazole-2-carboxamide, 9-benzyl-N-(diaminomethylene)-9H-carbazole-2-carboxamide, 5-chloro-N-(diaminomethylene)-9-isopropyl-9H-carbazole-2-carboxamide, and N-(diaminomethylene)-5-(hydroxymethyl)-9-isopropyl-9H-carbazole-2-carboxamide.

**6.** A compound represented by the following general formula (I') or a salt thereof.

[Chem. 21]

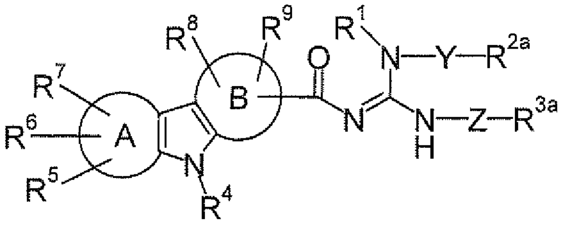 (I')

(the symbols in the formula represent the following meanings:

$R^1$: H, lower alkyl, halogeno-lower alkyl, $C_{2-6}$ alkylene-$OR^a$ or $C_{2-6}$ alkylene-$NR^aR^b$,
$R^{2a}$: H, -$OR^a$, -$NR^aR^b$, phenyl, cycloalkyl, or a monocyclic heterocyclic group, or $R^{2a}$ together with $R^1$ and with a nitrogen atom may form a monocyclic nitrogen-containing heterocyclic group,
$R^{3a}$: -$OR^a$, -$NR^aR^b$, phenyl, cycloalkyl, or a monocyclic heterocyclic group,
wherein phenyl, cycloalkyl, the monocyclic heterocyclic group, and the monocyclic nitrogen-containing heterocyclic group in aforementioned $R^{2a}$ and $R^{3a}$ may be substituted with lower alkyl or -$OR^a$,
$R^a$ and $R^b$: the same as or different from each other, each representing H or lower alkyl,
$R^4$: lower alkyl which may be substituted with one or two groups selected from the groups represented by Group G, H, -C(O)$R^a$, -$S(O)_p$-lower alkyl, -C(O)$NR^aR^b$, or -L-X,
Group G: -$NR^aR^b$, -$OR^a$, or -O-lower alkylene-$OR^a$,
L: a bond, -C(O)-, -$S(O)_p$-, lower alkylene, or lower alkylene-O-lower alkylene, wherein lower alkylene may be substituted with -$OR^a$,
X: a heterocyclic group, aryl, cycloalkyl, or cycloalkenyl, wherein each of the ring group represented by X may be substituted with one or two groups selected from lower alkyl, halogen, -$OR^a$, -C(O)$R^a$, -$CO_2R^a$, -$S(O)_p$-lower alkyl, -CN, lower alkylene-CN, benzhydryl, phenyl, monocyclic heteroaryl, and oxo,
p: 0, 1, or 2,

[Chem. 22]

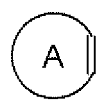 :

a benzene, thiophene, furan, cyclohexene or tetrahydropyridine ring,
$R^5$, $R^6$, and $R^7$: the same as or different from each other, each representing H, lower alkyl, lower alkenyl, halogen, -O-halogeno-lower alkyl, -CN, -$NO_2$, -$OR^a$, -OC(O)$R^a$, -$NR^aR^b$, -$NR^a$-C(O)$R^b$, -$NR^a$-$S(O)_2$-lower alkyl, -SH, -$S(O)_p$-lower alkyl, -$S(O)_2$-$NR^aR^b$, -C(O)$R^a$, -$CO_2R^a$, -C(O)$NR^aR^b$, lower alkylene-$OR^a$ or lower alkylene-$NR^aR^b$,

[Chem. 23]

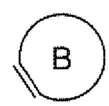 :

a benzene, cyclohexene or tetrahydropyridine ring,
$R^8$ and $R^9$: the same as or different from each other, each representing H, lower alkyl, lower alkenyl, halogen, -O-halogeno-lower alkyl, -CN, -$NO_2$ -$OR^a$, -OC(O)$R^a$, -$NR^aR^b$, -$NR^a$-C(O)$R^b$, -$NR^a$-$S(O)_2$-lower alkyl, -SH, -S(O)$_p$-lower alkyl, -$S(O)_2$-$NR^aR^b$, -C(O)$R^a$, -$CO_2R^a$, -C(O)$NR^aR^b$, lower alkylene-$OR^a$, or lower alkylene-$NR^aR^b$,

and
Y and Z: the same as or different from each other, each representing a bond, lower alkylene, or lower alkylene-O-.)

**7.** The compound or a salt thereof as described in claim 6, wherein A is a benzene ring and B is a benzene ring.

**8.** The compound or a salt thereof as described in claim 7, wherein $R^4$ is -L-X, in which L is a bond or $C_{1-4}$ alkylene, and X is a ring group selected from a monocyclic heterocyclic group, phenyl, cycloalkyl, and cycloalkenyl, and may be substituted with halogen, lower alkyl, or $-OR^a$.

**9.** The compound or a salt thereof as described in claim 7, wherein $R^4$ is lower alkyl.

**10.** The compound or a salt thereof as described in claim 6, which is selected from the group consisting of N-[amino(methylamino)methylene]-9-(tetrahydro-2H-pyran-4-yl)-9H-carbazole-2-carboxamide, N-{amino[(3-methoxypropyl)amino]methylene}-9-(tetrahydro-2H-pyran-4-yl)-9H-carbazole-2-carboxamide, N-{amino[(cyclopropylmethyl)amino]methylene}-9-(tetrahydro-2H-pyran-4-yl)-9H-carbazole-2-carboxamide, N-{amino[(4-methoxybenzyl)amino]methylene}-9-(tetrahydro-2H-pyran-4-yl)-9H-carbazole-2-carboxamide, N-{amino[(3-methoxybenzyl)amino]methylene}-9-(tetrahydro-2H-pyran-4-yl)-9H-carbazole-2-carboxamide, and N-{amino[(2,6-dimethoxybenzyl)amino]methylene}-9-(tetrahydro-2H-pyran-4-yl)-9H-carbazole-2-carboxamide.

**11.** A compound represented by the following general formula (I") or a salt thereof.

[Chem. 24]

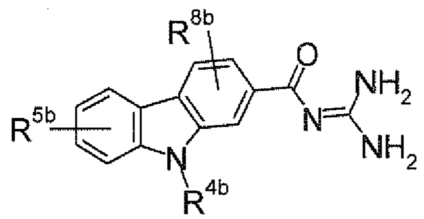

(I'')

(the symbols in the formula represent the following meanings:

$R^{4b}$: isopropyl, tetrahydropyranyl, piperidyl, cyclohexyl, cyclohexenyl, phenyl, thienyl, pyridyl, thienylmethyl, or isoxazolylmethyl, wherein the piperidyl group may be substituted with cyanomethyl or phenyl, and the other groups may be substituted with one or two groups selected from the group consisting of F, -O-methyl, and methyl,
$R^{5b}$: H, lower alkyl, -OH, -S-lower alkyl, halogen, lower alkylene-OH, or lower alkylene-O-lower alkyl, and
$R^{8b}$: H, lower alkyl, halogen, or lower alkylene-OH,

provided that when $R^{4b}$ is isopropyl, $R^{5b}$ is -OH, and when $R^{4b}$ is unsubstituted tetrahydropyranyl, unsubstituted piperidyl, or unsubstituted cyclohexyl, either of $R^{5b}$ and $R^{8b}$ represents a group other than H).

**12.** The compound or a salt thereof as described in claim 11, which is selected from the group consisting of N-(diaminomethylene)-5-fluoro-9-(tetrahydro-2H-pyran-4-yl)-9H-carbazole-2-carboxamide, N-(diaminomethylene)-4-methyl-9-(tetrahydro-2H-pyran-4-yl)-9H-carbazole-2-carboxamide, N-(diaminomethylene)-9-(4,4-difluorocyclohexyl)-9H-carbazole-2-carboxamide, N-(diaminomethylene)-9-(2-thienylmethyl)-9H-carbazole-2-carboxamide, N-(diaminomethylene)-5-fluoro-4-methyl-9-(tetrahydro-2H-pyran-4-yl)-9H-carbazole-2-carboxamide, N-(diaminomethylene)-4,5-difluoro-9-(tetrahydro-2H-pyran-4-yl)-9H-carbazole-2-carboxamide, and N-(diaminomethylene)-9-(4-fluorocyclohex-3-en-1-yl)-5-methyl-9H-carbazole-2-carboxamide.

**13.** A pharmaceutical composition for preventing or treating dementia, schizophrenia, bipolar disorder, or attention deficit hyperactivity disorder, which comprises the compound represented by the formula (I) or a salt thereof of claim 1 as an active ingredient.

**14.** Use of the compound represented by the formula (I) or a salt thereof of claim 1 for the manuafcture of a pharmaceutical composition for preventing or treating dementia, schizophrenia, bipolar disorder, or attention deficit hyperactivity

disorder.

**15.** A method for preventing or treating dementia, schizophrenia, bipolar disorder, or attention deficit hyperactivity disorder, which comprises administering to a mammal an effective amount of the compound represented by the formula (I) or a salt thereof of claim 1.

## INTERNATIONAL SEARCH REPORT

International application No.
PCT/JP2008/051962

A. CLASSIFICATION OF SUBJECT MATTER
*C07D209/88*(2006.01)i, *A61K31/403*(2006.01)i, *A61K31/407*(2006.01)i, *A61K31/4155*(2006.01)i, *A61K31/437*(2006.01)i, *A61K31/4439*(2006.01)i, *A61K31/454*(2006.01)i, *A61K31/496*(2006.01)i, *A61K31/506*(2006.01)i,

According to International Patent Classification (IPC) or to both national classification and IPC

B. FIELDS SEARCHED

Minimum documentation searched (classification system followed by classification symbols)
C07D209/88, A61K31/403, A61K31/407, A61K31/4155, A61K31/437, A61K31/4439, A61K31/454, A61K31/496, A61K31/506, A61K31/5377, A61P25/00, A61P25/14, A61P25/18, A61P25/20, A61P25/22, A61P25/24, A61P25/28, A61P43/00,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,A | WO 2007/018168 A1 (Astellas Pharma Inc.), 15 February, 2007 (15.02.07), (Family: none) | 1-14 |
| A | JP 2002-502419 A (Eli Lilly and Co.), 22 January, 2002 (22.01.02), & EP 0882726 A1 & WO 1998/055115 A1 & US 6221884 B1 | 1-14 |
| A | WO 2005/079845 A1 (Astellas Pharma Inc.), 01 September, 2005 (01.09.05), & EP 1716867 A1 | 1-14 |
| A | WO 2005/080322 A1 (Yamanouchi Pharmaceutical Co., Ltd.), 01 September, 2005 (01.09.05), & EP 1728784 A1 | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 March, 2008 (24.03.08) | Date of mailing of the international search report<br>08 April, 2008 (08.04.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/051962

Continuation of A. CLASSIFICATION OF SUBJECT MATTER
(International Patent Classification (IPC))

*A61K31/5377*(2006.01)i, *A61P25/00*(2006.01)i, *A61P25/14*(2006.01)i, *A61P25/18*(2006.01)i, *A61P25/20*(2006.01)i, *A61P25/22*(2006.01)i, *A61P25/24*(2006.01)i, *A61P25/28*(2006.01)i, *A61P43/00*(2006.01)i, *C07D401/04*(2006.01)i, *C07D401/06*(2006.01)i, *C07D401/14*(2006.01)i, *C07D403/04*(2006.01)i, *C07D405/04*(2006.01)i, *C07D405/14*(2006.01)i, *C07D409/04*(2006.01)i, *C07D409/06*(2006.01)i, *C07D409/14*(2006.01)i, *C07D413/06*(2006.01)i, *C07D471/04*(2006.01)i, *C07D491/048*(2006.01)i, *C07D495/04*(2006.01)i

(According to International Patent Classification (IPC) or to both national classification and IPC)

Continuation of B. FIELDS SEARCHED
Minimum documentation searched (International Patent Classification (IPC))

C07D401/04, C07D401/06, C07D401/14, C07D403/04, C07D405/04, C07D405/14, C07D409/04, C07D409/06, C07D409/14, C07D413/06, C07D471/04, C07D491/048, C07D495/04

Minimum documentation searched (classification system followed by classification symbols)

Form PCT/ISA/210 (extra sheet) (April 2007)

**INTERNATIONAL SEARCH REPORT**

International application No.
PCT/JP2008/051962

**Box No. II Observations where certain claims were found unsearchable (Continuation of item 2 of first sheet)**

This international search report has not been established in respect of certain claims under Article 17(2)(a) for the following reasons:

1. [X] Claims Nos.: 15
because they relate to subject matter not required to be searched by this Authority, namely:
Claim 15 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 19724979
- US 05082871 A
- US 04096771 A
- US 20060229323 A
- US 0041696 A
- US 9920599 B
- US 0017191 A
- US 05080322 A
- US 05079845 A
- US 07018168 B


### Non-patent literature cited in the description


- *Curr. Neurol. Neurosci. Rep.,* 2005, vol. 5 (6), 455-457
- *Eur. J. Pharmacol.,* 1998, vol. 346, 1-13
- *Curr. Psychiatry Rep.,* vol. 2 (6), 473-478
- *J. Psychopharmacol.,* 2000, vol. 14 (4), 406-408
- *J. Clin. Psychiatry,* 2005, vol. 66 (5), 658-659
- *Learning & memory,* 2001, vol. 8, 20-25
- *Togoshicchosho-chiryoyaku to Kanja eno Setsumei,* 2003, vol. 54, 287-304
- *Am J Psychiatry,* 2003, vol. 160, 1209-1222
- *Neuropsychopharmacology,* 2003, vol. 28 (8), 1400-1411
- *Diabetes Care,* 2004, vol. 27, 596
- *Rinsho-seishin-yakuri,* 2005, vol. 8 (12), 2151-2164
- *J. Abnorm. Psychol.,* 1997
- *Neuron,* 1999, vol. 22, 581-591
- *Neuroreport,* 2000, vol. 11, 2017-2020
- *Mol. Psychiatr.,* 2001, vol. 6, 217-219
- *J. Psychiatr. Res.,* 2004, vol. 38, 371-376
- **Jongen-Relo A. L. et al.** *36th Annual Meeting, Society of Neuroscience,* 14 October 2006
- *Prog. Med.,* 1985, vol. 5, 2157-2161
- Iyakuhin no Kaihatsu. Hirokawa Publishing Company, 1990, vol. 7
- *Bunshi Sekkei,* 163-198
- Protective Groups in Organic Synthesis. 1999
- Jikken Kagaku Koza. Yuki Gosei. Chemical Society of Japan, 1992, vol. 20, 300
- Synthetic Communications. *Synthetic Communications,* 1981, vol. 11, 513-519
- *Synlett,* 2000, vol. 6, 829-831
- *Chemistry Letters,* 1989, 1405-1408
- *Journal of the American Chemical Society,* 2001, vol. 123, 7727
- *Tetrahedron Letters,* 2002, vol. 43, 2187
- *Journal of Medicinal Chemistry,* 2005, vol. 48, 1540
- **Yamazaki M.** *J Pharmacol Exp Ther,* 1996, vol. 279, 1157-73